# EUROPEAN PATENT APPLICATION

(11) **EP 4 321 533 A1**
(43) Date of publication of application: **14.02.2024**
(21) Application number: 22784155.8
(22) Date of filing: 08.04.2022
(51) Int. Cl.: C07K 16/28, A61P 35/00

(54) **CELLULAR IMMUNOTHERAPY USE**

(30) Priority: 08.04.2021 CN 202110377518
(71) Applicant: Crage Medical Co., Limited, Wan Chai Hong Kong (CN)
(72) Inventor: LI, Zonghai, Shanghai 200231 (CN); WANG, Wei, Shanghai 200231 (CN); XIAO, Jun, Shanghai 200231 (CN)
(74) Representative: Lavoix
(86) International application number: PCT/CN2022/085921
(87) International publication number: WO 2022/214089

(57) **Abstract**

A cancer treatment composition and a cancer treatment method. Provided are a cancer treatment method and a treatment composition for patients for whom an anti-PD-1 antibody or anti-PD-L1 antibody treatment has failed.

## Description

### FIELD OF THE APPLICATION

The application belongs to the field of immunotherapy; in particular, it relates to an immune cell therapy that targets and recognizes tumor antigens, triggers the activation of immune effector cells, and exerts anti-tumor effects.

### BACKGROUND OF THE APPLICATION

Programmed death-1 (PD-1) is an immunosuppressive protein that negatively regulates TCR signaling. PD-L1 is the ligand of PD-1, expressed in many tumor types, such as ovarian cancer, breast cancer, cervical cancer, colon cancer, pancreatic cancer, gastric cancer, melanoma, glioblastoma, non-small cell lung cancer, etc. (Callahan 2014, J Leukoc Biol 94(1):41-53), the combination of PD-1 and PD-L1 can provide inhibitory signals, induce T cell apoptosis, and inhibit T cell activation and proliferation. Therefore, drugs targeting PD-1 or PD-L1 can exert anti-tumor effects through immune regulation. The currently marketed PD-1 antibodies or PD-L1 antibodies comprise Nivolumab, Pembrolizumab, Atezolizumab, Avelumab, Durvalumab, etc., but there are still many patients whose disease progresses after treatment with PD-1 antibodies or PD-L1 antibodies, and the treatment fails.

Cellular immunotherapy is a treatment method that uses the patients' autologous (or allogeneic) immune cells to repair tissues and organs. For example, CAR-T cell therapy is the use of chimeric antigen receptors to modify the patients' autologous (or allogeneic) T cells for the treatment of cancer and other diseases. Similar to the treatment of cancer with PD-1 antibodies or PD-L1 antibodies, both of them use the immune system to kill tumors. Since patients who have failed treatment with PD-1 antibody or PD-L1 antibodies may be less suitable for tumor immunotherapy, patients who are resistant to treatment with PD-1 antibodies or PD-L1 antibodies may also be insensitive to other immunotherapies (such as CAR-T cell therapy).

### SUMMARY OF THE APPLICATION

The object of the present application is to provide a composition for cancer treatment and a method for treating cancer, especially a method for treating cancer and a treatment composition for patients who have failed treatment with anti-PD-1 antibodies and/or anti-PD-L1 antibodies.

Specifically, the present application relates to the following items:
**1.** Use of a cell therapy product in the preparation of a medicament for the treatment of cancer in a patient who has failed previous treatment for cancers, said previous treatment includes treatment with an anti-PD-1 antibody and/or an anti-PD-L1 antibody.
**2.** The use according to item 1, wherein the cell therapy product contains an immune effector cell expressing an exogenous receptor.
**3.** The use according to item 2, wherein the immune effector cell is selected from a T cell, an NK cell, an NKT cell, a mast cell, a macrophage, a dendritic cell, a CIK cell, and a stem cell-derived immune effector cell, or any combination thereof.
**4.** The use according to item 2, wherein the immune effector cell is derived from a natural T cell and/or a T cell induced by a pluripotent stem cell.
**5.** The use according to any one of items 2-4, wherein the immune effector cell is an autologous/allogeneic T cell, or a primary T cell.
**6.** The use according to any one of items 3-5, wherein the T cell comprises a memory stem cell-like T cell (Tscm cell), a central memory T cell (Tcm), an effector T cell (Tef), a regulatory T cell (Tregs), en effector memory T cell (Tern), a γδ T cell, an αβ T cell, or any combination thereof.
**7.** The use according to any one of items 2-6, wherein the exogenous receptor is selected from a chimeric antigen receptor (CAR), a T cell receptor (TCR), a T cell fusion protein (TFP), and a T cell antigen coupler (TAC), or any combination thereof.
**8.** The use according to any one of items 2-7, wherein the antigen-binding domain of the exogenous receptor specifically recognizes a tumor antigen.
**9.** The use according to item 8, wherein the tumor antigen is selected from CD19, CD20, CD22, CD30, Mesothelin, BCMA, EGFR, EGFRvIII, PSMA, Mucl, claudin18.2, GPC3, IL13RA2, SLAMF7, GPRC5D, and LILRB4, or any combination thereof; preferably, the tumor antigen is selected from GPC3, CD19, BCMA, and Claudin18.2, or any combination thereof.
**10.** The use according to any one of items 1-9, wherein the cancer comprises a solid tumor and/or a blood tumor; preferably the solid tumor comprises gastrointestinal tumors; more preferably said gastrointestinal tumors comprise gastric cancer/esophagogastric junction adenocarcinoma, gallbladder cancer, liver cancer, Krukenberg tumor, or any combination thereof.
**11. The** use according to any one of items 1-10, wherein the cancer is selected from the group consisting of krukenberg tumor, gastric cancer, pancreatic cancer, gallbladder cancer, melanoma, non-small cell lung cancer (NSCLC), small cell lung cancer (SCLC), head and neck squamous cell carcinoma (HNSCC), classical Hodgkin lymphoma (cHL), primary mediastinal large B-cell lymphoma (PMBCL), urothelial carcinoma (UC), esophageal cancer, cervical cancer, liver cancer, Merkel cell carcinoma, renal cell carcinoma (RCC), colorectal cancer (mCRC), and breast cancer.
**12.** The use according to any one of items 1-11, wherein the anti-PD-1 antibody/PD-L1 antibody is selected from Nivolumab, Pembrolizumab, Cemiplimab, Camrelizumab, Toripalimab, Sintilimab, Tislelizumab, Cepalimumab (GLS-010), and Tislelizumab, or any combination thereof.
**13.** The use according to any one of items 1-12, wherein the previous treatment comprises drug therapy, surgical treatment, radiotherapy or any combination thereof; the drug therapy comprises giving chemical drugs and/or biological drugs; preferably, the previous treatment comprises administration of olaparib, lenvatinib, cabozantinib, axitinib, ipilimumab, platinum-based chemotherapy drugs, pemetrexed, etoposide, taxanes compound, bevacizumab, regorafenib, rotinib, apatinib, lenvatinib, fruquintinib, regorafenib, or any combination thereof.
**14.** The use according to item 1, wherein at least one cycle of a cell therapy product is administered to a patient with the cancer for treatment; preferably, 1-3 cycles of the cell therapy product are administered to patients with the cancer for treatment.
**15.** The use according to any one of items 7-14, wherein the chimeric antigen receptor (CAR) comprises:
   (i) an antigen-binding domain specifically binding to a tumor antigen, a transmembrane region of CD28 or CD8, and CD3ζ;
   (ii) an antigen-binding domain specifically binding to a tumor antigen, a transmembrane region of CD28 or CD8, a co-stimulatory signal domain of CD28, and CD3ζ;
   (iii) an antigen-binding domain specifically binding to a tumor antigen, a transmembrane region of CD28 or CD8, a co-stimulatory signal domain of CD137, and CD3ζ; and/or
   (iv) an antigen-binding domain specifically binding to a tumor antigen, a transmembrane region of CD28 or CD8, a co-stimulatory signal domain of CD28, a co-stimulatory signal domain of CD137, and CD3ζ.
**16.** The use according to item 14 or 15, wherein the dose of cells in the cell therapy product administered per cycle does not exceed about 2×10⁹ cells/kg, 2×10⁸ cells/kg, or 2x10' cells/kg of patient body weight; or the dose of cells in the cell therapy product does not exceed about 1×10¹¹ cells/patient, 1×10¹⁰ cells/patient, 5×10⁹ cells/patient, 2×10⁹ cells/patient, or 1×10⁹ cells/patient.
**17.** The use according to item 16, wherein the dose of cells in the cell therapy product administered per cycle is from about 1×10⁵ cells/kg to 2×10⁷ cells/kg of patient body weight, or from about 1×10⁶ cells/kg to 2×10⁷ cells/kg patient body weight; or
   the dose of cells in the cell therapy product administered per cycle is about 1×10⁷ cells to 5×10⁹ cells/patient, about 1×10⁷ cells to 2×10⁹ cells/patient, or about 1×10⁷ cells to 1×10⁹ cells/patient; or the dose of cells in the cell therapy product administered per cycle is about 1×10⁸ cells to 5×10⁹ cells/patient, about 1×10⁸ cells to 2×10⁹ cells/patient, or about 1×10⁸ cells to 1×10⁹ cells/patient; or the dose of cells in the cell therapy product administered per cycle is about 2.5×10⁸ cells to 5×10⁸ cells/patient.
**18.** The use according to any one of items 14-17, wherein pretreatment is performed before administration of the cell therapy product per cycle, and the pretreatment comprises administration of a chemical drug, a biological drug, radiotherapy, or any combination thereof to the patient.
**19.** The use according to item 18, wherein the pretreatment is implemented 1-8 days before administration of the cell therapy product; preferably, 2-6 days before administration of the cell therapy product; preferably, a chemical drug, a biological drug, a radiotherapy or any combination thereof is administered for no more than 4 consecutive days.
**20.** The use according to item 18 or 19, wherein the chemical drug is selected from any one or at least two of the following: cyclophosphamide, fludarabine, a tubulin inhibitor, pyrimidine antineoplastic drugs; or the chemical drug comprises cyclophosphamide and fludarabine; or the chemical drug comprises cyclophosphamide, fludarabine and a tubulin inhibitor.
**21.** The use according to item 20, wherein the tubulin inhibitor is a taxane compound; preferably the taxane compound is selected from paclitaxel, nab-paclitaxel, and docetaxel; more preferably the taxane compound is nab-paclitaxel.
**22.** The use according to item 20 or 21, wherein the amount of fludarabine to be administered is about 10-50mg/m²/day, or about 15-40mg/m²/day, or about 15-30mg/m²/day, or about 20-30mg/m²/day, or about 25mg/m²/day, or about 30-60mg/day, or about 30-50mg/day, or about 35-45mg/day;
   the amount of cyclophosphamide to be administered is about 200-400mg/m²/day, or about 200-300mg/m²/day, or about 250mg/m²/day, or about 300-700mg/day, or about 300-550mg/day, or about 300-500mg/day;
   the dosage of the taxane compound is not more than about 300mg/day, or not more than about 200 mg/day, or about 90-120 mg/day.
**23.** The use according to any one of items 20-22, wherein the cyclophosphamide is administered 2-3 times; or the fludarabine is administered 1-2 times; or the taxane compound is administered once.
**24.** The use according to any one of items 8-15, wherein the antigen binding domain has:
   HCDR1 shown in SEQ ID NO:1, HCDR2 shown in SEQ ID NO:2, HCDR3 shown in SEQ ID NO:3, LCDR1 shown in SEQ ID NO:4, LCDR2 shown in SEQ ID NO:5, LCDR3 shown in SEQ ID NO: 6; or
   HCDR1 shown in SEQ ID NO:16, HCDR2 shown in SEQ ID NO:17, HCDR3 shown in SEQ ID NO:18, LCDR1 shown in SEQ ID NO:19, LCDR2 shown in SEQ ID NO:20, LCDR3 shown in SEQ ID NO:21; or
   HCDR1 shown in SEQ ID NO:27, HCDR2 shown in SEQ ID NO:28, HCDR3 shown in SEQ ID NO:29, LCDR1 shown in SEQ ID NO:30, LCDR2 shown in SEQ ID NO:31, LCDR3 shown in SEQ ID NO:32;
   or the antigen binding domain has:
      the heavy chain variable region shown in SEQ ID NO:7 and the light chain variable region shown in SEQ ID NO:9; or
      the heavy chain variable region shown in SEQ ID NO:22 and the light chain variable region shown in SEQ ID NO:23; or
      the heavy chain variable region shown in SEQ ID NO:33 and the light chain variable region shown in SEQ ID NO:34;
      or the antigen-binding domain has the sequence shown in SEQ ID NO: 14, 24, 35, or 37.
**25.** The use according to any one of items 7-24, wherein the chimeric antigen receptor has the amino acid sequence shown in any one of SEQ ID NO: 11, 12, 13, 25, 26, 36, 38, or 52, or the polypeptide formed by sequentially connecting any sequence shown in SEQ ID NO: 58, 59, 60, 61, 62, 63, 64, 65, 66, or 67 with the sequence shown in SEQ ID NO: 80, 81, or 82, or the polypeptide formed by sequentially connecting any sequence shown in SEQ ID NO: 70, 71, 72, 73, 74, 75, 76, 77, 78 or 79 with the sequence shown in SEQ ID NO: 80, 81 or 82.
**26.** The use according to any one of items 14-25, wherein before administering the cell therapy product in each cycle, the serum levels of a cytokine indicating CRS, a cytokine indicating neurotoxicity, an indicator indicating tumor burden, and/or a factor indicating host anti-CAR immune response in the patient are evaluated.
**27.** The use according to any one of items 1-26, wherein after administration of the cell therapy product, the patient does not show severe CRS, or does not show neurotoxicity exceeding Level 3.
**28.** The use according to any one of items 1-27, wherein at least a part, preferably at least 40%, more preferably at least 50% of the patients who have failed to treat cancer with anti-PD-1 antibody or anti-PD-L1 antibody respond to the cell therapy product.
**29.** The use according to item 28, wherein the response means an ORR greater than 40%, preferably greater than 50%.
**30.** A method of using a cell therapy product to treat cancer in a patient who has failed previous treatment for the cancer, wherein the previous treatment comprises treatment with an anti-PD-1 antibody and/or an anti-PD-L1 antibody.
**31.** The method according to item 30, wherein the cell therapy product comprises an immune effector cell expressing an exogenous receptor.
**32.** The method according to item 31, wherein the immune effector cell is selected from the group consisting of a T cell, an NK cell, an NKT cell, a mast cell, a macrophage, a dendritic cell, a CIK cell, and a stem cell-derived immune effector cell, or any combination thereof.
**33.** The method according to item 31, wherein the immune effector cell is derived from a natural T cell and/or a T cell induced by a pluripotent stem cell.
**34.** The method according to any one of items 31-33, wherein the immune effector cell is an autologous/allogeneic T cell, or a primary T cell.
**35.** The method according to any one of items 32-34, wherein the T cell comprises a memory stem cell-like T cell (Tscm cell), a central memory T cell (Tcm), an effector T cell (Tef), a regulatory T cell (Tregs), an effector memory T cell (Tern), a γδ T cell, an αβ T cell or any combination thereof.
**36.** The method according to any one of items 31-35, wherein the exogenous receptor is selected from a chimeric antigen receptor (CAR), a T cell receptor (TCR), a T cell fusion protein (TFP), and a T cell antigen coupler (TAC), or any combination thereof.
**37.** The method according to any one of items 31-36, wherein the antigen binding domain of the exogenous receptor specifically recognizes a tumor antigen.
**38.** The method according to item 37, wherein the tumor antigen is selected from CD19, CD20, CD22, CD30, Mesothelin, BCMA, EGFR, EGFRvIII, PSMA, Mucl, claudin18.2, GPC3, IL13RA2, SLAMF7, GPRC5D, and LILRB4, or any combination thereof; preferably, the tumor antigen is selected from GPC3, CD19, BCMA, and Claudin18.2, or any combination thereof.
**39.** The method according to any one of items 30-38, wherein the cancer comprises a solid tumor and/or a hematological tumor; preferably the solid tumor comprises a gastrointestinal tumor; more preferably the gastrointestinal tumor comprises gastric cancer/esophagogastric junction adenocarcinoma, gallbladder cancer, liver cancer, Krukenberg tumor, or any combination thereof.
**40.** The method according to any one of items 30-39, wherein the cancer is selected from the group consisting of krukenberg tumor, gastric cancer, pancreatic cancer, gallbladder cancer, melanoma, non-small cell lung cancer (NSCLC), small cell lung cancer (SCLC), head and neck squamous cell carcinoma (HNSCC), classical Hodgkin lymphoma (cHL), primary mediastinal large B-cell lymphoma (PMBCL), urothelial carcinoma (UC), esophageal cancer, cervical cancer, liver cancer, Merkel cell carcinoma, renal cell carcinoma (RCC), colorectal cancer (mCRC), and breast cancer.
**41.** The method according to any one of items 30-40, wherein the anti-PD-1 antibody/PD-L1 antibody is selected from Nivolumab, Pembrolizumab, Cemiplimab, Camrelizumab, Toripalimab, Sintilimab, Tislelizumab, Cepalimumab (GLS-010), and Tislelizumab, or any combination thereof.
**42.** The method according to any one of items 30-41, wherein the previous treatment comprises drug therapy, surgical treatment, radiotherapy or any combination thereof; the drug therapy comprises administration of a chemical drug and/or a biological drug; preferably, the previous treatment comprises administration of olaparib, lenvatinib, cabozantinib, axitinib, ipilimumab, platinum-based chemotherapy drugs, pemetrexed, etoposide, a taxane compound, bevacizumab, regorafenib, rotinib, apatinib, lenvatinib, fruquintinib, regorafenib, or combinations thereof.
**43.** The method according to item 30, wherein at least one cycle of cell therapy product is administered to a patient with the cancer for treatment; preferably, 1-3 cycles of cell therapy product are administered to a patient with the cancer for treatment.
**44.** The method according to any one of items 36-43, wherein the chimeric antigen receptor (CAR) comprises:
   (i) an antigen-binding domain specifically binding to a tumor antigen, a transmembrane region of CD28 or CD8, and CD3ζ;
   (ii) an antigen-binding domain specifically binding to a tumor antigen, a transmembrane region of CD28 or CD8, a co-stimulatory signal domain of CD28, and CD3ζ;
   (iii) an antigen-binding domain specifically binding to a tumor antigen, a transmembrane region of CD28 or CD8, a co-stimulatory signal domain of CD137, and CD3ζ; and/or
   (iv) an antigen-binding domain specifically binding to a tumor antigen, a transmembrane region of CD28 or CD8, a co-stimulatory signal domain of CD28, a co-stimulatory signal domain of CD137, and CD3ζ.
**45.** The method according to item 43 or 44, wherein the dose of cells in the cell therapy product administered per cycle does not exceed about 2×10⁹ cells/kg, 2×10⁸ cells/kg, or 2x10' cells/kg of patient body weight; or the dose of cells in the cell therapy product does not exceed about 1×10¹¹ cells/patient, 1×10¹⁰ cells/patient, 5×10⁹ cells/patient, 2×10⁹ cells/patient, or 1×10⁹ cells/patient.
**46.** The method according to item 45, wherein the dose of cells in the cell therapy product administered per cycle is from about 1×10⁵ cells/kg patient body weight to 2x10' cells/kg patient body weight, or from about 1×10⁶ cells/kg patient body weight to 2×10⁷ cells/kg patient body weight; or
   the dose of cells in the cell therapy product administered per cycle is about 1×10⁷ cells to 5×10⁹ cells/patient, about 1×10⁷ cells to 2×10⁹ cells/patient, or about 1×10⁷ cells to 1×10⁹ cells/patient; or the dose of cells in the cell therapy product administered per cycle is about 1×10⁸ cells to 5×10⁹ cells/patient, about 1×10⁸ cells to 2×10⁹ cells/patient, or about 1×10⁸ cells to 1×10⁹ cells/patient; or the dose of cells in the cell therapy product administered per cycle is about 2.5×10⁸ cells to 5×10⁸ cells/patient.
**47.** The method according to any one of items 43-46, wherein pretreatment is performed before administration of the cell therapy product per cycle, and the pretreatment comprises administration of a chemical drug, a biological drug, radiotherapy, or any combination thereof to the patient.
**48.** The method according to item 47, wherein the pretreatment is implemented 1-8 days before administration of the cell therapy product; preferably, 2-6 days before administration of the cell therapy product; preferably, a chemical drug, a biological drug, radiotherapy or any combination thereof is administered for no more than 4 consecutive days.
**49.** The method according to item 47 or 48, wherein the chemical drug is selected from any one or at least two of the following: cyclophosphamide, fludarabine, a tubulin inhibitor, a pyrimidine antineoplastic drug; or the chemical drug comprises cyclophosphamide and fludarabine; or the chemical drug comprises cyclophosphamide, fludarabine and a tubulin inhibitor.
**50.** The method according to item 49, wherein the tubulin inhibitor is a taxane compound;
   preferably the taxane compound is selected from paclitaxel, nab-paclitaxel, and docetaxel; more preferably the taxane compound is nab-paclitaxel.
**51.** The method according to item 49 or 50, wherein the amount of fludarabine to be administered is about 10-50mg/m²/day, or about 15-40mg/m²/day, or about 15-30mg/m²/day, or about 20-30mg/m²/day, or about 25mg/m²/day, or about 30-60mg/day, or about 30-50mg/day, or about 35-45mg/day;
   the amount of cyclophosphamide to be administered is about 200-400mg/m²/day, or about 200-300mg/m²/day, or about 250mg/m²/day, or about 300-700mg/day, or about 300-550mg/day, or about 300-500mg/day;
   the dosage of the taxane compound is not more than about 300mg/day, or not more than about 200 mg/day, or about 90-120 mg/day.
**52.** The method according to any one of items 49-51, wherein the cyclophosphamide is administered 2-3 times; or the fludarabine is administered 1-2 times; or the taxane compound is administered once.
**53.** The method according to any one of items 37-44, wherein the antigen binding domain has:
   HCDR1 shown in SEQ ID NO:1, HCDR2 shown in SEQ ID NO:2, HCDR3 shown in SEQ ID NO:3, LCDR1 shown in SEQ ID NO:4, LCDR2 shown in SEQ ID NO:5, LCDR3 shown in SEQ ID NO: 6; or
   HCDR1 shown in SEQ ID NO:16, HCDR2 shown in SEQ ID NO:17, HCDR3 shown in SEQ ID NO:18, LCDR1 shown in SEQ ID NO:19, LCDR2 shown in SEQ ID NO:20, LCDR3 shown in SEQ ID NO:21; or
   HCDR1 shown in SEQ ID NO:27, HCDR2 shown in SEQ ID NO:28, HCDR3 shown in SEQ ID NO:29, LCDR1 shown in SEQ ID NO:30, LCDR2 shown in SEQ ID NO:31, LCDR3 shown in SEQ ID NO:32;
   or the antigen binding domain has:
      the heavy chain variable region shown in SEQ ID NO:7 and the light chain variable region shown in SEQ ID NO:9; or
      the heavy chain variable region shown in SEQ ID NO:22 and the light chain variable region shown in SEQ ID NO:23; or
      the heavy chain variable region shown in SEQ ID NO:33 and the light chain variable region shown in SEQ ID NO:34;
      or the antigen-binding domain has the sequence shown in SEQ ID NO: 14, 24, 35, or 37.
**54.** The method according to any one of items 36-53, wherein the chimeric antigen receptor has the amino acid sequence shown in any one of SEQ ID NO: 11, 12, 13, 25, 26, 36, 38, or 52, or the polypeptide formed by sequentially connecting any sequence shown in SEQ ID NO: 58, 59, 60, 61, 62, 63, 64, 65, 66, or 67 with the sequence shown in SEQ ID NO: 80, 81, or 82, or the polypeptide formed by sequentially connecting any sequence shown in SEQ ID NO: 70, 71, 72, 73, 74, 75, 76, 77, 78 or 79 with the sequence shown in SEQ ID NO: 80, 81 or 82.
**55.** The method according to any one of items 43-54, wherein before administering the cell therapy product per cycle, the serum levels of a cytokine indicating CRS, a cytokine indicating neurotoxicity, an indicator indicating tumor burden, and/or a factor indicating host anti-CAR immune response in the patient are evaluated.
**56.** The method according to any one of items 30-55, wherein after administration of the cell therapy product, the patient does not show severe CRS, or does not show neurotoxicity exceeding Level 3.
**57.** The method according to any one of items 30-56, wherein at least a part, preferably at least 40%, more preferably at least 50% of the patients who have failed to treat cancer with anti-PD-1 antibody or anti-PD-L1 antibody respond to the cell therapy product.
**58.** The method according to item 57, wherein the "response means an ORR greater than 40%, preferably greater than 50%.

In order to achieve the above object, the technical scheme provided by the application is as follows:
In a first aspect of the present application, provided is the use of cell therapy products for the cancer treatments that has failed to be treated with anti PD-1 antibodies or anti PD-L1 antibodies in previous treatments.

In a preferred embodiment, some patients who have failed previous treatments with the anti-PD-1 antibodies or anti-PD-L1 antibodies are responsive to the cell therapy products.

In a preferred embodiment, at least 40% of patients who have failed previous treatments with the anti-PD-1 antibodies or anti-PD-L1 antibodies are responsive to the cell therapy products.

In a preferred embodiment, at least 50% of patients who have failed previous treatments with anti-PD-1 antibodies or anti-PD-L1 antibodies are responsive to the cell therapy products.

In a preferred embodiment, the "responsive" refers to an ORR greater than 40%; preferably, greater than 50%.

In a preferred embodiment, chemical drugs or biological drugs are also used in the previous treatments; preferably, the chemical drugs or biological drugs are Olaparib, Lenvatinib, Cabozantinib, Axitinib, Ipilimumab, platinum-based chemotherapy drugs, Pemetrexed, Etoposide, taxane compounds, Bevacizumab, or Regorafenib.

In a preferred embodiment, the anti-PD-1 antibodies are selected from Nivolumab, Pembrolizumab, Cemiplimab, Camrelizumab, Toripalimab, Sintilimab, Tislelizumab, Sepalimumab (GLS-010), and Tislelizumab; preferably, the anti-PD-1 antibodies are selected from Nivolumab, Pembrolizumab, Camrelizumab, Toripalimab, Sintilimab, and Tislelizumab.

In a preferred embodiment, the anti-PD-L1 antibodies are selected from Atezolizumab, Durvalumab, Avelumab, and Sugemalimumab (CS1001).

In a preferred embodiment, the cell therapy products contain immune effector cells expressing exogenous receptors, preferably, the immune effector cells are selected from T cells, NK cells, NKT cells, mast cells, macrophages, dendritic cells, CIK cells, and stem cell-derived immune effector cells.

In a preferred embodiment, the immune effector cells are selected from T cells, and the exogenous receptors are selected from chimeric antigen receptors (CARs), T cell receptors (TCRs), T cell fusion proteins (TFPs) and T cell antigen couplers (TACs).

In a preferred embodiment, the exogenous receptors are selected from chimeric antigen receptors (CARs).

In a preferred embodiment, the immune effector cells are selected from T cells, NK cells, and NKT cells.

In a preferred embodiment, the cell therapy products specifically recognize tumor antigens, preferably, the tumor antigens are selected from CD19, CD20, CD22, CD30, Mesothelin, BCMA, EGFR, EGFRvIII, PSMA, Mucl, claudin18.2, GPC3, IL13RA2, SLAMF7, GPRC5D, and LILRB4; more preferably, the tumor antigens are selected from CD19, BCMA, and Claudin18.2.

In a preferred embodiment, the cancers are selected from gastric cancer, pancreatic cancer, gallbladder cancer, melanoma, non-small cell lung cancer (NSCLC), small cell lung cancer (SCLC), head and neck squamous cell carcinoma (HNSCC), classical Hodgkin lymphoma (cHL), primary mediastinal large B-cell lymphoma (PMBCL), urothelial carcinoma (UC), esophageal carcinoma, cervical carcinoma, hepatocellular carcinoma, Merkel cell carcinoma, renal cell carcinoma (RCC), colorectal cancer (mCRC), and breast cancer.

In a preferred embodiment, the cancers are selected from gastric cancer, non-small cell lung cancer (NSCLC), small cell lung cancer (SCLC), urothelial cancer (UC), liver cancer, and melanoma.

In a preferred embodiment, at least one cycle of cell therapy product is administered to the patient for treatment; preferably, 1-3 cycles of cell therapy products are administered to the patient for treatment.

In a preferred embodiment, the dose of cells in the cell therapy products administered per cycle does not exceed about 2×10⁹ cells/kg of body weight of the subject, or the doses of cells in the cell therapy products do not exceed about 1×10¹¹ cells/person. Preferably, the dose of cells in the cell therapy products administered per cycle does not exceed about 2×10⁸ cells/kg of body weight of the subject, or the doses of cells in the cell therapy products do not exceed about 1×10¹⁰ cells/person. More preferably, the dose of cells in the cell therapy products administered per cycle does not exceed about 2×10⁷ cells/kg of body weight of the subject, or the doses of cells in the cell therapy products do not exceed about 1×10⁹ cells/person.

In a preferred embodiment, the doses of cells in the cell therapy products administered per cycle are about 1×10⁵ cells/kg of body weight of the subject to 2×10⁷ cells/kg of body weight of the subject, or about 1×10⁶ cells/kg of body weight of the subject to 2×10⁷ cells/kg of body weight of the subject.

In a preferred embodiment, the doses of cells in the cell therapy products administered per cycle are 1×10⁷ cells to 1×10⁹ cells, preferably, 1×10⁸ cells to 1×10⁹ cells, more preferably, 2.5×10⁸ cells to 5×10⁸ cells.

In a preferred embodiment, subsequent cycles of treatments are administered after the cell products in the previous treatment cycle are undetectable in vivo.

In a preferred embodiment, the cell therapy products in the subsequent cycles are administered 30 days after administration of the cell products of the previous cycle is completed.

In a preferred embodiment, the dose of the cell products administered in the subsequent cycles is sufficient to stabilize or reduce the number of cells in the subjects' tumor burden.

In a preferred embodiment, the doses of the cell products administered in different cycles are different or the same.

In a preferred embodiment, the cell therapy products in each cycle is divided into 1-5 administrations, preferably, divided into 1-3 administrations.

In a preferred embodiment, the administration of the all cell therapy products in each cycle is completed within 5 days, more preferably, within 3 days.

In a preferred embodiment, the cell therapy products for each time is completely infused within 1 hour, preferably within 30 minutes, more preferably within 4-30 minutes.

In a preferred embodiment, when the treatments in the subsequent cycles are administered, the subjects have any of the following characteristics:
(i) the peak level of cytokine release syndrome (CRS)-related cytokines in the subjects' serum is smaller than that in the subjects' serum after the administration of cell therapy products in the previous cycle;
(ii) it does not show Level 3 or higher neurotoxicity;
(iii) the level of CRS is reduced compared with the level of CRS after administration of cell therapy products in the previous cycle;
(iv) the subjects do not show a detectable humoral or cell-mediated immune response to the cell therapy product.

In a preferred embodiment, in the above (i), the level of cytokines is reduced by at least 50%, preferably by at least 20%, more preferably by at least 5%, compared with the peak level of cytokines after administration of cell therapy products in the previous cycle.

In a preferred embodiment, the CRS level is comparable to the CRS level before administering the cell therapy products in previous cycles.

In a preferred embodiment, pretreatments are performed before administering the cell therapy products per cycle, and the pretreatments comprise administering chemical drugs and/or radiotherapies to the subjects.

In a preferred embodiment, the radiotherapies comprise whole body radiotherapies or local radiotherapies.

In a preferred embodiment, the pretreatments are carried out 1-8 days before administration of the cell therapy products; preferably, 2-6 days before administration of the cell therapy products.

In a preferred embodiment, the chemical drugs are selected from any one or at least two of the following: cyclophosphamide, fludarabine, tubulin inhibitors, and pyrimidine antineoplastic drugs.

In a preferred embodiment, the chemical drugs are cyclophosphamide and fludarabine; or cyclophosphamide, fludarabine and tubulin inhibitors.

In a preferred embodiment, the tubulin inhibitors are taxane compounds; preferably, the taxane compounds are selected from paclitaxel, nab-paclitaxel, and docetaxel; more preferably, the taxane compounds are nab-paclitaxel.

In a preferred embodiment, the pyrimidine antineoplastic drugs are selected from 5-fluorouracil, bisfururacil, carmofur, doxifluridine, and capecitabine.

In a preferred embodiment, the amount of fludarabine to be administered is about 10-50mg/m²/day, or about 15-40mg/m²/day, or about 15-30mg/m²/day, or about 20-30mg/m²/day; or about 25mg/m²/day.

In a preferred embodiment, the amount of fludarabine to be administered is about 30-60 mg/day, or about 30-50 mg/m²/day.

In a preferred embodiment, the amount of cyclophosphamide to be administered is about 200-400 mg/m²/day, about 200-300 mg/m²/day, or about 250 mg/m²/day.

In a preferred embodiment, the amount of cyclophosphamide to be administered is about 300-700 mg/day, about 300-550 mg/day, or about 300-500 mg/day.

In a preferred embodiment, the amount of the taxane compounds to be administered is not more than about 300 mg/day, or not more than about 200 mg/day; preferably, the amount of the taxane compounds to be administered is about 90-120 mg/day .

In a preferred embodiment, each of the chemical drugs is used continuously for no more than 4 days.

In a preferred embodiment, the cyclophosphamides are administered 2-3 times; or the fludarabines are administered 1-2 times.

In a preferred embodiment, the taxane compounds are administered once.

In a preferred embodiment, the exogenous receptors comprise extracellular antigen-binding domains, transmembrane domains and intracellular signaling domains, and the extracellular antigen-binding domains comprise antibodies or fragments thereof that specifically recognize tumor antigens, wherein the antibodies or fragments thereof comprise:
HCDR1 shown in SEQ ID NO: 1, HCDR2 shown in SEQ ID NO: 2, HCDR3 shown in SEQ ID NO: 3, LCDR1 shown in SEQ ID NO: 4, LCDR2 shown in SEQ ID NO: 5, LCDR3 shown in SEQ ID NO: 6; or
HCDR1 shown in SEQ ID NO: 16, HCDR2 shown in SEQ ID NO: 17, HCDR3 shown in SEQ ID NO: 18, LCDR1 shown in SEQ ID NO: 19, LCDR2 shown in SEQ ID NO: 20, LCDR3 shown in SEQ ID NO: 21; or
HCDR1 shown in SEQ ID NO: 27, HCDR2 shown in SEQ ID NO: 28, HCDR3 shown in SEQ ID NO: 29, LCDR1 shown in SEQ ID NO: 30, LCDR2 shown in SEQ ID NO: 31, LCDR3 shown in SEQ ID NO:32.

Preferably, the antibodies or fragments thereof comprise:
the heavy chain variable region shown in SEQ ID NO: 7 and the light chain variable region shown in SEQ ID NO: 9; or
the heavy chain variable region shown in SEQ ID NO: 22 and the light chain variable region shown in SEQ ID NO: 23; or
the heavy chain variable region shown in SEQ ID NO:33 and the light chain variable region shown in SEQ ID NO:34.

In a preferred embodiment, the extracellular antigen-binding domains of the exogenous receptors have the sequence shown in SEQ ID NO: 14, 24, 35, or 37.

In a preferred embodiment, the chimeric antigen receptors have amino acid sequences shown in any one of SEQ ID NO: 11, 12, 13, 25, 26, 36, 38, or 52.

In a preferred embodiment, the subjects have not received cell therapies before administrating the cell therapy products.

In a preferred embodiment, before administering the cell therapy products, the subjects also received drug treatments, surgical treatments, radiotherapies, or any combinations thereof.

In a preferred embodiment, the drug therapies comprise chemical drug therapies or biological drug therapies.

In a preferred embodiment, the chemical drug therapies comprise Anlotinib, Apatinib, Lenvatinib, Fruquintinib and Regorafenib.

In a preferred embodiment, before administering the cell therapy products per cycle, the serum levels of cytokines indicating CRS, cytokines indicating neurotoxicity, indicators indicating tumor burden, and/or factors indicating host anti-CAR immune response in the subjects are evaluated.

In a preferred embodiment, the indicators indicating tumor burden are: the total number of tumor cells in the subjects, or the total number of tumor cells in the organs of the subjects, or the total number of tumor cells in the tissues of the subjects, or the mass or volume of the tumors, or the degree of tumor metastasis, or the number of the tumors.

In a preferred embodiment, the dosage of the cell therapy products to be administered is determined based on the results of said evaluation.

In a preferred embodiment, after administration of the cell therapy product, the subjects do not show severe CRS, or do not show neurotoxicity exceeding Level 3.

In a preferred embodiment, after administration of the cell therapy product, the cell therapy products proliferate in the subjects.

In the second aspect of the present application, also provided is a method for treating tumors with immune effector cells modified by exogenous receptors. The method comprises administrating pretreatment drugs before administering immune effector cells to tumor patients, and the pretreatments comprise cyclophosphamide, fludarabine, and tubulin inhibitors.

In a preferred embodiment, the amount of fludarabine to be administered is about 10-50mg/m²/day, about 15-40mg/m²/day, about 15-30mg/m²/day, or about 20-30mg/m²/day; or about 25mg/m²/day.

In a preferred embodiment, the amount of fludarabine to be administered is about 30-60 mg/day, or about 30-50 mg/m²/day.

In a preferred embodiment, the amount of cyclophosphamide to be administered is about 200-400 mg/m²/day, about 200-300 mg/m²/day, or about 250 mg/m²/day.

In a preferred embodiment, the amount of cyclophosphamide to be administered is about 300-700 mg/day, or about 300-550 mg/day, or about 300-500 mg/day.

In a preferred embodiment, the amount of the taxane compounds to be administered are not more than about 300 mg/day, or not more than about 200 mg/day; preferably, the amount of the taxane compounds to be administered is about 90-120 mg/day.

In a preferred embodiment, each of the pretreatment drugs is used continuously for no more than 4 days.

In a preferred embodiment, the cyclophosphamides are administered 2-3 times;

In a preferred embodiment, the fludarabines are administered 1-2 times.

In a preferred embodiment, the tubulin inhibitors are administered once.

In a preferred embodiment, the tubulin inhibitors are the taxane compounds; preferably, the taxane compounds are selected from paclitaxel, nab-paclitaxel, and docetaxel; more preferably, the taxane compounds are nab-paclitaxel.

In a preferred embodiment, the exogenous receptors comprise extracellular antigen-binding domains, transmembrane domains and intracellular signaling domains, and the extracellular antigen-binding domains contain antibodies or fragments thereof that specifically recognize tumor antigens, wherein the antibodies or fragments thereof have:
HCDR1 shown in SEQ ID NO: 1, HCDR2 shown in SEQ ID NO: 2, HCDR3 shown in SEQ ID NO: 3, LCDR1 shown in SEQ ID NO: 4, LCDR2 shown in SEQ ID NO: 5, LCDR3 shown in SEQ ID NO: 6; or
HCDR1 shown in SEQ ID NO: 16, HCDR2 shown in SEQ ID NO: 17, HCDR3 shown in SEQ ID NO: 18, LCDR1 shown in SEQ ID NO: 19, LCDR2 shown in SEQ ID NO: 20, LCDR3 shown in SEQ ID NO: 21; or
HCDR1 shown in SEQ ID NO: 27, HCDR2 shown in SEQ ID NO: 28, HCDR3 shown in SEQ ID NO: 29, LCDR1 shown in SEQ ID NO: 30, LCDR2 shown in SEQ ID NO: 31, LCDR3 shown in SEQ ID NO:32.

Preferably, the antibodies or fragments thereof have:
the heavy chain variable region shown in SEQ ID NO: 7 and the light chain variable region shown in SEQ ID NO: 9; or
the heavy chain variable region shown in SEQ ID NO: 22 and the light chain variable region shown in SEQ ID NO: 23; or

The heavy chain variable region shown in SEQ ID NO:33 and the light chain variable region shown in SEQ ID NO:34.

In a preferred embodiment, the extracellular antigen-binding domains of the exogenous receptors have the sequence shown in SEQ ID NO: 14, 24, 35, or 37.

In a preferred embodiment, the immune effector cells are selected from T cells, NK cells, NKT cells, mast cells, macrophages, dendritic cells, CIK cells, and stem cell-derived immune effector cells.

In a preferred embodiment, the immune effector cells are selected from T cells, and the exogenous receptors are selected from chimeric antigen receptors (CARs), T cell receptors (TCRs), T cell fusion proteins (TFPs) and T cell antigen couplers (TACs).

In a preferred embodiment, the exogenous receptors are selected from chimeric antigen receptors (CARs); preferably, the chimeric antigen receptors have amino acid sequences shown in any one of SEQ ID NO: 11, 12, 13, 25, 26, 36, 38, or 52.

In a preferred embodiment, the immune effector cells are selected from T cells, NK cells, and NKT cells.

In a preferred embodiment, the exogenous receptor-modified immune effector cells specifically recognize tumor antigens, preferably, the tumor antigens are selected from CD19, CD20, CD22, CD30, Mesothelin, BCMA, EGFR, EGFRvIII, PSMA, Mucl, claudin18.2, GPC3, IL13RA2, SLAMF7, GPRC5D, and LILRB4; more preferably, the tumor antigens are selected from CD19, BCMA, and Claudin18.2.

In a preferred embodiment, the tumors are selected from gastric cancer, pancreatic cancer, gallbladder cancer, melanoma, non-small cell lung cancer (NSCLC), small cell lung cancer (SCLC), head and neck squamous cell carcinoma (HNSCC), classical Hodgkin lymphoma (cHL), primary mediastinal large B-cell lymphoma (PMBCL), urothelial carcinoma (UC), esophageal carcinoma, cervical carcinoma, hepatocellular carcinoma, Merkel cell carcinoma, renal cell carcinoma (RCC), colorectal cancer (mCRC), and breast cancer.

In a preferred embodiment, the tumors are selected from gastric cancer, non-small cell lung cancer (NSCLC), small cell lung cancer (SCLC), urothelial cancer (UC), liver cancer, and melanoma.

In a preferred embodiment, the subjects have not received cell therapies before administering the immune effector cells.

In a preferred embodiment, before administering the immune effector cells, the subjects also received drug treatments, surgical treatments, radiotherapies, or any combinations thereof.

In a preferred embodiment, the drug therapies comprise chemical drug therapies or biological drug therapies; preferably, the chemical drug therapies comprise Anlotinib, Apatinib, Lenvatinib, Fruquintinib, and Regorafenib.

In a preferred embodiment, the dose of cells in the cell therapy products administered per cycle does not exceed about 2×10⁹ cells/kg of body weight of the subject, or the dose of cells in the cell therapy products does not exceed about 1×10¹¹ cells/person. Preferably, the dose of immune effector cells administered per cycle does not exceed about 2×10⁸ cells/kg of body weight of the subject, or the dose of cells in a cell therapy products does not exceed about 1×10¹⁰ cells/person, more preferably, the dose of immune effector cells administered per cycle does not exceed about 2×10⁷ cells/kg of body weight of the subject, or the dose of cells in cell therapy products does not exceed about 1×10⁹ cells/person.

In a preferred embodiment, the dose of immune effector cells administered per cycle is about 1×10⁵ cells/kg of body weight of the subject to 2×10⁷ cells/kg of body weight of the subject, or about 1×10⁶ cells/kg of body weight of the subject to 2×10⁷ cells/kg of body weight of the subject; or the dose of immune effector cells administered per cycle is 1×10⁷ cells to 1×10⁹ cells, preferably, 1×10⁸ cells to 1×10⁹ cells, more preferably, 2.5×10⁸ cells to 5×10⁸ cells.

It should be understood that within the scope of the present application, the above-mentioned technical features of the present application and the technical features specifically described in the following (such asexamples) can be combined with each other to form new or preferred technical solutions. Due to space limitations, they will not be repeated here.

### BRIEF DESCRIPTION OF THE DRAWINGS

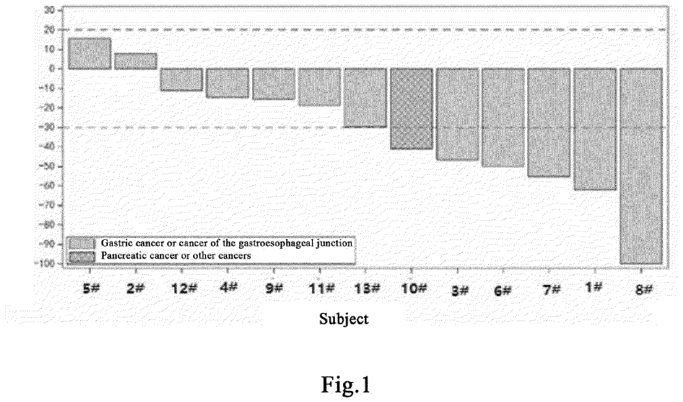

Figure 1 is a waterfall plot of the optimal change in tumor burden relative to the baseline.

### DETAIL DESCRIPTION OF THE APPLICATION

After extensive and intensive research, the inventors unexpectedly found that when anti-PD-1 antibody and/or anti-PD-L1 antibody are used in the previous treatment of cancer patients and the treatment failed (progressive disease PD according to RECIST 1.1), it will not affect the efficacy of cell therapy products, and at least 40% of the patients still respond to the cell therapy products. Exemplarily, patients with solid tumors like gastric cancer/esophagogastric junction adenocarcinoma, krukenberg tumor, liver cancer, and gallbladder cancer response to the cell therapy products(for example, CAR-T cells) after treatment failure with anti PD-1 antibodies and/or anti PD-L1 antibodies. Among patients with gastric cancer/esophagogastric junction adenocarcinoma who failed anti-PD-1 antibody and/or anti-PD-L1 antibody treatment, the disease control rate after CAR-T cell therapy reached about 79.2%. For liver cancer patients who failed anti-PD-1 antibody and/or anti-PD-L1 antibody treatment, the disease control rate after CAR-T cell therapy reached about 54.5%.

Unless otherwise defined, all technical terms, symbols and other technical and scientific terms or proprietary vocabularies used herein are intended to have the same meaning as commonly understood by those skilled in the art to which this application belongs. In some cases, terms with conventionally understood meanings are further defined herein for the purpose of clarification and/or ease of reference, and such further limitations comprised herein should not be construed as representing substantial differences from those conventionally understood in the art.

All publications, including patent documents, academic papers, and databases, mentioned in this application are independently incorporated by reference in their entirety. To the extent that a definition shown herein differs or is otherwise inconsistent with the definition shown in patents, published applications, and other publications incorporated herein by reference, the definition shown herein controls.

In this application, some claimed subject matter is presented in range format. It should be understood that the description in range format is merely for convenience and brevity and should not be construed as an inflexible limitation on the scope of the claimed subject matter. Accordingly, the description of a range should be considered to have specifically disclosed all the possible subranges as well as individual values within that range. For example, in a range provided with an upper and lower range limit, each intervening value between the upper and lower range limit is encompassed within the claimed subject matter, the upper and lower limits of the range also belong to the range of the claimed subject matter.

Any concentration range, percentage range, ratio range, or integer range recited herein is to be understood to comprise any integer within the stated range, and, where appropriate, fractions thereof (e.g., tenths and hundredths of the integers), unless otherwise indicated.

As used herein, the term "about" refers to the usual error range for each value readily known to those skilled in the art. Reference herein to "about" a value or parameter comprises (and describes) embodiments referring to the value or parameter itself. For example, description of "about X" comprises description of "X." For example, "about" can mean that the actual standard deviation in the art is within 1 or more than 1. Alternatively "about" can mean a range of up to 20%, or up to 10%, or up to 5%, or up to 1%. For example, about 5 mg can comprise any number between 4.5 mg and 5.5 mg. Where specific values or compositions are provided in the applications and claims, unless otherwise indicated, "about" should be assumed to be within an acceptable error range for that specific value or composition.

When describing amino acid or nucleic acid sequences, "having" a specific sequence in this patent should be understood as covering variants of the specific sequence. The amino acid or nucleic acid sequence mentioned in this patent has a specific sequence means that the amino acid or nucleic acid sequence has more than 80%, 81%, 82%, 83%, 84%, 85%, 86%, 87%%, 88%, 89%, 90%, 91%, 92%, 93%, 94%, 95%, 96%, 97%, 98%, or 99% sequence identity with the specific sequence. Sequence identity can be measured by sequence analysis software (e.g., programs such as BLAST, BESTFIT, GAP, PILEUP/PRETTYBOX, etc.). Such softwares match identical or similar sequences by assigning degrees of homology to various substitutions, deletions and/or other modifications. Conservative substitutions typically comprise substitutions within the following groups: glycine, alanine; valine, isoleucine, leucine; aspartic acid, glutamic acid, asparagine, glutamine; serine, threonine, lysine, arginine; and phenylalanine, tyrosine. In an exemplary method of determining the degree of identity, the BLAST program can be used, wherein a probability score between e-3 and e-100 indicates closely related sequences.

The "failure of anti-PD-1 antibody and/or anti-PD-L1 antibody treatment in previous treatment" as described herein means that the patient has received PD-1 and/or PD-L1 antibody treatment during the previous treatment, or patient has received therapy containing PD-1 and/or PD-L1 antibodies treatment, and the previous treatment cannot control the progression of the disease (for example, according to the Response Evaluation Criteria Version RECIST 1.1 of in Solid Tumors, the progression of the disease into disease progression (PD)).

The "therapy containing PD-1 and/or PD-L1 antibodies " described herein refers to the use of other drugs or therapies besides PD-1 and/or PD-L1 antibodies according to the needs of the treatment. For example, in addition to the use of PD-1 and/or PD-L1 antibodies, radiotherapy, stem cell transplantation, etc. are also used in the treatment; or in addition to the use of PD-1 and/or PD-L1 antibodies, other chemical or biological drugs, such as olaparib, lenvatinib, cabozantinib, axitinib, ipilimumab, platinum-based chemotherapy drugs, pemetrexed, etoposide, taxane compounds, bevacizumab, etc. or any combination thereof.

The treatment of cancer patients will be a long process. The so-called first-line, second-line, third-line, and later-line (including fourth-line, fifth-line, etc.) treatments actually refer to the selection and application sequence of tumor treatment regimens. First-line treatment generally refers to the first round of systemic chemotherapy and/or biological drug therapy for patients with unresectable tumors after tumor diagnosis; the first round of systemic chemotherapy and/or biological drug therapy for relapse for patients with resectable tumors after surgery (with or without adjuvant therapy). Second-line treatment generally refers to the need to change to a different treatment plan after failure of the first-line treatment (including the recurrence of tumor progression in patients after effective first-line treatment). Third-line treatment generally refers to switching to other treatment regimens after failure of the second-line treatment. Generally when most tumors reach the third-line treatment, there are fewer and fewer drugs and effective treatment regimens available.

PD1 and/or PD-L1 antibody therapy has been approved for different lines of treatment in different tumors. Now clinically, PD1 and/or PD-L1 antibody therapy is administered to patients with PD-L1 positive or negative tumors, In first-line, second-line, third-line, and later-line (including fourth-line, fifth-line, etc.) of tumor treatment, there are clinical applications of PD1 and/or PD-L1 antibody therapy alone, or PD1 and/or PD-L1 antibody therapy in combination with other drugs.

Existing treatment regimens for gastric cancer, such as the objective remission rate of administration of chemotherapy drugs (taxanes, irinotecan, etc.), targeted drugs (apatinib) and/or immune checkpoint inhibitors (Nivolumab) is about 2%-11.2%, mPFS is about 2-3 months, and mOS is about 6 months. The present application is aimed at administering cell therapy products (exemplary, CAR-T cells) in the second-line, third-line or later-line treatment of patients with gastric cancer/esophagogastric junction adenocarcinoma who have failed PD-1 and/or PD-L1 antibody therapy, showing a higher remission rate of about 50%, a disease control rate of about 79.2%, and a longer progression-free survival and overall survival, enabling the patients to obtain significant clinical benefits: among the 12 patients who achieved PR after CAR-T treatment, the mDOR of the subject is 6.3 months; among them, the overall survival of 6 patients reached about more than 11 months, and 4 of them are still in the survival follow-up period. The present application also observed that in patients with gastric cancer/esophagogastric junction adenocarcinoma who have failed with PD-1 and/or PD-L1 antibody therapy, the treatment effect is evaluated as complete remission (CR) after administration of CAR-T cell therapy, and are under continued clinical observing.

Krukenberg tumor is a kind of metastatic malignant ovarian cancer mainly originating from the stomach and intestinal tract. It is highly malignant and lacks specific clinical features. It is often found to be accompanied by systemic metastasis, and often combined with ascites and diffuse peritoneal metastasis, is insensitive to chemotherapy, poor treatment effect and poor prognosis. The present application administers cell therapy products (exemplary, CAR-T cells) to patients with Krukenberg tumors who have failed treatment with PD-1 and/or PD-L1 antibodies, showing longer progression-free survival and overall survival, resulting in significant clinical benefits for patients: PR has been achieved after CAR-T cell therapy, the disease progression-free survival period has exceeded 4 months, and is still in remission.

The results of published clinical studies have shown that the progression-free survival of gallbladder cancer who have previously received first-line standard treatment is less than about 6 months (1.8-5.6 months). The present application administers cell therapy products (exemplary, CAR-T cells) to patients with gallbladder cancer who have failed PD-1 and/or PD-L1 antibody therapy, showing longer progression-free survival and overall survival, allowing the patients to obtain significant clinical benefits: CAR-T cell therapy has reached SD, and the disease progression-free survival period has reached 7.5 months, and is still in a stable period.

Primary liver cancer is currently the fourth most common malignant tumor and the second leading cause of cancer death in the country. Because most of the patients are in the advanced stage of disease and the degree of tumor deterioration is high, the treatment effect is not ideal. The present application administers cell therapy products (exemplary, CAR-T cells) to patients with liver cancer who have failed PD-1 and/or PD-L1 antibody treatment, showing longer progression-free survival and overall survival, allowing the patients to obtain significant clinical benefits: the disease control rate after CAR-T cell therapy is about 54.5%; the average progression-free survival period of the 6 patients who achieved disease control is about 4.4 months, and the overall survival of 4 patients exceeded 1 year, 3 cases of the 4 patients are still in the survival follow-up period.

The "cell therapy products" described herein refer to cell products with medical therapeutic effects, such as immune cell therapy products, which is a product that uses the body's autologus or donor-derived immune cells to treat diseases through in vitro operations, including but not limited to separation, purification, cultivation, expansion, induction of differentiation, activation, genetic manipulation, establishment of cells (lines), cryopreservation and recovery, etc., and then infused (or implanted) into the patients to treat diseases to induce, enhance or inhibit the body's immune function. Including Adoptive cell therapy (ACT) products, therapeutic vaccines, etc. The types of immune cell therapy products mainly comprise but are not limited to Cytokine-Induced Killer (CIK), Tumor Infiltrating Lymphocyte (TIL), Chimeric Antigen Receptor T-cell (CAR-T), T Cell Receptor-engineering T-cell (TCR-T), Natural Killer (NK) cells, Dendritic cell (DC), macrophages, etc.

The term "anti-PD-1 antibody" refers to an antibody capable of recognizing or binding to PD-1, for example, an antibody capable of recognizing or binding to human PD-1 (extracellular segment SEQ ID NO: 39). The "antibody recognizing or binding to human PD-1" may specifically bind to human PD1 antigen with a binding affinity of KD value of 1.0×10⁻⁸ mol/l or lower. In one embodiment, the anti-PD-1 antibody may be Nivolumab, Pembrolizumab, Cemiplimab, Camrelizumab, Toripalimab, Sintilimab, Tislelizumab, or any combination thereof.

The term "anti-PD-L1 antibody" refers to an antibody capable of recognizing or binding to PD-L1, for example, an antibody capable of recognizing or binding to human PD-L1 (extracellular segment SEQ ID NO: 40). The "antibody recognizing or binding to human PD-L1" may specifically bind to human PD-L1 antigen with a binding affinity of KD value of 1.0×10⁻⁸ mol/l or lower. In one embodiment, the anti-PD-L1 antibody may be Atezolizumab, Durvalumab, Avelumab, sugemalimumab (CS1001) or any combination thereof.

The term "bind" refers to selectively binding a target. Tumor antigens can be recognized by immune effector cells expressing receptors that bind to tumor antigens.

"Specific binding" means that a polypeptide or fragment thereof recognizes and binds to a biomolecule of interest (e.g., a polypeptide) but does not substantially recognize and bind to other molecules in a sample.

Binding affinity can be determined using standard binding assays, such as surface plasmon resonance technique (GE-Healthcare, Uppsala, Sweden).

The "dose" described herein may be a dose calculated on a weight basis, or a dose calculated on a body surface area (BSA) basis, or a dose calculated on an individual person basis. The dose calculated on the basis of weight is the dose administered to the patient calculated on the basis of the patient's body weight, such as mg/kg, cell number/kg, etc. The dose calculated on the basis of BSA is the dose administered to the patient calculated on the basis of the surface area of the patient, such as mg/m², cell number/m², etc. The dose calculated on the basis of an individual refers to the dose administered per cycle or each time for each patient, such as mg/person, cell number/person.

"Number of administrations" as used herein refers to the frequency with which the cell therapy product or each pretreatment drug is administered per cycle. For example, fludarabine can be administered once a day for 4 consecutive days, once a day for 3 consecutive days, once a day for 2 consecutive days, or once a day. Cyclophosphamide can be administered once a day for 4 consecutive days, once a day for 3 consecutive days, once a day for 2 consecutive days, or once a day. Nab-paclitaxel can be administered once a day for 4 consecutive days, once a day for 3 consecutive days, once a day for 2 consecutive days, or once a day. Exemplarily, the cell therapy product administered in each cycle can be administered once; or divided into 2, 3, 4, 5, 6, 7, 8, 9, 10 administrations, can be administered on consecutive days, or at intervals of 1, 2, 3, 4, 5, 6 days.

The term "responding" or "responsive" described herein refers to according to the Response Evaluation Criteria in Solid Tumors Version 1.1 (RECIST1.1): a complete remission CR (all target lesions disappear, and the short diameter of all pathological lymph nodes must be reduced to < 10 mm); or partial remission PR: the sum of the diameters of target lesions is reduced by at least 30% compared with the baseline level; or stable disease (SD): the degree of reduction of the target lesions does not reach the PR level, and the degree of increase does not reach the level of PD, between the two, during the study, the minimum value of the sum of the diameters can be used as a reference. According to the Response Evaluation Criteria in Solid Tumors Version 1.1 (RECIST1.1), it is evaluated as progressive disease (PD): taking the minimum value of the sum of the diameters of all target lesions measured during the entire experimental study as a reference, the relative increase in the sum of the diameters is at least 20% (if the baseline measurement value is the smallest, the baseline value is used as a reference); in addition, the absolute value of the sum of the diameters must increase at least 5 mm (the appearance of one or more new lesions is also considered PD). The objective remission rate (ORR) is defined as the percentage of subjects with the best response to confirmed CR or PR in the analysis population, and the disease control rate (DCR) is defined as percentage of cases with confirmed best efficacy in remission (CR+PR) and stable disease (SD) in the analysis set. Progression-free survival (PFS) is defined as the time from the first dose of the treatment method of this application to the first appearance of disease progression or death due to any cause. Subjects who had neither disease progression nor died by the data cutoff date will be removed on the date of their last adequate tumor assessment. PFS will be summarized using descriptive statistics (mean, standard deviation, median, minimum and maximum) for the treated population. Except for the median calculated using the Kaplan-Meier method based on both observed and deleted values, all other statistics (mean, standard deviation, minimum and maximum) will be calculated only based on observed values. Overall survival (OS) is measured as the time from the first dose of the treatment method of the present application to death due to any cause and will be analyzed in a manner similar to that described for PFS.

In some embodiments, "responsive" comprises appearance of CR or PR after treatment with the therapeutic methods of the present application. In some embodiments, "responsive" comprises appearance of CR, PR or SD after treatment with the treatment method of the present application; in some embodiments, "responsive" means that subjects receiving the treatment method of the present application can obtain obvious clinical benefits, PFS and/or OS is significantly prolonged, such as prolonged by 2 weeks, 3 weeks, 4 weeks, 1 month, 2 months, 3 months, 4 months, 5 months, 6 months or longer. In some embodiments, "responsive" refers to an ORR reaching 10%, 15%, 20%, 25%, 30%, 35%, 40%, 45%, 50%, 55%, 60%, 65%, 70%, 75%, 80%, 85%, 90%, 95% or more, or 100%. In some embodiments, "responsive" refers to a DCR reaching 10%, 15%, 20%, 25%, 30%, 35%, 40%, 45%, 50%, 55%, 60%, 65%, 70%, 75%, 80%, 85%, 90%, 95% or more, or 100%.

The term "composition" refers to a mixture of two or more substances. It can be a solution, suspension, liquid, powder, paste, aqueous, non-aqueous, or any combination thereof.

The cells or cell populations described herein are "positive" for a certain marker, meaning that the presence of a certain marker (usually a surface marker) of the cell can be proved by detection, such as the marker is detected to have surface expression by flow cytometry, for example, by staining with an antibody that specifically binds to the marker, and detecting the antibody, wherein the staining is detectable at a level by flow cytometry, the level is significantly higher than the level of staining detected by the control using homotypic matching under the same procedure under other equal conditions, and/or is substantially similar to the level of cells known to be positive for the marker, and and/or is significantly higher than the level of cells known to be negative for the marker. In specific embodiments, the markers comprise but are not limited to tumor antigens. Exemplary, patients with gastric cancer/esophagogastric junction adenocarcinoma, krukenberg tumor, liver cancer, and gallbladder cancer who are positive for markers (tumor antigen CLDN18.2 or GPC3) receive CAR-T cell therapy after failure of PD-1 and/or PD-L1 antibody treatment.

As used herein, a cell or cell population that is "negative" for a marker means that the cells do not have a marker or cannot be detected despite having it, such as the surface expression of the marker is not detectable by flow cytometry, or although surface expression is detectable (for example, when surface expression is detected by staining with an antibody that specifically binds to the marker and detecting the antibody, the staining can be detected by flow cytometry at a certain extent), the level is significantly lower than the level of staining detected by the control using homotypic matching when the same procedure is performed under the same other conditions, and/or significantly lower than the level known to be positive for the marker and/or substantially similar to the level of cells known to be negative for the marker. In a specific embodiment, for patients whose tumor cell marker PD-L1 is positive or negative, CAR-T cell therapy can be administered after previous failure treatment of anti-PD-1 antibody and/or anti-PD-L1 antibody.

The term "vector" as used herein is a composition comprising an isolated nucleic acid and which can be used to deliver the isolated nucleic acid to the interior of a cell. Many vectors are known in the art, including but not limited to linear polynucleotides, polynucleotides associated with ionic or amphiphilic compounds, plasmids and viruses. Thus, the term "vector" comprises autonomously replicating plasmids or viruses. Non-plasmid and non-viral compounds that facilitate the transfer of nucleic acids into cells may also be comprised, such as polylysine compounds, liposomes, and the like.

The term "cell" may refer to a human or non-human cell of animal origin.

"Subject" and "patient" are used interchangeably herein to refer to an individual, such as a human or other animal, and usually a human being, to be treated with a cell product. Before entering the cell therapy study, the subject has been treated with PD-1 antibody and/or PD-L1 antibody, and is responsive or non-responsive to PD-1 antibody or PD-L1 antibody.

The term "immune effector cells" refers to cells that participate in the immune response and produce immune effects, such as T cells, B cells, natural killer (NK) cells, natural killer T (NKT) cells, dendritic cells, CIK cells, macrophages, mast cells, or stem cell-derived immune effector cells. In some embodiments, the immune effector cells are T cells, NK cells, NKT cells. In some embodiments, the T cells may be natural T cells and/or T cells induced by pluripotent stem cells. In some embodiments, the T cells may be autologous T cells, heterologous T cells, allogeneic T cells. In some embodiments, the NK cells can be autologous NK cells or allogeneic NK cells.

The term "artificially modified cell with immune effector cell function" refers to a cell without immune effector, or a cell that obtains the function of immune effector cell after being artificially modified or stimulated by a stimulant. For example, 293T cells are artificially modified to have the function of immune effector cells; for example, stem cells are induced in vitro to differentiate into immune effector cells.

In some instances, "T cells" may be pluripotent stem cells derived from bone marrow that differentiate and mature into immunocompetent mature T cells within the thymus. In some cases, a "T cell" may be a cell population with specific phenotypic characteristics, or a mixed population of cells with different phenotypic characteristics, such as a "T cell" may be a cell comprising at least one subpopulation of T cells: stem cell-like memory T cells (Tscm cells), central memory T cells (Tcm), effector T cells (Tef, Teff), regulatory T cells (tregs) and/or effector memory T cells (Tern). In some cases, "T cells" may be a specific subtype of T cells, such as γδ T or αβ T cells.

T cells can be obtained from many sources including, but not limited to, PBMC (peripheral blood mononuclear cells), bone marrow, lymph node tissue, cord blood, thymus tissue, and tissue from sites of infection, ascites, pleural effusion, spleen tissue, and tumors. In some cases, T cells can be obtained from blood collected from an individual using any number of techniques known to those skilled in the art, such as Ficoll(TM) isolation. In one embodiment, the cells from the circulating blood of the individual are obtained by apheresis. Apheresis products usually contain lymphocytes, including T cells, monocytes, granulocytes, B cells, other nucleated white blood cells, red blood cells, and platelets. In one embodiment, cells collected by apheresis can be washed to remove plasma molecules and placed in a suitable buffer or culture medium for subsequent processing steps. Alternatively, cells can be derived from healthy donors or from patients diagnosed with cancer. T cells can be of any type and of any developmental stage, including but not limited to CD4+/CD8+ double positive T cells, CD4+ helper T cells such as Th1 and Th2 cells, CD8+ T cells (e.g. cytotoxic T cells), tumor infiltrating cells, memory T cells, naive T cells, etc.

The term "exogenous" as used herein refers to a nucleic acid molecule or polypeptide, cell, tissue, etc. that is not expressed endogenously in the organism itself, or the expression level is insufficient to achieve the function of overexpression.

The term "endogenous" means that a nucleic acid molecule or polypeptide etc. is derived from the organism itself.

The term "chimeric receptor" refers to a fusion molecule formed by linking DNA fragments from different sources or corresponding cDNA or polypeptide fragments of proteins by genetic recombination technology, including extracellular domains, transmembrane domains and intracellular domains. Chimeric receptors comprise, but are not limited to: Chimeric Antigen Receptor (CAR), Chimeric T Cell Receptor (TCR), T Cell Antigen Coupler (TAC).

The term "chimeric antigen receptor" (CAR) comprises an extracellular antigen binding domain, a transmembrane domain and an intracellular signaling domain. In specific embodiments, the extracellular binding domain of CAR comprises a scFv. Intracellular signaling domains comprise functional signaling domains of stimulatory and/or co-stimulatory molecules; or comprise all intracellular portions of stimulatory and/or co-stimulatory molecules, or all native intracellular signaling domains, or functional fragments or derivatives thereof. In one aspect, the stimulatory molecule comprises a CD3ζ chain bound to the T cell receptor complex; in one aspect, the intracellular signaling domain further comprises one or more functional signaling domains of co-stimulatory molecules, e.g. 4-1BB (i.e. CD137), CD27 and/or CD28. In certain embodiments, groups of polypeptides are linked to each other. Exemplarily, the extracellular antigen-binding domain of the CAR comprises the sequence shown in SEQ ID NO: 14, 24, 35, 37, 58, 59, 60, 61, 62, 63, 64, 65, 66, 67, 70, 71, 72, 73, 74, 75, 76, 77, 78 or 79.

The term "T cell receptor (TCR)" mediates T cell recognition of specific major histocompatibility complex (MHC)-restricted peptide antigens, including natural TCR receptors and modified TCR receptors. Natural TCR receptor consists of two peptide chains α and β, and each peptide chain can be divided into a variable region (V region), a constant region (C region), a transmembrane region and a cytoplasmic region, etc., and its antigen-specificity exists in the V region, and the V region (Vα, Vβ) each has three hypervariable regions CDR1, CDR2, and CDR3. In a specific embodiment, the modified TCR extracellular antigen-binding domain comprises an antibody recognizing a tumor antigen. Exemplarily, the TCR comprises the sequence shown in SEQ ID NO: 14, 24, 35, 37, 58, 59, 60, 61, 62, 63, 64, 65, 66, 67, 70, 71, 72, 73, 74, 75, 76, 77, 78 or 79.

The term "TCR fusion protein" or "TFP" comprises recombinant proteins derived from various proteins of TCR, comprising an antigen binding domain, generally capable of: i) binding to a surface antigen on a target cell; ii) interacting with other peptide components of the intact TCR complex when located on T cells. In some embodiments, the T cells are CD4+ T cells, CD8+ T cells, or CD4+/CD8+ T cells. In some embodiments, the TFP T cells are NKT cells. In some embodiments, the TFP T cells are γδ T or αβ T cells. In a specific embodiment, the antigen-binding domain of TFP comprises an antibody recognizing a tumor antigen. Exemplarily, the TFP comprises the sequence shown in SEQ ID NO: 14, 24, 35, 37, 58, 59, 60, 61, 62, 63, 64, 65, 66, 67, 70, 71, 72, 73, 74, 75, 76, 77, 78 or 79.

The term "T cell antigen coupler (TAC)" comprises three functional domains: 1. antigen binding domain, including single-chain antibody, designed ankyrin repeat protein (DARPin) or other targeting groups; 2. the extracellular region domain, the single-chain antibody that binds to CD3, so that the TAC receptor and the TCR receptor are close; 3. the transmembrane region and the intracellular region of the CD4 co-receptor, wherein, the intracellular domain links to protein kinase LCK, catalyzes the phosphorylation of immunoreceptor tyrosine-activating motifs (ITAMs) of the TCR complex as an initial step in T cell activation. In a specific embodiment, the TAC antigen-binding domain comprises an antibody recognizing a tumor antigen. Exemplarily, the TAC comprises the sequence shown in 14, 24, 35, 37, 58, 59, 60, 61, 62, 63, 64, 65, 66, 67, 70, 71, 72, 73, 74, 75, 76, 77, 78 or 79.

The term "antigen-binding domain" refers to an immunoglobulin molecule (used interchangeably with "antibody") or an immunologically active portion of an immunological molecule, i.e., molecules that contain antigen-binding sites that specifically bind to antigens ("immune reactions"). The molecules can contain antibodies, such as fully human antibodies, chimeric antibodies, humanized antibodies, murine antibodies, or ligands that recognize antigens.

The term "antibody" is used herein in the broadest sense and comprises various antibody structures including, but not limited to, monoclonal antibodies, polyclonal antibodies, multispecific antibodies (e.g., bispecific antibodies), and antibody fragments, so long as they show the desired antigen-binding activity. The term "antibody" herein refers to an antigen-binding protein of the immune system. An "antibody" comprises an intact full-length antibody having an antigen-binding region and any fragment thereof in which the "antigen-binding portion" or "antigen-binding region" is retained, or a single chain thereof, such as a single-chain variable fragment (scFv). Each heavy chain is composed of a heavy chain variable region (abbreviated herein as VH) and a heavy chain constant region. Each light chain is composed of a light chain variable region (abbreviated herein as VL) and a light chain constant region. The term "variable region or variable domain" refers to the domains of an antibody heavy or light chain that participate in antibody antigen binding. The variable regions of the heavy and light chains contain binding domains that interact with the antigen.

Antibody fragments comprise, but are not limited to: (i) Fab fragments consisting of VL, VH, CL, and CH1 domains, including Fab' and Fab'-SH, (ii) Fd fragments consisting of VH and CH1 domains, (iii) Fv fragments consisting of the VL and VH domains of a single antibody; (iv) dAb fragments consisting of a single variable domain (Ward et al., 1989, Nature 341:544-546); (v) F(ab')2 fragments, a bivalent fragment comprising two linked Fab fragments; (vi) antigen-binding site of single-chain Fv molecule (Bird et al., 1988, Science 242:423-426; Huston et al., 1988, Proc. Natl. Acad. Sci. U.S.A 85:5879-5883); (vii) bispecific single chain Fv dimer (PCT/US92/09965); (viii) "dibody" or "tribody", multivalent or multispecific fragments constructed through gene fusion (Tomlinson et al., 2000, Methods Enzymol. 326:461-479; WO94/13804; Holliger et al., 1993, Proc. Natl. Acad. Sci. U.S.A 90:6444-6448); and (ix) scFv genetically fused to the same or a different antibody (Coloma & Morrison, 1997, Nature Biotechnology 15, 159-163).

In particular embodiments, the antigen binding domain may comprise a scFv, Fv, Fab, Fab', Fab'-SH, F(ab')2, a single domain fragment, or a natural ligand that binds the antigen, and any derivative thereof. In some aspects, an extracellular antigen binding domain (e.g., scFv), can comprise light chain CDRs and/or heavy chain CDRs specific for an antigen.

In a preferred embodiment, the antibody or fragment thereof contained in the antigen-binding domain may have the following CDR sequences: HCDR1 shown in SEQ ID NO: 1, HCDR2 shown in SEQ ID NO: 2, and HCDR3 shown in SEQ ID NO: 3, LCDR1 shown in SEQ ID NO: 4, LCDR2 shown in SEQ ID NO: 5, LCDR3 shown in SEQ ID NO: 6; or HCDR1 shown in SEQ ID NO: 16, HCDR2 shown in SEQ ID NO: 17, HCDR3 shown in SEQ ID NO: 18, LCDR1 shown in SEQ ID NO: 19, LCDR2 shown in SEQ ID NO: 20, LCDR3 shown in SEQ ID NO: 21; or HCDR1 shown in SEQ ID NO: 27, HCDR2 shown in SEQ ID NO: 28, HCDR3 shown in SEQ ID NO: 29, LCDR1 shown in SEQ ID NO: 30, LCDR2 shown in SEQ ID NO: 31, LCDR3 shown in SEQ ID NO: 32.

In a preferred embodiment, the antibody or fragment thereof contained in the antigen-binding domain may have the following heavy chain variable region and light chain variable region sequences: the heavy chain variable region shown in SEQ ID NO: 7 and the light chain variable region shown in SEQ ID NO: 9; or the heavy chain variable region shown in SEQ ID NO: 22 and the light chain variable region shown in SEQ ID NO: 23; or the heavy chain variable region shown in SEQ ID NO: 33 and the light chain variable region shown in SEQ ID NO:34.

In specific embodiments, the antibody or fragment thereof contained in the antigen binding domain may have the scFv sequence shown in SEQ ID NO: 58, 59, 60, 61, 62, 63, 64, 65, 66, 67, 70, 71, 72, 73, 74, 75, 76, 77, 78 or 79.

In some cases, the extracellular region of the CAR comprises an antigen-binding domain that recognizes a tumor antigen and a linking fragment (also known as hinge, spacer). The linking fragment can be considered as part of the CAR used to provide flexibility to the extracellular antigen-binding domain. Linking fragments can be of any length. Exemplarily, in one embodiment, the CAR comprises a hinge domain, exemplary, a CD8α hinge, represented by SEQ ID NO:41.

The term "transmembrane domain" may anchor an exogenous receptor to the plasma membrane of a cell. For example, the transmembrane domain of CD28 or CD8, having the sequence shown in SEQ ID NO:42 or 43 can be used. The term "signaling domain" refers to a functional portion of a protein that functions by transmitting information within a cell, used to regulate cell activity through a determined signal transduction pathway by producing a second messenger or by acting as an effector in response to such a messenger. The intracellular signaling domain may comprise the entire intracellular portion of the molecule, or the entire native intracellular signaling domain, or a functional fragment or derivative thereof. Exemplarily, the intracellular signaling domain has the sequence shown in SEQ ID NO:44, 45, or 46.

The functional signaling domain of the stimulatory molecule, also called the "primary signaling domain", regulates the initial activation of the CAR or TCR complex in a stimulatory manner. In one aspect, the primary signaling domain is elicited by, for example, the binding of a TCR/CD3 complex to a peptide-loaded MHC molecule, thereby mediating a T cell response (including, but not limited to, proliferation, activation, differentiation, etc.). Primary signaling domains acting in a stimulatory manner may comprise immunoreceptor tyrosine activation motifs or signaling motifs of ITAMs. Examples of ITAM-containing primary signaling domains that are particularly useful in this application comprise, but are not limited to, those sequences derived from CD3ζ, FcRy, FcRβ, CD3y, CD36, CD3ε, CD5, CD22, CD79a, CD79b, CD278 (also known as "ICOS") and CD66d.

The term "co-stimulatory molecule" refers to a signal that binds to a cell-stimulating signal molecule, such as TCR/CD3, and causes T cell proliferation and/or up-regulation or down-regulation of key molecules, and has a sequence as shown in SEQ ID NO:44 or 45. It is a cognate binding partner on a T cell that specifically binds a co-stimulatory ligand, thereby mediating a co-stimulatory response of the T cell, including but not limited to cell proliferation. Co-stimulatory molecules are non antigen receptor cell surface molecules or their ligands that are required for effective immune response. Co-stimulatory molecules comprise, but are not limited to, MHC class I molecules, BTLA and Toll ligand receptors, and OX40, CD2, CD27, CD28, CDS, ICAM-1, LFA-1 (CD11a/CD18) and 4-1BB (CD137).

In some cases, intracellular signaling domains can be designed to contain several potential stimulatory molecules and/or co-stimulatory signaling domains: exemplarily, the first-generation CARs contain a single stimulatory domain, such as the CD3ζ chain; the second-generation CAR contains a co-stimulatory domain, such as CD3ζ chain and CD28, or contains CD3ζ chain and 4-1BB; the third-generation CAR contains two co-stimulatory domains (the third-generation CAR), such as contains CD3ζ chain and CD28 and OX40, or contains the CD3ζ chain with CD28 and 4-1BB.

In a specific embodiment, the CAR comprises an optional leader sequence. In one aspect, the CAR further comprises a leader sequence at the N-terminus of the extracellular antigen recognition domain, wherein the leader sequence is optionally cleaved from the antigen recognition domain (e.g., scFv) during the cellular processing and localization of the CAR to the cell membrane. In one embodiment, the leader sequence comprises a sequence shown in SEQ ID NO:53.

In a specific embodiment, the antigen-binding domain targeting GPC3-CAR of the present application comprises the sequence shown in SEQ ID NO: 70, 71, 72, 73, 74, 75, 76, 77, 78 or 79, and the transmembrane domain comprises the sequence shown in SEQ ID NO: 42 or 43, the intracellular signaling domain comprises the sequences shown in SEQ ID NO: 44 and 46, or SEQ ID NO: 45 and 46, or SEQ ID NO: 45, 44 and 46. Exemplarily, the administration of the GPC3-CAR-T cells to patients with liver cancer who failed PD1 and/or PD-L1 antibody treatment showed longer progression-free survival and overall survival, which brought significant clinical benefits to the patients. In the embodiments of the present application, the GPC3-CAR-T cells and the transcription factor RUX3 polypeptide are administered to patients with liver cancer who failed PD1 and/or PD-L1 antibody treatment, and some patients are evaluated as PR after treatment, showing significant clinical benefit. In specific embodiments, the GPC3-CAR-T expresses a RUNX3 polypeptide.

The term "RUNX3 (Runt-related transcription factor 3)", also known as osteogenesis-related transcription factor 3, is a member of the transcription factor runt domain family, with the amino acid sequence of GenBank Accession No. NP_004341.1. It plays an important role in cell growth, development, apoptosis, signal transduction and its biological effects.

The terms "activation" and "activating" are used interchangeably and can refer to the process by which a cell transitions from a quiescent state to an active state. The process can comprise responses to antigenic, phenotypic or genetic changes in migration and/or functional activity status. For example, the term "activation" may refer to the gradual activation process of T cells. The activation process is jointly regulated by the first stimulatory signal and co-stimulatory signal. "T cell activation" or "T cell activating" refers to the state of a T cell that is stimulated to induce detectable cell proliferation, cytokine production, and/or detectable effector function. Using CD3/CD28 magnetic beads, in vitro antigen stimulation or in vivo antigen stimulation will affect the degree and duration of T cell activation. In one embodiment, the engineered T cells are co-incubated with tumor cells containing a specific target antigen or activated after virus infection.

The term "treatment" refers to interventions in an attempt to alter the course of a disease, to alleviate or reduce, in whole or in part, a disease or symptoms associated therewith. Said therapeutic effects comprise, but are not limited to, preventing the occurrence or recurrence of the disease, relieving symptoms, reducing any direct or indirect pathological consequences of the disease, preventing metastasis, slowing down the rate of disease progression, ameliorating or alleviating the disease state, and alleviating or improving the prognosis. As used herein, "delaying the development of a tumor" means postponing, hindering, retarding, slowing, stabilizing, suppressing and/or delaying the development of said tumor. This delay can be of varying lengths of time, depending on the disease history and/or the individual to be treated. It will be appreciated by those skilled in the art that a sufficient or significant delay may encompass prophylaxis in individuals who do not develop said tumor. For example, advanced cancers, such as the development of metastases, can be delayed.

In some embodiments, the cell therapy products provided are used to delay tumor development or slow tumor progression.

As used herein, "inhibit" refers to the reduction of function or activity when compared to the otherwise identical condition or compared to another condition.

"Effective amount" or "therapeutically effective amount" refers to a dose sufficient to prevent or treat the disease (cancer) in an individual. Effective doses for therapeutic or prophylactic use will depend on the stage and severity of the disease to be treated, the age, weight and general health of the subject, and the judgment of the prescribing physician. The size of the dose will also depend on the active substance chosen, the method of administration, the time and frequency of administration, the existence, nature and extent of adverse side effects that may accompany the administration of the particular active substance, and the desired physiological effect. One or more rounds, or multiple administrations of the cell therapy products described herein may be required according to the judgment of the prescribing physician or those skilled in the art.

The term "tumor antigen" refers to an antigen emerging or overexpressed during the onset, progression of a hyperproliferative disease. In certain aspects, a hyperproliferative disorder of the application refers to cancer (including tumors). The tumor antigens described in the present application may be solid tumor antigens or blood tumor antigens.

The term "cycle" refers to the period between the baseline period (i.e. the evaluation period) before receiving treatment and the baseline period before the next treatment for each cell therapy; for example, from the administration of pretreatment drug treatment to before the start of the next cycle of pretreatment drug treatment.

The term "pretreatment" refers to administrating other drugs or treatments to the patient before administrating the product (such as a cell therapy product), so that the patient's physical condition is more suitable for the product treatment. For example, previous to the administration of cell therapy products (such as CAR-T cells), pretreatment with chemical drugs, biological drugs, radiotherapy or any combination thereof is performed. Preferably, chemical drugs are administered for pretreatment, and more preferably, the chemical drugs are chemotherapeutic drugs.

In some embodiments, pretreatment is required before administering the cell therapy product in each cycle.

In some embodiments, after pretreatment, the patient's lymphocytes are reduced about 50%, 55%, 60%, 65%, or 70% compared to that before pretreatment.

The term "chemical medicine" or "chemical drug" refers to an anti-cancer drug prepared by chemical synthesis, which can be a traditional chemotherapeutic drug, such as an alkylating agent, an anti-metabolite, an anti-cancer antibiotic, etc., or a targeted drug , such as afatinib, alectinib, etc.

The term "biological medicine " or "biological drug" refers to a drug prepared by means of molecular biology or genetic engineering, such as antibody drug, ADC, etc.

The term "chemotherapeutic drug" refers to a drug that treats tumors. Chemotherapeutic drugs can kill tumor cells, or inhibit the growth, migration or invasion of tumor cells. These drugs can act on different stages of tumor cell growth and reproduction, inhibit or kill tumor cells. Treatment with chemotherapeutic drugs is one of the main means of treating tumors at present.

In some embodiments, the chemotherapeutic drugs described herein refer to drugs used in chemotherapy, and refer to chemical drugs that have preventive and therapeutic effects on malignant tumors. Chemotherapeutic drugs comprise but are not limited to alkylating agents, anti-metabolites, anti-tumor antibiotics, plant-based anti-cancer drugs, hormones, immune preparations, etc. For example, diterpene alkaloids (such as taxanes), cyclophosphamide, fludarabine, cyclosporine, rapamycin, melphalan, bendamustine, asparaginase, Busulfan, carboplatin, cisplatin, daunorubicin, doxorubicin, fluorouracil, hydroxyurea, methotrexate, rituximab, vinblastine and/or vincristine, etc. In some embodiments, the anti-metabolites comprise but are not limited to anti-metabolites such as carmofur, tegafur, pentostatin, doxifluridine, trimesat, fludarabine, Capecitabine, galocitabine, cytarabine sodium octadecyl phosphate, fosteabine sodium hydrate, raltitrexed, paltitrexid, emiterur, tiazofurin, nolatrexed, pemetrexed, nelzarabine, 2'-deoxy-2'-methylenecytidine, 2'-fluoromethylene-2'-deoxycytidine, N-[5-(2,3-dihydro-benzofuryl)sulfonyl]-N'-(3,4-dichlorophenyl)urea, N6-[4-deoxy-4-[N2-[2(E),4(E)-tetradecadienyl]glycylamino]-L-glycerol-B-L-mannose-heptopyranos yl] adenine, aplidine, sacidine, 4-[2-amino-4-oxo-4,6,7,8-tetrahydro-3H-pyrimido[5,4-b]thiazin-6-yl-(S)-ethyl]-2,5-thienoyl-L-gluta mate, Aminopterin, 5-fluorouracil, aranosine, 11-acetyl-8-(aminoformyloxymethyl)-4-formyl-6-methoxy-14-oxa-1,11-diazotetracyclo(7.4.1.0.0)-te tradec-2,4,6-trien-9-yl acetate, swainsonine, lometrexol, dexrazoxane, methioninase, 2'-cyano-2'-deoxy-N4-palmitoyl-1-B-D-arabinofuranosyl cytosine and 3-aminopyridine-2-aldehyde thiosemicarbazone, etc. In some embodiments, such alkylating agents comprise, but are not limited to, dacarbazine, melphalan, cyclophosphamide, temozolomide, chlorambucil, busulfan, nitrogen mustard, and nitrosoureas, etc.

### Use or method of cell therapy product

The present application provides the use or method of a cell therapy product for the treatment of cancer (used interchangeably with "tumor") which failed previous treatment with an anti-PD-1 antibody and/or an anti-PD-L1 antibody.

In some embodiments, at least 40% of patients who have failed previous treatment with an anti-PD-1 antibody and/or an anti-PD-L1 antibody respond to the cell therapy product.

In some embodiments, at least 50% of patients who have failed previous treatment with an anti-PD-1 antibody and/or an anti-PD-L1 antibody respond to the cell therapy product.

In some embodiments, the patient's ORR is greater than 40% after administration of the cell therapy product; preferably, greater than 50%.

For purposes herein, "dosage" refers to the amount of drug administered, e.g., the amount of a cell therapy product administered in a cycle, or the amount of a cell therapy product administered each time in a cycle, or in a cycle, the amount of pretreatment drug administered each time.

In some embodiments, the patient is treated with at least one cycle of the cell therapy product. In a specific embodiment, the dosage may be the same or different for each cycle.

In some embodiments, the dose in a cycle is administered to the subject once or in divided doses, and the divided doses for each administration may be the same or different. For example, the cell therapy product in one cycle is administered to the subject once, or divided into 2, 3, 4, 5 or more times.

In some embodiments, when the dose in one cycle is administered to the subject in multiple doses, the dose of the cell therapy product or pretreatment drug administered each time is determined by the doctor according to the specific conditions of the subject. The specific situation of the subject may be the overall health condition of the subject, the severity of the disease, the response to the previous dosage in the same cycle, the response to the previous cycle, the combined drug use of the subject, the degree or likelihood of toxic reactions, complication, cancer metastasis, and any other factors considered by physicians to affect the dose of cell therapy or pre-treatment drugs received by the subject. In some embodiments, the dose administered each time is in an increasing trend. In some embodiments, the dose administered each time is in a decreasing trend. In some embodiments, the dose administered each time tends to increase first and then decrease. In some embodiments, the dose administered each time tends to decrease first and then increase.

The multi-cycle administration of cell therapy products means that there are multiple time periods, and a certain total amount of cell therapy products is administered in each time period. In some embodiments, the intervals of each time period are consistent. In some embodiments, the intervals of each time period are inconsistent. In some embodiments, at least 2 cycles of the cell therapy product are administered, such as 2, 3, 4, 5, 6, 7, 8, 9, or 10 cycles of the cell therapy product are administered.

In some embodiments, the design of the dose of cells administered in each cycle and the number of administrations, and the interval between different cycles are evaluated with the goal of improving one or more outcomes, for example, the outcome may be a reduction in the degree or likelihood of side effects in the subject, or an improvement in treatment effectiveness.

In some embodiments, the dose of cells in the cell therapy product administered per cycle is not more than about 2×10⁹ cells/kg of the body weight of the subject, or the dose of cells in the cell therapy product is not more than about 1×10¹¹ cells/person. Preferably, the dose of cells in the cell therapy product administered per cycle is not more than about 2×10⁸ cells/kg of the body weight of the subject, or the dose of cells in the cell therapy product is not more than about 1×10¹⁰ cells/person. More preferably, the dose of cells in the cell therapy product administered per cycle is not more than about 2×10⁷ cells/kg of the body weight of the subject, or the dose of cells in the cell therapy product is not more than about 5×10⁹ cells/person, or 2×10⁹ cells/person, or 1×10⁹ cells/person, or 5×10⁸ cells/person.

In some embodiments, the dose of cells in the cell therapy product administered per cycle is about 1×10⁵ cells/kg of the body weight of the subject to 2×10⁷ cells/kg of the body weight of the subject, or about 1×10⁶ cells/kg of the body weight of the subject to 2×10⁷ cells/kg of the body weight of the subject.

In some embodiments, the dose of cells in the cell therapy product administered per cycle is about 1×10⁷ cells to 1×10⁹ cells, 1×10⁷ cells to 2×10⁹ cells, 1×10⁷ cells to 5×10⁹ cells, or 1×10⁷ cells to 1×10¹⁰ cells, preferably 1×10⁸ cells to 1×10⁹ cells, 1×10⁸ cells to 2×10⁹ cells, 1×10⁸ cells to 5×10⁹ cells, more preferably 1×10⁸ cells to 5×10⁸ cells, or 2.5×10⁸ cells to 5×10⁸ cells, 3.75×10⁸ cells to 5×10⁸ cells.

In some embodiments, before administering the cell therapy products in each cycle, patients are pretreated, which comprises administering a chemotherapeutic agent and/or radiotherapy to the subjects. In some embodiments, the radiotherapy may be whole body radiotherapy or localized radiotherapy.

In some embodiments, the pretreatment chemotherapeutic agent may be any one or at least two of cyclophosphamide, fludarabine, tubulin inhibitors, and pyrimidine antineoplastic drugs.

The tubulin inhibitors described herein comprise tubulin polymerization promoters and tubulin polymerization inhibitors. Tubulin inhibitors comprise, but are not limited to, taxanes, epothilones, spongolactones, and laulimalide, etc.

The term "taxanes drug" refers to a drug whose main component contains a taxane compound having a bridged methylenebenzocyclodecene core structure similar to a taxane. In some embodiments, the bridged methylenebenzocyclodecene core structure of the taxane compound contains unsaturated bonds. In some embodiments, the bridged methylenebenzocyclodecene core structure of the taxane compound does not contain unsaturated bonds. In some embodiments, the carbon atoms on the bridged methylenebenzocyclodecene core structure of the taxanes are replaced by heteroatoms selected from N, O, S, and P. In some embodiments, the administration method of the taxane compound is injection administration.

In some embodiments, the pretreatment chemotherapeutic agent may be cyclophosphamide and fludarabine; or cyclophosphamide, fludarabine, and a tubulin inhibitor.

In some embodiments, the tubulin inhibitor is a taxane compound, preferably, the taxane compound is selected from paclitaxel, nab-paclitaxel, docetaxel, more preferably, the taxane compound is nab-paclitaxel.

In some embodiments, the pyrimidine antineoplastic drug is selected from 5-fluorouracil, gimeracil, oteracil potassium, bisfururacil, carmofur, doxifluridine, and capecitabine.

In some embodiments, the amount of fludarabine to be administered is about 20-60 mg/day, about 30-55 mg/day, or about 30-50 mg/day.

In some embodiments, the amount of fludarabine to be administered is about 10-50 mg/m²/day, about 15-40 mg/m²/day, about 15-30 mg/m²/day, or about 20-30 mg/m²/day; or about 25mg/m²/day.

In some embodiments, the amount of cyclophosphamide to be administered is about 200-700 mg/day, about 300-600 mg/day, about 300-560 mg/day, about 300-550 mg/day, or about 300-500 mg/day.

In some embodiments, the amount of cyclophosphamide to be administered is about 200-400 mg/m²/day, about 200-300 mg/m²/day, or about 250 mg/m²/day.

In some embodiments, the dosage of the taxane compound is not more than about 300 mg/day, or not more than about 200 mg/day, preferably, the dosage of the taxane compound is about 90-200 mg/day, more preferably, the dosage of the taxane compound is about 90-120 mg/day.

In some embodiments, each chemotherapeutic agent is administered for no more than 4 consecutive days.

In some embodiments, cyclophosphamide is administered 2-3 times. In some embodiments, fludarabine is administered 1-2 times. In some embodiments, the taxane compound is administered once.

In some embodiments, the methods or uses provided herein are based on the applicant's observation of the subject's response to the cell therapy product administered and the outcome of the treatment. For example, administering different doses or different numbers of cycles of CAR-T cells targeting CLDN18A2 to subjects with CLDN18A2-positive cancers who have failed with previous anti-PD-1 antibodies and/or anti-PD-L1 antibodies treatment, CAR-T cells can still show better therapeutic outcomes. For example, administering different doses or different numbers of cycles of CAR-T cells targeting GPC3 to subjects with GPC3-positive cancers who have failed with previous anti-PD-1 antibodies and/or anti-PD-L1 antibodies treatment, CAR-T cells can still show better therapeutic outcomes. For example, administering different doses or different numbers of cycles of RUNX3-expressing GPC3-CAR-T cells to subjects with GPC3-positive cancers who have failed with previous anti-PD-1 antibody and/or anti-PD-L1 antibody treatment, CAR-T cells still can show good treatment results. For example, such treatment outcomes comprise survival, remission, or stabilization of the patient's condition.

In some embodiments, the use or method described herein further comprises monitoring the degree or risk of the subject's toxic response to the cell therapy product, and determining the subsequent divided administration or the administration dose and interval of the following cycle according to the degree or risk of the toxicity. In some embodiments, the degree or risk of toxic response comprises, but is not limited to, CRS, neurotoxicity, macrophage activation syndrome, tumor lysis syndrome, and the like.

In some embodiments, when the risk of a toxic response or its symptoms or biochemical indicators (such as CRS or neurotoxicity, macrophage activation syndrome or tumor lysis syndrome) is at or below the acceptable levels after administration of the cell therapy product, treatment is administered in a subsequent cycle. In some embodiments, the toxic response or its symptoms or biochemical indicators comprise one or more of fever, hypotension, hypoxia, neurological disorders, inflammatory cytokines, serum levels of C-reactive protein (CRP). In some embodiments, the acceptable level of risk of the toxic response or its symptoms or biochemical indicators refers to 95%, 90%, 85%, 80%, 75%, 70%, 65%, 60%, 55%, 50%, 45%, 40%, 35%, 30%, 25%, 20%, 15%, 10%, or 5% of the peak level after giving the previous cycle treatment.

In some embodiments, after administration of the cell therapy product based on the previous cycle, when the serum level of a factor indicating cytokine-release syndrome (CRS) in a subject does not exceed 5, 6, 7, 8, 9, 10, 11, 12, 13, 14, 15, 16, 17, 18, 19, 20, 21, 22, 23, 24, 25, 26, 27, 28, 29, 30 times the serum level of the subject before given the previous cycle, treatment in the subsequent cycle will be given.

In some embodiments, the timing of administration in a subsequent cycle is chosen to avoid the possibility that a host immune response caused by administration in a previous cycle would induce reduced efficacy in a subsequent cycle. In some aspects, the subsequent cycle is administered previous to the emergence of a host immune response, e.g., fitness or specificity, e.g., a humoral or cell-mediated immune response, directed against the administered cells and/or the chimeric antigen receptors they express. In some embodiments, a subsequent period is administered before such a response becomes detectable, e.g., by one or more specialized detection methods. One or more subsequent cycles are typically administered when a host adaptive immune response against the cells has not been detected, has not yet been established and/or has not yet reached a certain level or degree or stage.

In some embodiments, after the previous cycle treatment, after the tumor burden has stabilized or decreased, before the adaptive host immune response (that is, the immune rejection of the body against CAR-T cells) occurs, one or more treatment in the subsequent cycle is administered. Under such conditions, immune surveillance, eradication, or prevention of proliferation or metastasis of residual tumor cells can be safely and effectively provided in the subsequent cycle. Thus, in some embodiments, the subsequent cycle is a disease consolidating dose.

In some embodiments, the subsequent cycle treatment is administered at least 28, 29, 30, 31, 32, 33, 34, 35 days after administering cell therapy product in the previous cycle.

The term "tumor burden" comprises tumor size or degree of differentiation, or type and stage of metastasis, and/or appearance and disappearance of common complications in middle-advanced cancer, such as malignant pleural and ascitic fluid, and/or appearance or changes in expression level of tumor markers, and/or likelihood or incidence of toxic outcomes in subjects, such as CRS, macrophage activation syndrome, tumor lysis syndrome, neurotoxicity and/or host immune response to cells administered and/or chimeric antigen receptors. In some embodiments, tumor size is measured by the built-in scales of PET (Positron Emission Tomography) and CT (X-ray Computed Tomography).

The tumor markers, also known as tumor lables, refer to a type of substance that is characterized by the presence in malignant tumor cells, abnormally produced by malignant tumor cells, or produced by the host's stimulus response to the tumor, and can reflect the occurrence and development of the tumor, and monitor the tumor's response to treatment. Tumor markers exist in the tissues, body fluids and excreta of tumor patients, and can be detected by immunological, biological and chemical methods, exemplary, including alpha-fetoprotein (AFP), CA125, CA15-3, squamous cellular carcinoma antigen (SCC), soluble fragment of cytokeratin 19 (CYFRA21-1), carcinoembryonic antigen (CEA), CA199, CA724, etc.

The uses or methods provided by the present application comprise administering at least one cycle of cell therapy products, such as those immune effector cells or any combination thereof expressing CAR, TCR, TFP, or TAC, to cancer patients who have failed with previous anti-PD-1 antibodies and/or anti-PD-L1 antibodies treatment.

"Tumor antigen" as used herein includes, but is not limited to, thyroid stimulating hormone receptor (TSHR); CD171; CS-1; C-type lectin-like molecule-1; ganglioside GD3; Tn antigen; CD19; CD20; CD 22; CD 30; CD 70; CD 123; CD 138; CD33; CD44; CD44v7/8; CD38; CD44v6; B7H3 (CD276), B7H6; KIT (CD117); interleukin-13 receptor subunit α (IL-13R α); interleukin 11 receptor alpha (IL-11Rα); prostate stem cell antigen (PSCA); prostate specific membrane antigen (PSMA); carcinoembryonic antigen (CEA); NY-ESO-1; HIV-1 Gag; MART-1; gp100; tyrosinase; mesothelin; EpCAM; protease serine 21 (PRSS21); vascular endothelial growth factor receptor; Lewis (Y) antigen; CD24; platelet-derived growth factor receptor beta (PDGFR-β); stage-specific embryonic antigen-4 (SSEA-4); cell surface-associated mucin 1 (MUC1), MUC6; epidermal growth factor receptor family and its mutants (EGFR, EGFR2, ERBB3, ERBB4, EGFRvIII); neural cell adhesion molecule (NCAM); carbonic anhydrase IX (CAIX); LMP2; ephrin type A receptor 2 (EphA2); fucosyl GM1; sialyl Lewis adhesion molecule (sLe); ganglioside GM3 (aNeu5Ac(2-3)bDGalp(1-4)bDGlcp(1-1)Cer; TGS5; high molecular weight melanoma-associated antigen (HMWMAA); ortho-acetyl GD2 ganglioside (OAcGD2); folate receptor; tumor vascular endothelial marker 1 (TEM1/CD248); tumor vascular endothelial marker 7-related (TEM7R); Claudin 6, Claudin18.2, Claudin18.1; ASGPR1; CDH16; 5T4; 8H9; αvβ6 integrin; B cell maturation antigen (BCMA ); CA9; kappa light chain; CSPG4; EGP2, EGP40; FAP; FAR; FBP; embryonic AchR; HLA-A1, HLA-A2; MAGEA1, MAGE3; KDR; MCSP; NKG2D ligand; PSC1; ROR1; Sp17; SURVIVIN; TAG72; TEM1; fibronectin; tenascin; carcinoembryonic variant of tumor necrosis zone; G protein-coupled receptor class C group 5-member D (GPRC5D); X chromosome open reading frame 61 (CXORF61); CD97; CD179a; anaplastic lymphoma kinase (ALK); polysialic acid; placenta-specific 1 (PLAC1); hexose moiety of globoh glycoceramide (GloboH); breast differentiation antigen (NY-BR-1); uroplakin 2 (UPK2); hepatitis A virus cellular receptor 1 (HAVCR1); adrenergic receptor beta 3 (ADRB3); pannexin 3 (PANX3); G protein-coupled receptor 20 (GPR20); Lymphocyte antigen 6 complex locus K9 (LY6K); olfactory receptor 51E2 (OR51E2); TCRy alternating reading frame protein (TARP); Wilms tumor protein (WT1); ETS translocation variant gene 6 (ETV6-AML); sperm protein 17 (SPA17); X antigen family member 1A (XAGE1); angiogenin-binding cell surface receptor 2 (Tie2); Melanoma cancer-testis antigen-1 (MAD-CT-1); Melanoma cancer-testis antigen-2 (MAD-CT-2); Fos-associated antigen 1; p53 mutant; human telomerase reverse transcriptase (hTERT); sarcoma translocation breakpoint; melanoma inhibitor of apoptosis (ML-IAP); ERG(transmembrane protease serine 2 (TMPRSS2) ETS fusion gene); N-acetylglucosaminyltransferase V (NA17); paired box protein Pax-3 (PAX3); androgen receptor; cyclin B1; V-myc avian myelocytomatosis viral oncogene neuroblastoma-derived homolog (MYCN); Ras homolog family member C (RhoC); cytochrome P450 1B1 (CYP1B1); CCCTC-binding factor (zinc finger protein)-like (BORIS); squamous cell carcinoma antigen 3 (SART3) recognized by T cells; paired box protein Pax-5 (PAX5); proacrosin binding protein sp32 (OYTES1); lymphocyte-specific protein tyrosine kinase (LCK); A kinase anchor protein 4 (AKAP-4); synovial sarcoma, breakpoint X 2 (SSX2); CD79a; CD79b; CD72; leukocyte-associated immunoglobulin-like receptor 1 (LAIR1); Fc fragment of IgA receptor (FCAR); leukocyte immunoglobulin-like receptor subfamily member 2 (LILRA2); CD300 molecule-like family member f (CD300LF); C-type lectin domain family 12 member A (CLEC12A); bone marrow stromal cell antigen 2 (BST2); EGF like module containing mucin like hormone receptor 2 (EMR2); lymphocyte antigen 75 (LY75); glypican-3 (GPC3); Fc receptor-like 5 (FCRL5); immunoglobulin λ-like Polypeptide 1 (IGLL1). In some embodiments, the tumor antigen is EGFR, EGFRvIII, glypican 3, claudin 18.2, or BCMA.

In some embodiments, the tumor antigen can be at least one of CD19, CD20, CD22, CD30, Mesothelin, BCMA, EGFR, EGFRvIII, PSMA, Mucl, claudin18.2, GPC3, IL13RA2.

In some embodiments, the tumor antigen can be at least one of CD19, BCMA, Claudin18.2, GPC3.

In some embodiments, the tumor or cancer may be colon cancer, gallbladder cancer, krukenberg tumor, rectal cancer, renal cell carcinoma, liver cancer, lung cancer, small cell lung cancer, small bowel cancer, esophageal cancer, melanoma, bone cancer, pancreatic cancer, skin cancer, head and neck cancer, skin or intraocular malignant melanoma, uterine cancer, ovarian cancer, rectal cancer, anal region cancer, gastric cancer, testicular cancer, uterine cancer, fallopian tube cancer, endometrial cancer, cervical cancer, vaginal cancer, Hodgkin's disease, non-Hodgkin's lymphoma, endocrine system cancer, thyroid cancer, parathyroid cancer, adrenal gland cancer, soft tissue sarcoma, urethral cancer, penile cancer, childhood solid tumors, bladder cancer, kidney or ureter cancer, renal pelvis carcinoma, central nervous system (CNS) tumor, primary CNS lymphoma, tumor angiogenesis, spinal tumor, brainstem glioma, pituitary adenoma, Kaposi's sarcoma, epidermoid carcinoma, squamous cell carcinoma, T-cell lymphomas, environmentally induced cancers, combinations of said cancers and metastatic lesions of said cancers. In some embodiments, gastric cancer comprises esophagogastric junction adenocarcinoma. In some embodiments, the liver cancer comprises hepatocellular carcinoma.

### CLD18 and CLD18-positive tumors

Claudin 18 (CLD18) molecular (Genbank Accesssion Number: splicing variant 1(Claudin 18.1, CLD18A1): NP_057453, NM016369, and splicing variant 2 (Claudin 18.2, CLD18A2): NM_001002026, NP_001002026) is an intrinsic transmembrane protein with a molecular weight of approximately 27,9/27,72 kD. Claudin is an intrinsic membrane protein located in the tight junctions of epithelium and endothelium. In a specific embodiment, CLD18A2 comprises the sequence shown in SEQ ID NO:56. CLD18A2 is strongly expressed in several cancer types, including gastric/esophagogastric junction adenocarcinoma, esophageal cancer, gallbladder cancer, pancreatic cancer, lung cancer, and Krukenberg tumors. Expression is predominantly in the adenocarcinoma subtype for these indications.

### GPC3 and GPC3-positive tumors

"GPC3" or "glypican 3" as used herein is a member of the Glypican family, which plays an important role in the regulation of cell growth and differentiation. In a specific embodiment, GPC3 comprises the sequence shown in SEQ ID NO:68. Abnormal expression of GPC3 is closely related to the occurrence and development of various tumors, such as abnormal expression in liver cancer, lung cancer, breast cancer, ovarian cancer, kidney cancer, thyroid cancer, gastric cancer, and colorectal cancer.

Methods of administering cell therapy products are known. In some embodiments, cell therapy products (e.g., CAR-T cells) can be administered to patients by autologous reinfusion. Thus, in some aspects, the cell therapy product is derived from a subject in need thereof and administered to the same subject following genetic modification in vitro. In some embodiments, cell therapy products (e.g., CAR-T cells) can be administered to patients by allogeneic reinfusion. Thus, in other aspects, the cell therapy product is derived from a healthy donor, which is different from the subject into which the cells are reinfused. In these aspects, the cell therapy product is administered to a subject with high genetically histocompatible, e.g., the donor and subject are genetically identical or similar. In some embodiments, the donor and subject have the same HLA class or supertype.

The cells may be administered by any suitable means, for example, by injection, for example, intravenous or subcutaneous injection, intraperitoneal injection, intraocular injection, fundus injection, subretinal injection, intravitreal injection, reverse interval injection, subscleral injection, intrachoroidal injection, anterior chamber injection, subconjectval injection, subconjunctival injection, episcleral injection, retrobulbar injection, periocular injection or peribulbar delivery. In some embodiments, they are administered parenterally, intrapulmonarily and intranasally, and if desired for local treatment, also intralesional. Extraperitoneal infusions comprise intramuscular, intravenous, intraarterial, intraperitoneal or subcutaneous administration.

For disease prevention or treatment, appropriate dosages may depend on the type of disease being treated, the chimeric antigen receptor or cell type, the severity and course of the disease, whether the cells are being administered for preventive or therapeutic purposes, previous treatment, the clinical history of the subject and their response to the cells, and the judgment of the attending physician. In some embodiments, the compositions and cells are suitable for administration to a subject at one point in time or over a series of treatments.

Pretreatment previous to administration of cell therapy products improves the efficacy of immune effector cell therapy. The day of the first infusion of immune effector cells (e.g., CAR-T cells) in each cycle is defined as day 0, and pretreatment is administered before the infusion of immune effector cells. In some embodiments, pretreatment is performed at least 2, 3, 4, 5, 6, 7, 8, 9, or 10 days before administration of adoptive cells or immune effector cells. In some embodiments, the pretreatment is implemented 2-8 days before administering the cell therapy product; preferably, the pretreatment is implemented 3-6 days before administering the cell therapy product. As described above, the day on which CAR-T cell therapy is administered to patients for each cycle is designated as day 0. In some embodiments, pretreatment can be administered at any time previous to administration of CAR-T cell therapy.

In some embodiments, the efficacy of the cell therapy product can be maximized by adjusting the timing or frequency of administration of the chemotherapeutic agent or treatment administered in the pretreatment.

In some embodiments, the cyclophosphamide is administered 2-3 times; or the fludarabine is administered 1-2 times; or the nab-paclitaxel is administered once.

In some embodiments, each chemotherapeutic agent is administered for no more than 4 consecutive days.

In some embodiments, the amount of fludarabine to be administered is about 20-60 mg/day, or about 30-60 mg/day, or about 30-55 mg/day, or about 30-50 mg/day.

In some embodiments, the amount of fludarabine to be administered is about 10-50 mg/m²/day, or about 15-40 mg/m²/day, or about 15-30 mg/m²/day, or about 20-30 mg/m²/day; or about 25mg/m²/day.

In some embodiments, the amount of cyclophosphamide to be administered is about 200-700 mg/day, or about 300-700 mg/day, or about 300-600 mg/day, or about 300-560 mg/day, or about 300-550 mg/day, or about 300-500mg/day.

In some embodiments, the amount of cyclophosphamide to be administered is about 200-400 mg/m²/day, or about 200-300 mg/m²/day, or about 250 mg/m²/day.

In some embodiments, the dosage of the taxane compound is not more than about 300 mg/day, or not more than about 200 mg/day; preferably, the dosage of the taxane compound is about 90-200 mg/day. More preferably, the dosage of the taxane compound is about 90-120 mg/day.

The day on which patients are administered CAR-T cell therapy in each round is designated as day 0. In some embodiments, the patient is administered fludarabine on Day -6 and Day -5. In some embodiments, the patient is administered fludarabine on Day -5 and Day -4. In some embodiments, the patient is administered fludarabine on Day -4 and Day -3. In some embodiments, the patient is administered fludarabine on Day -6, Day -5, Day -4, and Day -3. In some embodiments, the patient is administered fludarabine on Day -7, Day -6, Day -5, and Day -4. In some embodiments, the patient is administered cyclophosphamide on Day -6, Day -5, Day -4, and Day -3. In some embodiments, the patient is administered cyclophosphamide on Day -6, Day -5, and Day -4. In some embodiments, the patient is administered cyclophosphamide on Day -5, Day -4, and Day -2. In some embodiments, the patient is administered cyclophosphamide on Day -5, Day -4, and Day -3. In some embodiments, the patient is administered cyclophosphamide on Days -4, -3, and -2. In some embodiments, the patient is administered cyclophosphamide on Day -4 and Day -3. In some embodiments, the patient is administered cyclophosphamide on Day -6 and Day -5. In some embodiments, the patient is administered cyclophosphamide on Day -5 and Day -4. In some embodiments, the patient is administered a taxane compound (e.g., nab-paclitaxel) on Day -2, Day -3, Day -4, Day -5, or Day -6.

Fludarabine, cyclophosphamide, and nab-paclitaxel can be administered on the same day or on different days. If fludarabine, cyclophosphamide, and nab-paclitaxel are administered on the same day, cyclophosphamide and/or nab-paclitaxel can be administered before or after fludarabine; or fludarabine and/or nab-paclitaxel can be administered before or after cyclophosphamide; or cyclophosphamide and/or fludarabine can be administered before or after nab-paclitaxel.

In some embodiments, fludarabine, cyclophosphamide, nab-paclitaxel may be administered simultaneously or sequentially. In some embodiments, cyclophosphamide is administered to the patient previous to administration of fludarabine. In some embodiments, cyclophosphamide is administered to the patient after administration of fludarabine. In some embodiments, nab-paclitaxel is administered to the patient previous to administration of fludarabine. In some embodiments, nab-paclitaxel is administered to the patient after administration of fludarabine. In some embodiments, nab-paclitaxel is administered to the patient previous to administration of cyclophosphamide. In some embodiments, nab-paclitaxel is administered to the patient after administration of cyclophosphamide.

Fludarabine, cyclophosphamide, and nab-paclitaxel can be administered by any route, including intravenous injection(IV).

Various other interventions can be comprised in the methods described herein. For example, cyclophosphamide and fludarabine may cause adverse events in patients after administration. It is within the scope of the application to administer compositions to patients to reduce some of these adverse events. In some embodiments, the method comprises administering saline to the patient. Normal saline can be administered to the patient before or after administration of cyclophosphamide and/or fludarabine, or before and after administration of cyclophosphamide and/or fludarabine. In some embodiments, the patient is administered saline before and after cyclophosphamide and/or fludarabine is administered on each infusion day. In addition, adjuvants and vehicles can also be administered to the patient. For example, Mesna (sodium 2-mercaptoethanesulfonate). In addition, exogenous cytokines can also be administered to patients.

The biological activity of the population of engineered immune effector cells can be measured by any one of a variety of known methods after administration of the cell therapy product to the subject. Parameters used for assession comprise: specific binding of cells to antigen, either in vivo (e.g., by imaging) or ex vivo (e.g., by ELISA or flow cytometry). In certain embodiments, the ability of engineered immune effector cells to destroy target cells can be detected using any suitable method known in the art, such as the cytotoxicity assays described in, e.g., Kochenderfer et al., J. Immunotherapy, 32(7):689-702 (2009) and Herman et al. J. Immunological Methods, 285(1):25-40 (2004). In certain embodiments, the biological activity of immune effector cells can also be measured by measuring the expression and/or secretion of certain cytokines, such as CD107a, IFN₇, IL-2 and TNF. In some aspects, biological activity is assessed by assessing clinical outcome, such as reduction of tumor burden. In some aspects, by assessing reduction of tumor markers. In some aspects, by assessing cell toxic outcome, persistence and/or proliferation, and/or the presence or absence of a host immune response.

As used herein, "previous cycle" is relative to "subsequent cycle", the administration cycle before the administration of the subsequent cycle, such as the administration of the second cycle and the third cycle after completion of the first cycle of administration, the second cycle is relative to the first cycle, the first cycle is the previous cycle, the second cycle is the subsequent cycle, and the third cycle is relative to the second cycle, the second cycle is the previous cycle, the third cycle is the subsequent cycle.

In a specific embodiment, the dose of the cell therapy product refers to the number of cells contained in the cell therapy product.

In some embodiments, the amount or size in a subsequent cycle is sufficient to reduce tumor burden or an indicator thereof, and/or one or more symptoms of a disease or disorder. In some embodiments, the dose is of a size effective to improve the survival of the subject, e.g., improve the survival of the subject, e.g., induce survival, relapse-free survival, or event-free survival of the subject for at least 1 month or at least 1, 2 , 3, 4 or 5 years.

In some embodiments, tumor burden, including tumor size, tumor volume, and/or tumor mass, is reduced at least equal to or about 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90% or more after a subsequent cycle, compared to the moment of administration in a previous cycle or before a subsequent cycle.

In other embodiments, the number of immune effector cells administered in a subsequent cycle is lower than the number of immune effector cells administered in a previous cycle.

In some aspects, whether to administer a subsequent cycle of therapy is based on one or more criteria, such as the subject's response to a previous cycle of therapy, e.g., chemotherapy, the subject's tumor burden, e.g., tumor volume, size or degree, extent, or type of metastasis, stage and/or complication common to middle-advanced cancers, such as malignant pleural and ascitic fluid, and/or likelihood or incidence of toxic outcomes in subjects, such as CRS, macrophage activation syndrome, tumor lysis syndrome, neurotoxicity and/or host immune response against administered cells and/or chimeric antigen receptors.

Based on the teachings of the present application, those skilled in the art should understand that the dose specifically disclosed in the present application is a safe and effective dose obtained by the inventor through research, and those skilled in the art, such as clinicians, can determine the dosage in each cycle according to various actual conditions, such as the patient's tumor burden and the patient's physical condition; if further administration in the subsequent cycle is required, those skilled in the art can determine the dose in the subsequent cycle according to, for example, the change in tumor burden after administration of immune effector cells.

In some aspects, the dosage of the cell therapy product administered in a subsequent cycle is determined based on the tumor burden before administration of the previous cycle of therapy. In some embodiments, for example, wherein, the tumor burden reduces or decreases, or is already below a specific threshold quantity or level due to a previous cycle, e.g., an increase in the risk of toxicity results above which it indicates, the dose is increased in subsequent cycles.

In some cases, the dose of a cell therapy product in a subsequent cycle may be increased even if the subject's tumor burden has not decreased after receiving the previous cycle of therapy.

T cells (e.g., CAR-T cells) can be administered through any route, including intravenous (IV) injection. In some embodiments, the CAR-T cells are administered by IV within the following time periods: about 3 minutes, about 4 minutes, about 5 minutes, about 6 minutes, about 7 minutes, about 8 minutes, about 9 minutes, about 10 minutes, about 11 minutes, about 12 minutes, about 13 minutes, about 14 minutes, about 15 minutes, about 16 minutes, about 17 minutes, about 18 minutes, about 19 minutes, about 20 minutes, about 21 minutes, about 22 minutes, about 23 minutes, about 24 minutes, about 25 minutes, about 26 minutes, about 27 minutes, about 28 minutes, about 29 minutes, about 30 minutes, about 40 minutes, about 50 minutes, about 60 minutes.

In some embodiments, if the clinical risk indicated by biochemical indicators or indicators indicative of side effects (such as: cytokine release syndrome (CRS), macrophage activation syndrome, or tumor lysis syndrome, or neurotoxicity) does not exist or has passed, treatment in a subsequent cycle may be administered when desired.

In some embodiments, whether to administer the treatment in the subsequent cycle, when to administer the treatment in the subsequent cycle, and/or the dose of the cell therapy product administered in the subsequent cycle is determined based on the presence, absence, or degree of immune response or detectable immune response to cells in the previous cycle or the chimeric antigen receptors they expressed in the subject.

In some embodiments, the time of dosing in the subsequent cycle is calculated from completion of the cell therapy product in the previous cycle (the date of completion of the total dose infusion of the previous cycle is set as day 0).

In some embodiments, when the serum level of a factor indicating CRS in the subject is no more than about 10-fold, 25-fold, 50-fold, or 100-fold of the serum level of that indicator in a subject at the time before the administration of the previous cycle, treatment in a subsequent cycle is administered.

In some embodiments, a CRS-associated outcome (e.g., a serum factor associated with or indicative of CRS) or clinical signs or symptoms thereof such as fever, hypoxia, hypotension, or neurological disturbance has reached a peak level in the subject, and after the onset of decline, the subsequent cycle treatment is administered after the administration of the previous cycle.

In some embodiments, a subsequent cycle treatment is administered when a host adaptive immune response has not been detected, has not yet been established, or has not yet reached a certain level, extent or stage. In some aspects, the subsequent cycle treatment is administered before the subject develops an anamnestic immune response.

In some embodiments, tumor burden is monitored between previous and subsequent cycles.

A detectable immune response refers to an amount detectable by any one of a number of known methods for assessing specific immune responses to particular antigens and cells. For example, in some embodiments, specific types of immune responses are detected by performing ELISPOT, ELISA, or cell-based antibody detection methods (such as flow cytometry) on the serum of subjects to detect the presence of antibody that specifically binds and/or neutralizes those antigens present on cells (such as epitopes that bind chimeric antigen receptors, such as CAR). In some such assays, the isotype of the antibody detected is determined and can indicate the type of response and/or whether the response is a memory response.

In some aspects, for example, the amount and extent of the immune response is detected or measured following administration of a previous cycle treatment or a subsequent cycle treatment.

In some aspects, disease burden is measured or detected previous to administration of a previous cycle treatment, or between administration of a previous cycle treatment and administration of a subsequent cycle treatment, or after administration of a subsequent cycle treatment.

In some embodiments, the disease burden is reduced about 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, or 100% after administration of a previous cycle treatment. In some aspects, the disease burden is further reduced after administration of a subsequent cycle treatment, for example, compared to the disease burden before the subsequent cycle treatment, after the subsequent cycle treatment, the disease burden is reduced about 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90% or 100%.

In some embodiments, the progression-free rate or overall survival rate of the subject is improved by the method compared to other methods. For example, in some embodiments, one month after the previous cycle treatment, the subject has a progression-free survival rate of greater than about 10%, greater than about 20%, greater than about 30%, greater than about 40%, greater than about 50%, greater than about 60%, greater than about 70%, greater than about 80%, greater than about 90%, or greater than about 95%. In some aspects, the overall survival rate is greater than about 10%, greater than about 20%, greater than about 30%, greater than about 40%, greater than about 50%, greater than about 60%, greater than about 70%, greater than about 80%, greater than about 90% or greater than about 95%. In some embodiments, the subject shows at least about 1 month, or at least about 2, 3, 4, 5, 6, 7, 8, 9, 10, 11 months, or 1, 2, 3, 4, 5, 6, 7, 8, 9 or 10 years of progression-free survival, relapse-free survival, or overall survival. In some embodiments, the time of progression-free survival is, for example, greater than or about 1 month, or at least about 2, 3, 4, 5, 6, 7, 8, 9, 10, 11 months, or 1, 2, 3, 4, 5, 6, 7, 8, 9 or 10 years.

In some embodiments, treatment by this method reduces the probability of recurrence compared to other methods. For example, in some embodiments, the probability of recurrence or progression 1 month after the previous cycle is less than about 80%, less than about 70%, less than about 60%, less than about 50%, less than about 40%, less than about 30%, less than about 20%, less than about 10%, less than about 9%, less than about 8%, less than about 7%, less than about 6%.

### Exogenous receptors expressed by cells

Cells can be exogenously modified by known biological techniques to express exogenous receptors, such as exogenous expression of TCR receptors, chimeric antigen receptors (CAR) and T cell fusion protein (TFP) and T cell antigen coupler (TAC).

Exemplary exogenous receptors, such as sequences including CAR, may have amino acid sequences as shown in any one of SEQ ID NO: 11, 12, 13, 25, 26, 36, 38, or 52. Exemplarily, the CAR of the present application comprises combinations of sequence shown in SEQ ID NO: 58, 59, 60, 61, 62, 63, 64, 65, 66 or 67 and the sequence shown in SEQ ID NO: 80, 81 or 82 sequentially connected, respectively. Exemplarily, the sequence shown in SEQ ID NO: 58 is sequentially connected with the sequence shown in SEQ ID NO: 80, or the sequence shown in SEQ ID NO: 58 is sequentially connected with the sequence shown in SEQ ID NO: 81, and the sequence shown in SEQ ID NO: 58 is sequentially connected with the sequence shown in SEQ ID NO: 82; the sequence shown in SEQ ID NO: 59 is sequentially connected with the sequence shown in SEQ ID NO: 80, the sequence shown in SEQ ID NO: 59 is sequentially connected with the sequence shown in SEQ ID NO: 81, the sequence shown in SEQ ID NO: 59 is sequentially connected with the sequence shown in SEQ ID NO: 82; the sequence shown in SEQ ID NO: 60 is sequentially connected with the sequence shown in SEQ ID NO: 80, the sequence shown in SEQ ID NO: 60 is sequentially connected with the sequence shown in SEQ ID NO: 81, the sequence shown in SEQ ID NO: 60 is sequentially connected with the sequence shown in SEQ ID NO: 82; the sequence shown in SEQ ID NO: 61 is sequentially connected with the sequence shown in SEQ ID NO: 80, the sequence shown in SEQ ID NO: 61 is sequentially connected with the sequence shown in SEQ ID NO: 81, the sequence shown in SEQ ID NO: 61 is sequentially connected with the sequence shown in SEQ ID NO: 82; the sequence shown in SEQ ID NO: 62 is sequentially connected with the sequence shown in SEQ ID NO: 80, the sequence shown in SEQ ID NO: 62 is sequentially connected with the sequence shown in SEQ ID NO: 81, the sequence shown in SEQ ID NO: 62 is sequentially connected with the sequence shown in SEQ ID NO: 82; the sequence shown in SEQ ID NO: 63 is sequentially connected with the sequence shown in SEQ ID NO: 80, the sequence shown in SEQ ID NO: 63 is connected with the sequence shown in SEQ ID NO: 81; the sequence shown in SEQ ID NO: 63 is sequentially connected with the sequence shown in SEQ ID NO: 82; the sequence shown in SEQ ID NO: 64 is sequentially connected with the sequence shown in SEQ ID NO: 80, the sequence shown in SEQ ID NO: 64 is sequentially connected with the sequence shown in SEQ ID NO: 81, the sequence shown in SEQ ID NO: 64 is sequentially connected with the sequence shown in SEQ ID NO: 82; the sequence shown in SEQ ID NO: 65 is connected with the sequence shown in SEQ ID NO: 80, the sequence shown in SEQ ID NO: 65 is sequentially connected with the sequence shown in SEQ ID NO: 81, the sequence shown in SEQ ID NO: 65 is sequentially connected with the sequence shown in SEQ ID NO: 82; the sequence shown in SEQ ID NO: 66 is sequentially connected with the sequence shown in SEQ ID NO: 80, the sequence shown in SEQ ID NO: 66 is sequentially connected with the sequence shown in SEQ ID NO: 81, the sequence shown in SEQ ID NO: 66 is sequentially connected with the sequence shown in SEQ ID NO: 82; the sequence shown in SEQ ID NO: 67 is sequentially connected with the sequence shown in SEQ ID NO: 80, the sequence shown in SEQ ID NO: 67 is sequentially connected with the sequence shown in SEQ ID NO: 81, the sequence shown in SEQ ID NO: 67 is sequentially connected with the sequence shown in SEQ ID NO: 82.

Exemplarily, the CAR of the present application comprises sequencial combination of the sequence shown in SEQ ID NO: 70 and the sequence shown in SEQ ID NO: 80, or sequencial combination of the sequence shown in SEQ ID NO: 70 and the sequence shown in SEQ ID NO: 81, or sequencial combination of the sequence shown in SEQ ID NO: 70 and the sequence shown in SEQ ID NO: 82; sequencial combination of the sequence shown in SEQ ID NO: 71 and the sequence shown in SEQ ID NO: 80, or sequencial combination of the sequence shown in SEQ ID NO: 71 and the sequence shown in SEQ ID NO: 81, sequencial combination of the sequence shown in SEQ ID NO: 71 and the sequence shown in SEQ ID NO: 82; sequencial combination of the sequence shown in SEQ ID NO: 72 and the sequence shown in SEQ ID NO: 80; sequencial combination of the sequence shown in SEQ ID NO: 72 and the sequence shown in SEQ ID NO: 81, sequencial combination of the sequence shown in SEQ ID NO: 72 and the sequence shown in SEQ ID NO: 82; sequencial combination of the sequence shown in SEQ ID NO: 73 and the sequence shown in SEQ ID NO: 80, sequencial combination of the sequence shown in SEQ ID NO: 73 and the sequence shown in SEQ ID NO: 81, sequencial combination of the sequence shown in SEQ ID NO: 73 and the sequence shown in SEQ ID NO: 82; sequencial combination of the sequence shown in SEQ ID NO: 74 and the sequence shown in SEQ ID NO: 80, sequencial combination of the sequence shown in SEQ ID NO: 74 and the sequence shown in SEQ ID NO: 81, sequencial combination of the sequence shown in SEQ ID NO: 74 and the sequence shown in SEQ ID NO: 82; sequencial combination of the sequence shown in SEQ ID NO: 75 and the sequence shown in SEQ ID NO: 80, sequencial combination of the sequence shown in SEQ ID NO: 75 and the sequence shown in SEQ ID NO: 81, sequencial combination of the sequence shown in SEQ ID NO: 75 and the sequence shown in SEQ ID NO: 82; sequencial combination of the sequence shown in SEQ ID NO: 76 and the sequence shown in SEQ ID NO: 80, sequencial combination of the sequence shown in SEQ ID NO: 76 and the sequence shown in SEQ ID NO: 81, sequencial combination of the sequence shown in SEQ ID NO: 76 and the sequence shown in SEQ ID NO:82; sequencial combination of the sequence shown in SEQ ID NO: 77 and the sequence shown in SEQ ID NO:80, sequencial combination of the sequence shown in SEQ ID NO: 77 and the sequence shown in SEQ ID NO:81, sequencial combination of the sequence shown in SEQ ID NO:77 and the sequence shown in SEQ ID NO:82; sequencial combination of the sequence shown in SEQ ID NO: 78 and the sequence shown in SEQ ID NO: 80, sequencial combination of the sequence shown in SEQ ID NO: 78 and the sequence shown in SEQ ID NO:81, sequencial combination of the sequence shown in SEQ ID NO:78 and the sequence shown in SEQ ID NO: 82; sequencial combination of the sequence shown in SEQ ID NO: 79 and the sequence shown in SEQ ID NO: 80, sequencial combination of the sequence shown in SEQ ID NO: 79 and the sequence shown in SEQ ID NO:81, sequencial combination of the sequence shown in SEQ ID NO:79 and the sequence shown in SEQ ID NO:82. Exemplarily, the sequence shown as SEQ ID NO: 83, 84 or 85.

The terms "polypeptide", "peptide" and "protein" are used interchangeably to refer to a polymer of amino acid residues and are not limited to a minimum length. Polypeptides including receptors provided and other polypeptides (e.g., linkers or peptides) may comprise amino acid residues, including natural and/or unnatural amino acid residues. The term also comprises post-expression modifications of the polypeptide, such as glycosylation, sialylation, acetylation and phosphorylation. In some aspects, the polypeptides may contain modifications to the original or natural sequence so long as the protein retains the desired activity. These modifications may be deliberate, such as through site-directed mutagenesis, or may be accidental, such as through mutation of the host producing the protein or errors due to PCR amplification.

In the present application, cells expressing exogenous receptors are generally eukaryotic cells, such as mammalian cells, and usually human cells. In some embodiments, the cells are derived from blood, bone marrow, lymphoid or lymphoid organs and are cells of the immune system, such as cells of innate or adaptive immunity, e.g., myeloid or lymphoid cells, including lymphocytes, typically T cells, NKT cells and/or NK cells. Other exemplary cells comprise stem cells, such as multipotent and pluripotent stem cells, including induced pluripotent stem cells (iPSCs). Cells are typically primary cells, such as cells isolated directly from a subject and/or isolated from a subject and frozen. In some embodiments, the cells comprise one or more subpopulations of T cells or other cell types, such as the entire T cell population, CD4+ T cells, CD8+ T cells, and subpopulations thereof, such as T cells defined by function, activation state, maturation, differentiation potential, expansion, recycling, localization, and/or persistence capacity, antigen specificity, type of antigen receptor, presence in specific organs or compartments, secretion profiles of marker or cytokine, and/or degree of differentiation. The cells may be allogeneic and/or autologous with respect to the subject to be treated. Methods for preparing the cells expressing exogenous receptor comprise existing methods. In some embodiments, the method comprises isolating cells from a subject, preparing, treating, culturing and/or engineering them, and reintroducing them into the same subject either before or after cryopreservation.

Subtypes and subpopulations of T cells and/or CD4+ T cells and/or CD8+ T cells comprise: naive T (TN) cells, effector T cells (TEFF), memory T cells and their subtypes, such as stem cell memory T (TSCM), central memory T (TCM), effector memory T (TEM), or terminally differentiated effector memory T cells, tumor-infiltrating lymphocytes (TIL), immature T cells, mature T cells, helper T cells, cytotoxic T cells, mucosa-associated non-variant T (MAIT) cells, naturally occurring and adoptive regulatory T (Treg) cells, helper T cells such as TH1 cells, TH2 cells, TH3 cells, TH17 cells, TH9 cells, TH22 cells, follicular helper T cells, α/β T cells, and δ/γ T cells or combinations thereof.

In some embodiments, the cells are natural killer (NK) cells. In some embodiments, the cells are monocytes or granulocytes, e.g., myeloid cells, macrophages, neutrophils, dendritic cells, mast cells, eosinophils, basophils, or combinations thereof.

In some embodiments, the cell comprises one or more nucleic acids introduced by genetic engineering and thereby expresses a recombinant or genetically engineered product of the nucleic acids. In some embodiments, the nucleic acid is heterologous, not normally present in the cell or a sample obtained from the cell, such as a sample obtained from another organism or cell, or not usually found in cells in process of being engineered and/or organisms of such cell originates. In some embodiments, the nucleic acid is not naturally occurring, such as the nucleic acid is not found in nature, including chimeric combinations comprising nucleic acids encoding various domains from multiple different cell types, such as CAR, chimeric cytokine receptor.

### Methods and vectors for genetic engineering

The application also provides methods, compositions and kits for producing genetically engineered cells expressing chimeric antigen receptors. Genetic engineering generally involves introducing nucleic acid encoding the recombinant or engineered portion into a cell, for example, by viral (e.g. lentivirus, retrovirus, AAV) transduction, transfection or transformation.

The term "engineered" refers to a comprehensive science and technology that applies the principles and methods of cell biology and molecular biology to change the genetic material within cells or obtain cell products at the cellular or organelle level according to people's wishes through certain engineering methods. In one embodiment, the "engineered" refers to one or more alterations of a nucleic acid, such as a nucleic acid within the genome of an organism. In one embodiment, "engineered" refers to changes, additions and/or deletions of genes. In one embodiment, the engineered cells may also refer to cells with added, deleted and/or altered genes.

In some embodiments, gene transfer is performed by first stimulating the cell (e.g., by combining it with a stimulus that induces a response, e.g., proliferation, survival and/or activation, e.g., is detected by expression of cytokines or activation markers), then transducing the activated cells and expanding in culture to quantities sufficient for clinical use.

In some aspects, cells are also engineered to promote expression of cytokines or other factors.

In some embodiments, recombinant nucleic acid is transferred into cells using recombinant infectious viral particles, e.g., vectors derived from simian virus 40 (SV40), adenovirus, adeno-associated virus (AAV). In some embodiments, recombinant nucleic acids are transferred into T cells using recombinant lentiviral vectors or retroviral vectors, such as γ-retroviral vectors.

In some embodiments, the recombinant nucleic acid is transferred into T cells by electroporation. In some embodiments, the recombinant nucleic acid is transferred into T cells by translocation (see, e.g., Manuri et al., (2010) Hum Gene Ther 21(4):427-437; Sharma et al., (2013) Molec Ther Nucl Acids 2, e74; and Huang et al., (2009) Methods Mol Biol 506:115-126). Other methods of introducing and expressing genetic material into immune cells comprise calcium phosphate transfection (e.g., as described in Current Protocols in Molecular Biology, John Wiley & Sons, New York, NY), protoplast fusion, cationic liposome-mediated transfection; microparticle bombardment facilitated by tungsten particles (Johnston, Nature, 346:776-777 (1990)); and strontium phosphate DNA co-precipitation (Brash et al., Mol. Cell Biol., 7:2031-2034 (1987)).

### Preparation of immune effector cells

In some embodiments, preparation of engineered cells comprises one or more culturing and/or one or more preparation steps. Cells for introducing a nucleic acid encoding a transgenic receptor (e.g., CAR) can be isolated from a sample (e.g., a biological sample, e.g., sample obtained or derived from a subject). In some embodiments, the subject from which the cells are isolated is a subject suffering from a certain disease or condition or in need of cell therapy or to be treated with cell therapy. In some embodiments, the subject is a human in need of a specific therapeutic intervention, for example, a human in need of adoptive cell therapy or immune effector cell therapy, the cells used for this therapy are isolated, processed and/or engineered. In some embodiments, the cells are primary cells, e.g., primary human cells. Such samples comprise samples obtained directly from tissues, body fluids, and other of a subject, as well as samples obtained from one or more processing steps, such as separation, centrifugation, genetic engineering (e.g., transduction with a viral vector), washing, and/or incubation. The biological sample may be a sample obtained directly from a biological source or a processed sample. Biological samples comprise, but are not limited to, body fluids such as blood, plasma, serum, cerebrospinal fluid, synovial fluid, urine, and sweat, tissue and organ samples, including processed samples derived therefrom.

In some aspects, the sample from which the cells are derived or isolated is blood or a blood-derived sample or, is or derived from an apheresis or leukapheresis product. Exemplary samples comprise whole blood, PBMC, white blood cells, bone marrow, thymus, tissue biopsy, tumor, leukemia, lymphoma, lymph node, gut-associated lymphoid tissue, mucosa-associated lymphoid tissue, spleen, other lymphoid tissue, liver, lung, stomach, intestine, colon, kidney, pancreas, breast, bone, prostate, cervix, testis, ovary, tonsil, or other organ, and/or cells derived therefrom. In the case of cell therapy (e.g., adoptive cell therapy or immune effector cell therapy), samples comprise samples from autologous and allogeneic sources.

In some embodiments, the cells are derived from a cell line, e.g., a T cell line. In some embodiments, the cells are obtained from a xenogeneic source, e.g., from a mouse, rat, non-human primate, or pig.

In some embodiments, isolation of cells comprises one or more preparative and/or non-affinity-based cell isolation steps. In some embodiments, cells are washed, centrifuged, and/or incubated in the presence of one or more substances, e.g., to remove unwanted components, enrich the desired components, lyse or remove cells sensitive to specific substances. In some embodiments, cells are separated based on one or more properties, such as density, adhesion properties, size, sensitivity and/or resistance to particular components. In some embodiments, cells from circulating blood of a subject are obtained, e.g., by apheresis or leukapheresis. In some aspects, the sample comprises lymphocytes, including T cells, monocytes, granulocytes, B cells, other nucleated blood leukocytes, red blood cells, and/or platelets, and in some aspects comprises cells that are different from red blood cells and platelets.

In some embodiments, the method comprises a density-based cell separation method, e.g., peripheral blood mononuclear cells (PBMCs) can be prepared by lysis of red blood cells or non lysis of red blood cells and gradient centrifugation of peripheral blood through a Percoll or Ficoll or by apheresis or leukapheresis of the sample.

In some embodiments, the isolating method comprises separating different cell types based on the expression or presence of one or more specific molecules (e.g., surface markers, e.g., surface proteins, intracellular markers, or nucleic acids) in the cells. In some embodiments, any known method for isolation based on such markers can be used. In some embodiments, the separation is an affinity-based or immunoaffinity-based separation. For example, in some aspects, the isolation comprises isolating cells and cell populations based on the expression or expression level of one or more markers (typically cell surface markers) of the cells, e.g. incubating with an antibody or binding partner that specifically bind to such markers, then usually followed by a washing step and separation of cells that have bound to the antibody or binding partner from those that have not yet bound to the antibody or binding partner.

There is no need for the isolation to result in 100% enrichment or removal of a particular cell population or cells expressing a particular marker.

For example, in some aspects, specific subpopulations of T cells, such as cells positive for one or more surface markers or cells expressing high levels of one or more surface markers, e.g., CD3+, CD28+, CD62L+, CCR7+ , CD27+, CD127+, CD4+, CD8+, CD45RA+ and/or CD45RO+ T cells, isolated by positive or negative selection techniques.

For example, CD3+, CD28+ T cells can be positively selected using CD3/CD28 linked magnetic beads (e.g., DYNA Beads M-450 CD3/CD28 T Cell Expander).

In some embodiments, T cells are isolated from a PBMC sample by negative selection for a marker (e.g., CD14) expressed on non-T cells (e.g., B cells, monocytes, or other blood leukocytes). In some aspects, a CD4+ or CD8+ selection step is used to isolate helper CD4+ and CD8+ cytotoxic T cells. Such CD4+ and CD8+ populations can be further sorted into subpopulations by positive or negative selection for markers expressed on or to a relatively high degree on one or more subpopulations of naive, memory and/or effector T cells.

In some embodiments, CD8+ cells are further enriched or depleted against naive, central memory, effector memory, and/or central memory stem cells, e.g., by positive or negative selection based on surface antigens associated with the respective subpopulations. In some embodiments, enrichment for central memory T (TCM) cells is performed to increase efficacy, e.g., to improve long-term survival, expansion, and/or engraftment following administration, and in some aspects, it is particularly robust in such subpopulations.

In some embodiments, the preparation method comprises a freezing step, e.g., freezing the cells before or after isolation, incubation and/or engineering. In some embodiments, the freezing and subsequent thawing steps remove granulocytes and, to some extent, remove monocytes from the cell population. In some embodiments, for example, after a washing step to remove plasma and platelets, the cells are suspended in a freezing solution. In some aspects, any of a variety of known freezing solutions and parameters can be used. One example involves the use of PBS containing 20% DMSO and 8% human serum albumin (HSA) or other suitable cell freezing medium. Then, it is diluted 1:1 with medium so that the final concentrations of DMSO and HSA are 10% and 4%, respectively. Then, the cells are generally frozen to -80°C or -90°C with a programmed cooling device according to a predetermined program or principle such as a rate of 1°/min, and stored in the vapor phase of a liquid nitrogen storage tank.

In some embodiments, cells are incubated and/or cultured previous to or simultaneously with genetic engineering. Incubating steps can comprise culturing, cultivating, stimulating, activating, and/or proliferating. In some embodiments, the cells or compositions are incubated in presence of stimulatory conditions or stimulatory agents. Such conditions comprise those designed to induce proliferation, multiplication, activation, and/or survival of cells in a population to mimic antigen contact, and/or to initiate cells for genetic engineering modification, such as those used to introduce recombinant antigen receptors.

In some embodiments, stimulating conditions or agents comprise one or more substances, e.g., ligands, which are capable of activating the intracellular signaling domain of a TCR complex. In some aspects, the substance turns on or initiates a TCR/CD3 intracellular signaling cascade in the T cell. Such substances may comprise antibodies, such as those specific for TCR components and/or co-stimulatory receptors, e.g., anti-CD3, anti-CD28, e.g., bound to a solid support, e.g., beads, and/or a or one or more cytokines. Optionally, the expansion method may further comprise the step of adding anti-CD3 and/or anti-CD28 antibodies to the culture medium (e.g., at a concentration of at least about 0.5 ng/ml). In some embodiments, the stimulating agent comprises IL-2 and/or IL-15 and/or IL-7 and/or IL-21, e.g., IL-2 at a concentration of at least about 10 units/mL.

In a certain embodiment, antigen-specific T cells, such as antigen-specific CD4+ and/or CD8+ T cells, are obtained by stimulating natural or antigen-specific T lymphocytes with an antigen. For example, antigen-specific T cell lines or clones can be produced against a cytomegalovirus antigen by isolating T cells from an infected subject and stimulating the cells in vitro with the same antigen.

### Treating cancer patients with autologous T cells expressing CAR

Methods of the present application can be summarized as:
T cells are prepared from peripheral blood mononuclear cells (PBMCs) of human subjects with cancer using a "demononuclear cell isolation technique", and cultured and transduced using a viral vector encoding chimeric antigen receptor (CAR), the chimeric antigen receptor (CAR) specifically binds an antigen expressed by a tumor cell in the subject, the antigen is a tumor-associated antigen or a tumor-specific antigen. Cells are cryopreserved in the infusion medium in individual flexible freezing bags. Each contains a single unit dose of cells, which is about 1 × 10⁶ cells to 5 × 10⁷ cells. The cells infused per subject in a previous cycle should be no more than about 1 × 10¹² cells, preferably no more than about 1 × 10¹¹ cells, more preferably no more than about 1 × 10¹⁰ cells or about 5×10⁹ cells or about 2×10⁹ cells. The cells are maintained at a temperature of about below -130°C or about below -175°C before infusion.

Before initiating cell therapy, blood is obtained from the subject, and the levels of one or more serum factors indicating cytokine release syndrome (CRS) in the serum is evaluated through ELISA and/or MSD and/or CBA, for example, tumor necrosis factor alpha (TNFα), interferon gamma (IFNγ), IL-10, or IL-6. Tumor burden can optionally be assessed by measuring the size or mass of the solid tumor, e.g., by PET or CT scan, before initiation of treatment.

Resuscitation is performed by warming to approximately 38°C, and subjects are administered cells from previous cycles by multiple infusions. Each infusion is continuously administered intravenously (IV) over a period of about 3-30 minutes.

Following administration of the previous cycle, subjects undergo a physical examination and are monitored for any toxicity or symptoms of toxic consequences, such as fever, hypotension, hypoxia, neurological disturbances, or raised serum levels of inflammatory cytokines or C-reactive protein (CRP). Optionally, blood is obtained from the patient in one or more instances following administration of the previous cycle and assessed for levels of serum factors indicative of CRS by methods of ELISA and/or MSD and/or CBA. The levels of serum factors are compared to the levels of serum factors obtained just before administration of the previous cycle. If necessary, anti-IL6 or other CRS therapy is administered to reduce the symptoms of CRS.

The presence or absence of an anti-CAR immune response in the subject is optionally detected (e.g., by qPCR, ELISA, ELISPOT, cell-based antibody assays and/or mixed lymphocyte reactions) after administration of the previous cycle, e.g., 1, 2, 3 and/or 4 weeks after initiation of dosing.

The percent reduction in tumor burden achieved by the previous cycle can optionally be measured one or more times after administration of the previous cycle in patients with solid tumors by scanning (e.g., PET and CT scans), and/or by quantification tumor-positive cells in the blood or tumor site.

Subjects are monitored periodically beginning from the first dose and continuing for up to several years. During follow-up, measure tumor burden, and/or detect CAR-expressing cells by flow cytometry and quantitative polymerase chain reaction (qPCR) to measure in vivo proliferation and persistence of administered cells, and/or assess the development of anti-CAR immune responses.

Cells infused in the previous cycle for each subject shall not exceed about 1 × 10¹² cells, preferably no more than about 1 × 10¹¹ cells, preferably no more than about 1 × 10¹⁰ cells, or no more than about 5×10⁹ cells, or no more than 2×10⁹ cells. Cells are kept at a temperature below -175°C before infusion. During infusion, the temperature is raised to about 38°C for resuscitation.

Dosing in subsequent cycles is patient-specific and based on tumor burden, presence of anti-CAR immune response, and level of CRS-related outcomes. The dose administered in the subsequent cycle is no more than about 1×10¹² cells, preferably no more than about 1×10¹¹ cells, more preferably no more than about 1×10¹⁰ cells, more preferably no more than about 5 × 10⁹ cells or no more than about 2 × 10⁹ cells.

Descriptive statistical methods (e.g., case numbers, mean, median, standard deviation [SD], minimum and maximum) are used for analysis. Categorical variables are analyzed using frequency tables (frequency and percentage). All adverse events (AEs) will be classified according to the codes of the latest version of MedDRA, the ICH International Dictionary of Medical Terms, and graded according to the commonly used standard terms for adverse events (CTCAE v5.0 version), and analyzed by frequency distribution, charts or other descriptive indicators, the case numbers and percentage of subjects with treatment-emergent adverse events (TEAEs) will be calculated by system organ classification, preferred term, and group. The copy numbers of CAR-T cells containing different detection time points will be generated. 95% confidence intervals are calculated using the Clopper-Pearson method for ORR and DCR.

### Example

The present application will be further illustrated in conjunction with the following specific Examples. It should be understood that these examples are only used to illustrate the present application and are not intended to limit the scope of the present application. The experimental methods that do not indicate specific condition in the following Examples, usually follow conventional conditions such as the conditions described in J. Sambrook et al., eds., A Laboratory Guide to Molecular Cloning, Third Edition, Science Press, 2002, or as recommended by the manufacturer.

### Materials and Methods:

Exemplary antigen receptors of the present application, include CAR, and methods for engineering and introducing receptors into cells, refered to, for example, the full text disclosed in Chinese Patent Application Publication NOs. CN107058354A, CN107460201A, CN105194661A, CN105315375A, CN105713881A, CN106146666A, CN106519037A, CN106554414A, CN105331585A, CN106397593A, CN106467573A, CN108884459A, CN108610420A, CN108341872A, CN108456250A, CN109796532A, CN108866003A, CN108853144A, CN109385403A, CN109385400A, CN109468279A, CN109503715A, CN109880803A, CN110055275 A, CN110123837A, CN 110438082 A, CN 110468105 A, International Patent Application Publication NOs. WO2018006882A1, WO2015172339A8, WO2016086813A1, WO2017032293A1, WO2017020812A1, WO2017080377A1, WO2018108106A1, WO2018/133877 A1, WO2018/149358 A1, WO 2018/219299 A1, WO2019/096261A1, WO2018/210279 A1, WO2019/024933 A1, WO2020118634A1, WO2019052562A1, WO2019047932A1, WO2019109980A1, WO2019/141270 A1, WO2019/149279 A1, WO2019/170147A1, WO 2019/210863 A1, WO2019/219029A1, WO2020020210A1, WO2020057666A1, WO2020057641A1, WO 2020/083406A1, WO2020114518A1, WO2020143631A1, WO2020156554A1, WO2021/027785 A1, WO2021/052496 A1, WO2021/057906 A1, WO2021/136550 A1, WO2021148019A1, WO2021232864A1, WO2016036973A1, WO2016049459A1.

Exemplarily, in the following Examples of the present application, the scFv of the chimeric antigen receptor can recognize the tumor antigen claudin18.2 and has the sequence shown in SEQ ID NO: 14; the chimeric antigen receptor has the sequence shown in SEQ ID NO: 11, the CDR regions thereof have HCDR1 shown in SEQ ID NO: 1, HCDR2 shown in SEQ ID NO: 2, HCDR3 shown in SEQ ID NO: 3, LCDR1 shown in SEQ ID NO: 4, LCDR2 shown in SEQ ID NO: 5, and LCDR3 shown in SEQ ID NO:6.

The construction method of CAR-T cells: first the CAR gene was constructed, which comprises successively from the 5' end to the 3' end: CD8α signal peptide (nucleotide sequence shown in SEQ ID NO: 47), scFv (nucleotide sequence shown in SEQ ID NO: 15), CD8 hinge region (nucleotide sequence shown in SEQ ID NO: 48), CD28 transmembrane region (nucleotide sequence shown in SEQ ID NO: 49) and CD28 intracellular signaling domain (nucleotide sequence shown in SEQ ID NO:50) and intracellular signaling domain of CD3ζ (nucleotide sequence shown in SEQ ID NO:51). Then the CAR gene was cloned into the plasmid PRRLSIN-cPPT.EF-1α to obtain the target plasmid containing the CAR gene. The constructed target plasmid and packaging plasmid were co-transfected into 293T cells to prepare a lentiviral vector, and the lentiviral vector was transduced into T cells obtained from the patient's peripheral blood to obtain CAR-T cells.

The positive intensity of antigen (such as claudin18.2) was defined by the staining intensity of immunohistochemistry, which was staining of part or all of the cell membrane or cytoplasm, and the staining intensity was divided into: 0 (negative, no staining), + (weak positive staining), ++ (positive staining,) and +++ (strong positive staining). The percentage of stained tumor cells refered to the percentage of tumor cells with each staining intensity to all tumor cells.

### Example 1: Therapy of CAR-T cells

CAR-T cell therapy was performed on 12 CLDN18.2-positive gastric cancer patients who had failed PD-1 and/or PD-L1 antibody therapy. The expression of CLDN18.2 was detected on tumor tissue sections from previous operations or biopsies, all of which were CLDN18.2 expression positive (≥+, 10%), all subjects had distant metastases, the situation of the 12 patients is shown in Table 1, among which, the PD-1 and/or PD-L1 antibody used by the patient before was: Camrelizumab, Pembrolizumab, Sintilimab, Toripalimab, and some monoclonal antibodies in clinical trials.

Before administration of CAR-T cells, the patients received the apheresis technique of "removal of mononuclear cells" and underwent pretreatment.

Through apheresis, PBMCs were obtained from the subjects, and CAR-T cells were obtained by transduction and amplification with a viral vector encoding anti-CLDN18A2 CAR. The obtained CAR-T cells were stored frozen in a freezing liquid using a freezing bag.

Before administering the CAR-T cells, patients were administered chemotherapy drugs or radiotherapy for lymphocyte depletion.

Before administering CAR-T cell therapy, tumor burden could optionally be assessed by, for example, PET or CT scan measuring the size or character of solid tumors, and tumor burden could also be assessed by detecting tumor markers and/or observing the occurrence and severity of tumor complications.

When the subject needs at least one cycle of CAR-T cell therapy, the administration of CAR-T cells in the subsequent cycle shall be administered 4 weeks after completion of the previous cycle of CAR-T administration. The administration of CAR T cells in each cycle can be administered once or divided into two or more intravenous (IV) infusions. The infusion of the cell products administered each time was completed in about 3-30 minutes, preferably in 5-25 minutes.

After each cycle of CAR-T cell administration, the subject would undergo a physical examination and be monitored for any symptoms of toxicity or toxic consequences, such as fever, hypotension, hypoxia, neurological disturbances, or elevated serum levels of inflammatory cytokines or C reactions protein (CRP). The examination may be to obtain blood from the patient and evaluate the level of cytokines indicative of CRS by ELISA, and/or MSD, and/or CBA methods. If necessary, anti-IL6 therapy, or other CRS treatments was given to reduce the symptoms of CRS.

After the administration of CAR-T cells in each cycle, for example, 1, 2, 3, and/or 4 weeks after the start of administration, the number of CAR-T cells in the subject can be evaluated by qPCR, ELISA, ELISPOT, antibody assay, etc.

Reduction in tumor burden after each cycle of treatment can be obtained by scans (e.g., PET and CT scans), and/or by quantification of cells positive for antigens (e.g., claudin18.2) in the blood or at the tumor site.

The combination of fludarabine, cyclophosphamide, and nab-paclitaxel was used as pretreatment for CAR-T cell therapy, and was administered to subjects 2-10 days before CAR-T cell infusion. All 12 patients received pretreatment with fludarabine, cyclophosphamide and nab-paclitaxel. Among them, fludarabine was administered twice through intravenous infusion of 30-50mg/day of fludarabine on the 6th and 5th day (D-6, -5), or the 5th and 4th day (D-5, -4), or the 4the, 3th day (D-4, -3) before CAR-T cell infusion, of which 7 cases were at 30-40mg/day, 5 cases were at 41-50mg/day, the average daily dose was 39.8( ±4.08) mg, the average daily dose according to body surface area was 25.0 (±0.30) mg/m². Cyclophosphamide was administered 2 or 3 times, and when cyclophosphamide was administered twice, it was administered respectively on the 6th, 5th day (D-6, -5), or the 5th, 4th day (D-5, -4), or the 4^{th}, 3^{rd} day (D-4, -3) before CAR-T cell administration; when cyclophosphamide was administered three times, it was administered respectively on the 6th, 5th, 4th day (D-6, -5, -4), or the 5th, 4th, 3rd day (D-5, -4, -3), or the 4th, 3rd, 2nd day (D-4, -3, -2) before CAR-T cell administration; the dose of cyclophosphamide administered was 300-600mg/day, wherein, the amount of cyclophosphamide to be administered to the patient was 300-400mg/day or 401-500mg/day, and the average daily dose was 395.8 (±47.81) mg, according to the body surface area was 249.6 (±16.38) mg/m². Nab-paclitaxel was administered once 3-5 days before the administration of CAR-T cells, such as nab-paclitaxel was administered 90-200 mg/day on D-4. The average daily dose of nab-paclitaxel was 102.2 (±8.60) mg.

Among the 12 patients, 6 patients received two cycles of cell infusion, and the median interval time between the end of administration of CAR-T cells in the first cycle and the start of administration of CAR-T cells in the second cycle was 64 days.

**Table 1 Conditions of 12 patients**

| No. | patient | Weight (kg) | disease | CLDN 18.2 Strength | Expression of PD-L1 | Total dose given to CAR T | Treatm ent cycle |
|---|---|---|---|---|---|---|---|
| 1 | 1# subject | 65 | gastric cancer | + + + | positive | 5.0×10⁸ | 2 |
| 2 | 2# subject | 41 | gastric cancer | + + + | positive | 2.5×10⁸ | 1 |
| 3 | 3# subject | 66 | gastric cancer | + + + | positive | 2.5×10⁸ | 2 |
| 4 | 4# subject | 52 | gastric cancer | + + + | positive | 2.5×10⁸ | 2 |
| 5 | 5# subject | 59 | gastric cancer | + + | positive | 3.75×10⁸ | 1 |
| 6 | 6# subject | 40 | gastric cancer | + + + | positive | 3.75×10⁸ | 1 |
| 7 | 7# subject | 48 | gastric cancer | +++ | unknow | 2.5×10⁸ | 2 |
| 8 | 8# subject | 65 | gastric cancer | + + | negative | 2.5×10⁸ | 1 |
| 9 | 9# subject | 77 | gastric cancer | + + + | negative | 2.5×10⁸ | 2 |
| 10 | 10# subject | 55 | gastric cancer | + + + | unknow | 3.75×10⁸ | 1 |
| 11 | 11# subject | 37 | gastric cancer | + + + | negative | 2.5×10⁸ | 2 |
| 12 | 12# subject | 60 | gastric cancer | + + + | negative | 2.5×10⁸ | 1 |

### Example 2: Evaluation of Therapy

For the treatment evaluation of the 12 subjects involved in Example 1, Kaplan Meier method was used for statistical analysis of PFS, DDC, DOR and OS, and descriptive analysis for TTR.

Evaluate the tumor burden before and after treatment by detecting target lesions and non-target lesions with imaging, and determine the number and size of target lesions. The efficacy evaluation criteria refers to the efficacy evaluation criteria for solid tumors, version 1.1.

Following administration of CAR-T cells, subjects were evaluated and monitored for neurotoxicity (neurological complications including symptoms of confusion, aphasia, seizures, convulsions, lethargy, and/or altered mental status), graded according to severity (using a scale of levels 1-5, e.g., Guide Cavaletti and Paola Marmiroli Nature Reviews Neurology 6, 657-666 (December 2010), wherein level 3 (severe symptoms), level 4 (life-threatening symptoms) or level 5 (death) were considered to be serious neurotoxicity.

### (1) Identify and monitor cytokine release syndrome (CRS)

Level 1 (mild) - not life-threatening: only systemic therapy such as antipyretics and antiemetics was needed (e.g., fever, nausea, fatigue, headache, myalgia, malaise).

Level 2 (moderate) - require and respond to moderate intervention: oxygen requirement <40%, or hypotension with corresponding body fluids or low dose single vasopressor, or Level 2 organ toxicity (by CTCAE v5.0).

Level 3 (serious) - require and respond to aggressive intervention: oxygen requirement ≥40%, or hypotension requiring high dose single vasopressors (e.g., norepinephrine ≥20 µg/kg/min, dopamine ≥10 µg/kg/min, phenylephrine ≥200 µg/kg/min, or epinephrine ≥10 µg/kg/min), or hypotension requiring multiple vasopressors (e.g., antidiuretics + one of the above agents, or combination of vasopressor drugs equal to ≥20 µg/kg/min norepinephrine), or Level 3 organ toxicity or Level 4 transaminase inflammation (by CTCAE v5.0).

Level 4 (life-threatening) - requiring ventilator support, or Level 4 organ toxicity (excluding transaminase inflammation).

Level 5 (fatal) - death.

### (2) Exemplary grading criteria for neurotoxicity

Level 1 (asymptomatic or mild) - mild or asymptomatic.

Level 2 (moderate) - with symptoms that limit daily active activities (ADL) such as cooking, grocery shopping or clothing shopping, using the phone, managing money.

Level 3 (severe) - presence of symptoms of limiting self-management ADL, such as bathing, dressing or undressing, eating, using the toilet, taking medications.

Level 4 (life-threatening) - life-threatening symptoms requiring urgent intervention;

Level 5 (fatal) - death.

### (3) Effectiveness of treatment

After the first cycle of CAR-T cell therapy, the safety and efficacy of CAR-T therapy were evaluated, and the results are shown in Table 2. As shown in Table 2, regardless of the disease burden before treatment, no severe CRS or severe neurotoxicity occurred in the first cycle of treatment, thus the first cycle of treatment was safe. In addition, it was also observed that after the first cycle of treatment, 5 patients were in stable disease (SD), 5 patients achieved PR, and 2 patients had progressive disease (PD), indicating that CAR-T cells could still produce significant therapeutic effects on patients who have used PD-1 or PD-L1 antibodies, ORR reached 41.6% (5/12), and disease control rate reached 83.3% (10/12).

**Table 2 Safety and efficacy of CAR-T therapy**

| No. | patient | Disease burden before administration | First cycle | | | | |
|---|---|---|---|---|---|---|---|
| | | | Drug administration regimen | Severe CRS | Severe neurotoxicity | Response | Disease burden after the first cycle of treatment |
| 1 | 1# subject | The total diameter of the target lesion was 29mm, with 2 non target lesions | 5.0×10⁸ ( Two consecutive days of administration, 2.5×10⁸ per day ) | none | none | SD | The total diameter of the target lesion was 30mm, with 2 non target lesions |
| 2 | 2# subject | The total diameter of the target lesion was 128mm, with 4 non target lesions | 2.5×10⁸ | none | none | PD | The total diameter of the target lesion was 138mm, with 4 non target lesions |
| 3 | 3# subject | The total diameter of the target lesion was 15mm, with 3 non target lesions | 2.5×10⁸ | none | none | PR | The total diameter of the target lesion was 8mm, with 3 non target lesions |
| 4 | 4# subject | The total diameter of the target lesion was 41mm, with 3 non target lesions | 2.5×10⁸ | none | none | SD | The total diameter of the target lesion was 38mm, with 3 non target lesions |
| 5 | 5# subject | The total diameter of the target lesion was 90mm, with 3 non target lesions | 3.75×10⁸ | none | none | PD | The total diameter of the target lesion was 104mm, with 3 non target lesions |
| 6 | 6# subject | The total diameter of the target lesion was 32mm, with 6 non target lesions | 3.75×10⁸ | none | none | PR | No target lesions detected, 6 non target lesions |
| 7 | 7# subject | The total diameter of the target lesion was 76mm, with 5 non target lesions | 2.5×10⁸ | none | none | PR | The total diameter of the target lesion was 34mm, with 5 non target lesions |
| 8 | 8# subject | The total diameter of the target lesion was 29mm, with 2 non target lesions | 2.5×10⁸ | none | none | PR | No target lesion detected, 1 non target lesion |
| 9 | 9# subject | The total diameter of the target lesion was 129mm, with 3 non target lesions | 2.5×10⁸ | none | none | SD | The total diameter of the target lesion was 109mm, with 3 non target lesions |
| 10 | 10# subject | The total diameter of the target lesion was 16mm, with 4 non target lesions | 3.75×10⁸ | none | none | PD | The total diameter of the target lesion was 16mm, with 3 non target lesions |
| 11 | 11# subject | The total diameter of the target lesion was 36mm, with 3 non target lesions | 2.5×10⁸ | none | none | SD | The total diameter of the target lesion was 32mm, with 3 non target lesions |
| 12 | 12# subject | The total diameter of the target lesion was 37mm, with 2 non target lesions | 2.5×10⁸ | none | none | PR | The total diameter of the target lesion was 26mm, with 2 non target lesions |

Some patients received a second cycle of treatment, and the time of the second cycle of treatment and the treatment effect after treatment are shown in Table 3.

**Table 3 Safety and efficacy of CAR-T therapy**

| No. | patient | Second cycle | | | | | |
|---|---|---|---|---|---|---|---|
| | | How long to administer after the first cycle administration begins | Drug administration regimen | Severe CRS | Severe neurotoxicity | Response | Disease burden after the second cycle of treatment |
| 1 | 1# subject | 35 days | 5.0×10⁸ | none | none | PR | The total diameter of the target lesion was 11mm, with 2 non target lesions |
| 2 | 3# subject | 140 days | 2.5×10⁸ | none | none | PD | The total diameter of the target lesion was 8mm, with 3 non target lesions and new lesions appearing |
| 3 | 4# subject | 77 days | 2.5×10⁸ | none | none | SD | The total diameter of the target lesion was 35mm, with 3 non target lesions |
| 4 | 7# subject | 232 days | 2.5×10⁸ | none | none | PD | The total diameter of the target lesion was 45mm, with 5 non target lesions, of which 1 was a new lesion |

As shown in Figure 1, according to the evaluation of RECIST 1.1 standard, after 12 patients who failed PD-L1 or PD-1 antibody therapy received 2.5×10⁸-5.0×10⁸ CAR-T cells, 11 patients showed varying degrees of reduction in the target lesion, 6 patients achieved partial remission, the objective response rate (ORR) (95%CI) reached 50% (21.09%, 78.91%), and the disease control rate (DCR) (95%CI) was 75% (42.81%, 94.51%).

The mDOR (median duration of response) of the 6 subjects who achieved objective remission was 5.4 months (1.9, NE). Among them, one subject who had only received one treatment had a continuous remission of 6.4 months; the target lesion was not detected in the treatment effect evaluation of one patient after receiving one treatment, and the continuous remission time has exceeded 4 months.

Progression-free survival (PFS) and overall survival (OS): the median follow-up time of the 12 subjects after apheresis and the first CAR-T cell infusion was about 6.4 months and 5.6 months respectively, which was calculated using the reverse K-M curve method; using the K-M curve method to calculate mPFS (95%CI), the mPFS of all subjects receiving CAR-T cell therapy was 4.2 months (2.2, NE); the mOS (95%CI) was 7.4 months (2.6, NE) calculated using the K-M curve method. The results suggest that the subjects can obtain more obvious clinical benefits from CAR-T therapy, and the PFS and OS are significantly prolonged.

In order to further evaluate the efficacy of the cells administered in the previous cycle and in the subsequent cycle by detecting the sustained survival period of CAR-T cells in vivo (that is, the time period of continuous survival of CAR-T cells' "implantation" in vivo), starting from the end of the initial infusion (on day 0), Q-PCR was used to detect the copy number of CAR-CLDN18 DNA in peripheral blood for each visit point (the probe used was: FAM-5'-CTGAGCAGCGTGACCGCCGC-3'TAMRA; the upstream primer sequence was: 5'-TGGAGTGGATCGGCTACATC-3'; the downstream primer sequence was: 5 ' - AGTAGTAGATGGCGGTGTCG-3 ').

The cell metabolism results of 12 subjects showed that after the first infusion of CAR-T cells, the median time for the in vivo amplified copy number to reach peak was about 7 days, and the median peak value of CAR copy (minimum value, maximum value ) were 7196 (3422, 39315) [copy number/µg gDNA], respectively. CAR-T cell expansion persisted in vivo for up to 203 days after the first infusion. No obvious dose-dependent relationship was found. After the second cycle of cell infusion, the median time for the cell expansion to reach its peak was advanced to within 5 days, and the amplitude was significantly lower than that of the first cycle, and the median Cmax was 1074 copies/µg gDNA.

### (4) Security

All adverse events (AE) will be classified according to the codes of the latest version of MedDRA, the ICH International Dictionary of Medical Terms, and graded according to Common Terminology Criteria for Adverse Events (CTCAE v5.0 version), and analyzed by frequency distribution, charts or other descriptive indicators, the number and percentage of subjects with adverse events (TEAEs) that occur after treatment will be calculated by system organ classification, preferred terminology, and group.

In this trial, 94.6% of subjects developed CRS, all of which were Level 1-2. The clinical manifestations were mainly fever, tachycardia, and peripheral edema, and hypotension and hypoxemia could also be seen, which could be recovered after symptomatic and supportive treatment. For patients undergoing the second cycle of infusion, the incidence of AEs with abnormal blood test indicators was roughly similar to that after the first treatment, suggesting that reinfusion did not cause cumulative toxicity and did not increase the risk of the subjects.

### Example 3. CAR-T therapy after failure of anti-PD-1/PD-L1 antibody therapy in gastric cancer

Similar to those described in the above example, 12 new patients with CLDN18.2 positive gastric cancer received CLDN18A2-CAR-T after showing progression under PD-1 and/or PD-L1 antibody treatment (progressive disease PD according to RECIST 1.1). The PD-1 and/or PD-L1 antibodies previously used by the patients are: Camrelizumab, Pembrolizumab, Sintilimab, Toripalimab, and some monoclonal antibody in clinical trials.

According to the evaluation of RECIST 1.1 standard, after 12 new patients who had previously failed PD-L1 or PD-1 antibody therapy received about 2.5×10⁸-5.0×10⁸ CAR-T cells/cycle treatment for 1 cycle or 2 cycles, 4 patients were in SD, 6 patients were in PR, and only 2 patients were in PD, indicating that CAR-T cells can still produce significant treatment effect on gastric cancer patients who have been treated with PD-1 or PD-L1 antibodies. The CRS occurred in all subjects in this trial was level 1 or 2, and no neurotoxicity occurred. The clinical manifestations were mainly fever, tachycardia, and peripheral edema, and hypotension and hypoxemia could also be seen, which could be recovered after symptomatic and supportive treatment.

Based on comprehensive statistics, the 12 gastric cancer patients described in Example 1 and the newly added 12 gastric cancer patients described in this Example received the CLDN18A2-CAR-T treatment after showing progression (progressive disease PD according to RECIST 1.1) under PD-1 and/or PD-L1 antibodies treatment, 12 patients achieved partial remission, ORR (95% CI) reached about 50% (29.1%, 70.9%), DCR (95% CI) reached about 79.2% (57.8%, 92.9%).

The mDOR of the 12 subjects who achieved PR after CAR-T treatment was 6.3 months (1.9, 9.5). Among them, the overall survival of 6 patients reached about 11 months or more, and they were still in the survival follow-up period.

The mPFS (95%CI) was calculated by the K-M curve method, and the mPFS of all subjects receiving CAR-T cell therapy was 4.9 months (2.6, 7.4); the mOS (95%CI) calculated by the K-M curve method was 6.7 months ( 3.7, 9.0). The results suggest that the subjects could obtain more obvious clinical benefits from CAR-T therapy, and the PFS and OS were significantly prolonged.

The cell metabolism results of the 24 subjects showed that after the first infusion of CAR-T cells, the median time for the in vivo amplified copy number to reach its peak was about 7 days, and the median value of CAR copy peak (minimum value, maximum value) were 4536 (433, 39315) [copy number/µg gDNA], respectively.

### Example 4. CAR-T therapy after failure of anti-PD-1/PD-L1 antibody therapy in krukenberg tumors

Similar to those described in the above examples, patients with CLDN18.2-positive krukenberg tumors received CLDN18A2-CAR-T therapy after showing progression (progressive disease PD according to RECIST 1.1) under PD-1 antibody (Pembrolizumab) treatment, about 2.5×10⁸ CAR-T cell therapy for 1 cycle, achieved PR, and the disease progression-free survival period was about 4 months, and it was still in a stable stage. Level 1 CRS occurred in this subject, and no neurotoxicity occurred.

### Example 5. CAR-T therapy after failure of anti-PD-1/PD-L1 antibody therapy in gallbladder cancer

Similar to those described in the above examples, patients with CLDN18.2-positive gallbladder cancer received CLDN18A2-CAR-T treatment after showing progression (progressive disease PD according to RECIST 1.1) under PD-1 antibody (Pembrolizumab) treatment, about 2.5×10⁸ CAR-T cells therapy for 1 cycle, achieved SD, and the progression-free survival period was about 7.5 months, and it was still in a stable stage. Level 2 CRS occurred in this subject, and no neurotoxicity occurred.

### Example 6. CAR-T therapy after failure of anti-PD-1/PD-L1 antibody therapy in liver cancer

Similar to those described in the above example, illustrative in this example, 11 liver cancer patients with positive tumor antigen GPC3 received GPC3-CAR-T cell therapy after showing progression (progressive disease PD according to RECIST 1.1) under PD-1 and/or PD-L1 antibody treatment. The PD-1 and/or PD-L1 antibodies previously used by patients are: Camrelizumab, Pembrolizumab, Sintilimab, Toripalimab, and some monoclonal antibody in clinical trials. All of these 11 patients had received at least 1st line of standard treatment in the past, among them, 1 patient received 1st line treatment, 8 patients received 2nd line treatment, 1 patient received 4th line treatment, and 1 patient received 6th line treatment.

According to the evaluation of RECIST 1.1 standard, after the 11 patients with liver cancer in this example underwent 1 cycle or 2 cycles of treatment with about 2.5×10⁸-5.0×10⁸ CAR-T cells/cycle in, 5 patients were in SD and 1 patient achieved PR, 5 patients were in PD, indicating that CAR-T cells can still produce significant treatment effect on liver cancer patients who have been treated with PD-1 or PD-L1 antibodies, and the disease control rate reached about 54.5% (6/11). The average progression-free survival period of the 6 patients who achieved disease control was about 4.4 months, and the overall survival of 4 patients exceeded 1 year, and 3 of these 4 patients were still in the survival follow-up period.

None of the subjects in this trial developed severe uncontrollable CRS, and no neurotoxicity occurred. The clinical manifestations were mainly fever, tachycardia, and peripheral edema, and hypotension and hypoxemia could also be seen, which could be recovered after symptomatic and supportive treatment.

As described in Example 2, the copy number of CAR-GPC3 DNA in the peripheral blood of the patient was detected by the Q-PCR method. The results showed that after the first infusion of CAR-T cells, the median time for the in vivo amplified copy number to reach its peak was about 7 days.

All literatures mentioned in the present application are incorporated by reference in the present application as if each were individually incorporated by reference. In addition, it should be understood that after reading the above teaching content of the present application, those skilled in the art can make various changes or modifications to the present application, and these equivalent forms also fall within the scope defined by the appended claims of the present application.

The sequences used in this application are listed in the following table:

| SEQ ID NO: | NAMES IN THE APPLICATION | SEQUENCE |
|---|---|---|
| 1 | antibody 1-HCDR1 | SGYNWH |
| 2 | antibody 1-HCDR2 | yihytgstnynpalrs |
| 3 | antibody 1-HCDR3 | IYNGNSFPY |
| 4 | antibody 1-LCDR1 | KSSQSLFNSGNQKNYLT |
| 5 | antibody 1-LCDR2 | WASTRES |
| 6 | antibody 1-LCDR3 | QNAYSFPYT |
| 7 | antibody 1- Amino acid sequence of VH | QVQLQESGPGLIKPSQTLSLTCTVSGGSISSGYNWHWIRQPPGKGLEWI GYIHYTGSTNYNPALRSRVTISVDTSKNQFSLKLSSVTAADTAIYYCARI YNGNSFPYWGQGTTVTVSS |
| 8 | antibody 1- Nucleic acid sequence of VH | CAGGTGCAGCTGCAGGAGAGCGGCCCCGGCCTGATCAAGCCCAGCC AGACCCTGAGCCTGACCTGCACCGTGAGCGGCGGCAGCATCAGCAG CGGCTACAACTGGCACTGGATCCGGCAGCCCCCCGGCAAGGGCCTG GAGTGGATCGGCTACATCCACTACACCGGCAGCACCAACTACAACCC CGCCCTGCGGAGCCGGGTGACCATCAGCGTGGACACCAGCAAGAAC CAGTTCAGCCTGAAGCTGAGCAGCGTGACCGCCGCCGACACCGCCA TCTACTACTGCGCCCGGATCTACAACGGCAACAGCTTCCCCTACTGG GGCCAGGGCACCACCGTGACCGTGAGCAGC |
| 9 | antibody 1- Amino acid sequence of VL | divmtqspdslavslgeratinckssqslfnsgnqknyltwyqqkpgqppklliywastresgvpdrfsgsgs gtdftltisslqaedvavyycqnaysfpytfgggtkleikr |
| 10 | antibody 1- Nucleic acid sequence of VL | GACATCGTGATGACCCAGAGCCCCGACAGCCTGGCCGTGAGCCTGG GCGAGCGGGCCACCATCAACTGCAAGAGCAGCCAGAGCCTGTTCAA CAGCGGCAACCAGAAGAACTACCTGACCTGGTACCAGCAGAAGCCC GGCCAGCCCCCCAAGCTGCTGATCTACTGGGCCAGCACCCGGGAGA GCGGCGTGCCCGACCGGTTCAGCGGCAGCGGCAGCGGCACCGACTT CACCCTGACCATCAGCAGCCTGCAGGCCGAGGACGTGGCCGTGTACT ACTGCCAGAACGCCTACAGCTTCCCCTACACCTTCGGCGGCGGCACC AAGCTGGAGATCAAGCGG |
| 11 | antibody 1-28z | Qvqlqesgpglikpsqtlsltctvsggsissgynwhwirqppgkglewigyihytgstnynpalrsrvtisvdt sknqfslklssvtaadtaiyycariyngnsfpywgqgttvtvssggggsggggsggggsdivmtqspdslav slgeratinckssqslfnsgnqknyltwyqqkpgqppklliywastresgvpdrfsgsgsgtdftltisslqaed vavyycqnaysfpytfgggtkleikrTttpaprpptpaptiasqplslrpeacrpaaggavhtrgldfacdFwv lvvvggvlacysllvtvafiifwvRskrsrllhsdymnmtprrpgptrkhyqpyapprdfaayrsrvkfsrsa |
| | | dapayqqgqnqlynelnlgrreeydvldkrrgrdpemggkpqrrknpqeglynelqkdkmaeayseigm kgerrrgkghdglyqglstatkdtydalhmqalppr |
| 12 | antibody 1-BBZ | Qvqlqesgpglikpsqtlsltctvsggsissgynwhwirqppgkglewigyihytgstnynpalrsrvtisvdt sknqfslklssvtaadtaiyycariyngnsfpywgqgttvtvssggggsggggsggggsdivmtqspdslav slgeratinckssqslfnsgnqknyltwyqqkpgqppklliywastresgvpdrfsgsgsgtdftltisslqaed vavyycqnaysfpytfgggtkleikrTttpaprpptpaptiasqplslrpeacrpaaggavhtrgldfacdIyi waplagtcgvlllslvitlycKrgrkkllyifkqpfmrpvqttqeedgcscrfpeeeeggcelrvkfsrsadapa yqqgqnqlynelnlgrreeydvldkrrgrdpemggkpqrrknpqeglynelqkdkmaeayseigmkger rrgkghdglyqglstatkdtydalhmqalppr |
| 13 | antibody 1-28BBZ | Qvqlqesgpglikpsqtlsltctvsggsissgynwhwirqppgkglewigyihytgstnynpalrsrvtisvdt sknqfslklssvtaadtaiyycariyngnsfpywgqgttvtvssggggsggggsggggsdivmtqspdslav slgeratinckssqslfnsgnqknyltwyqqkpgqppklliywastresgvpdrfsgsgsgtdftltisslqaed vavyycqnaysfpytfgggtkleikrTttpaprpptpaptiasqplslrpeacrpaaggavhtrgldfacdFwv lvvvggvlacysllvtvafiifwvRskrsrllhsdymnmtprrpgptrkhyqpyapprdfaayrsKrgrkkll yifkqpfmrpvqttqeedgcscrfpeeeeggcelrvkfsrsadapayqqgqnqlynelnlgrreeydvldkrr grdpemggkpqrrknpqeglynelqkdkmaeayseigmkgerrrgkghdglyqglstatkdtydalhmq alppr |
| 14 | antibody 1- Amino acid sequence of scFv | qvqlqesgpglikpsqtlsltctvsggsissgynwhwirqppgkglewigyihytgstnynpalrsrvtisvdt sknqfslklssvtaadtaiyycariyngnsfpywgqgttvtvssggggsggggsggggsdivmtqspdslav slgeratinckssqslfnsgnqknyltwyqqkpgqppklliywastresgvpdrfsgsgsgtdftltisslqaed vavyycqnaysfpytfgggtkleikr |
| 15 | antibody 1- Nucleic acid sequence of scFv | Caggtgcagctgcaggagagcggccccggcctgatcaagcccagccagaccctgagcctgacctgcaccgt gagcggcggcagcatcagcagcggctacaactggcactggatccggcagcccccggcaagggcctggagt ggatcggctacatccactacaccggcagcaccaactacaaccccgccctgcggagccgggtgaccatcagcgt ggacaccagcaagaaccagttcagcctgaagctgagcagcgtgaccgccgccgacaccgccatctactactgc gcccggatctacaacggcaacagcttcccctactggggccagggcaccaccgtgaccgtgagcagcggtgga ggcggttcaggcggaggtggttctggcggtggcggatcggacatcgtgatgacccagagccccgacagcctg gccgtgagcctgggcgagcgggccaccatcaactgcaagagcagccagagcctgttcaacagcggcaacca gaagaactacctgacctggtaccagcagaagcccggccagccccccaagctgctgatctactgggccagcacc cgggagagcggcgtgcccgaccggttcagcggcagcggcagcggcaccgacttcaccctgaccatcagcag cctgcaggccgaggacgtggccgtgtactactgccagaacgcctacagcttcccctacaccttcggcggcggc accaagctggagatcaagcgg |
| 16 | antibody 2-HCDR1 | DYGVS |
| 17 | antibody 2-HCDR2 | VIWGSETTYYNSALKS |
| 18 | antibody 2-HCDR3 | HYYYGGSYAMDY |
| 19 | antibody 2-LCDR1 | RASQDISKYLN |
| 20 | antibody 2-LCDR2 | HTSRLHS |
| 21 | antibody 2-LCDR3 | QQGNTLPYT |
| 22 | antibody 2-VH | evklqesgpglvapsqslsvtctvsgvslpdygvswirqpprkglewlgviwgsettyynsalksrltiikdns ksqvflkmnslqtddtaiyycakhyyyggsyamdywgqgtsvtvss |
| 23 | antibody 2-VL | diqmtqttsslsaslgdrvtiscrasqdiskylnwyqqkpdgtvklliyhtsrlhsgvpsrfsgsgsgtdysltisn leqediatyfcqqgntlpytfgggtkleit |
| 24 | antibody 2-scFv | diqmtqttsslsaslgdrvtiscrasqdiskylnwyqqkpdgtvklliyhtsrlhsgvpsrfsgsgsgtdysltisn leqediatyfcqqgntlpytfgggtkleitggggsggggsggggsevklqesgpglvapsqslsvtctvsgvsl pdygvswirqpprkglewlgviwgsettyynsalksrltiikdnsksqvflkmnslqtddtaiyycakhyyy ggsyamdywgqgtsvtvss |
| 25 | antibody 2-BBz | diqmtqttsslsaslgdrvtiscrasqdiskylnwyqqkpdgtvklliyhtsrlhsgvpsrfsgsgsgtdysltisn leqediatyfcqqgntlpytfgggtkleitggggsggggsggggsevklqesgpglvapsqslsvtctvsgvsl pdygvswirqpprkglewlgviwgsettyynsalksrltiikdnsksqvflkmnslqtddtaiyycakhyyy ggsyamdywgqgtsvtvssttpaprpptpaptiasqplslrpeacrpaaggavhtrgldfacdiyiwaplagt cgvllls lvitlyckrgrkkllyifkqpfmrpvqttqeedgcscrfpeeeeggcelrvkfsrsadapaykqgqnq lynelnlgrreeydvldkrrgrdpemggkprrknpqeglynelqkdkmaeayseigmkgerrrgkghdgl yqglstatkdtydalhmqalppr |
| 26 | antibody 2-BBz-T2A-EGFRt | diqmtqttsslsaslgdrvtiscrasqdiskylnwyqqkpdgtvklliyhtsrlhsgvpsrfsgsgsgtdysltisn leqediatyfcqqgntlpytfgggtkleitgstsgsgkpgsgegstkgevklqesgpglvapsqslsvtctvsgv slpdygvswirqpprkglewlgviwgsettyynsalksrltiikdnsksqvflkmnslqtddtaiyycakhyy yggsyamdywgqgtsvtvsseskygppcppcpmfwvlvvvggvlacysllvtvafiifwvkrgrkkllyif kqpfmrpvqttqeedgcscrfpeeeeggcelrvkfsrsadapayqqgqnqlynelnlgrreeydvldkrrgrd pemggkprrknpqeglynelqkdkmaeayseigmkgerrrgkghdglyqglstatkdtydalhmqalpp rlegggegrgsllltcgdveenpgprmlllvtslllcelphpaflliprkvcngigigefkdslsinatnikhfkncts isgdlhilpvafrgdsfthtppldpqeldilktvkeitgflliqawpenrtdlhafenleiirgrtkqhgqfslavvsl nitslglrslkeisdgdviisgnknlcyantinwkklfgtsgqktkiisnrgensckatgqvchalcspegcwgp eprdcvscrnvsrgrecvdkcnllegeprefvenseciqchpeclpqamnitctgrgpdnciqcahyidgph cvktcpagvmgenntlvwkyadaghvchlchpnctygctgpglegcptngpkipsiatgmvgalllllvva lgiglfm |
| 27 | antibody 3-HCDR1 | DYSIN |
| 28 | antibody 3-HCDR2 | WINTETREPAYAYDFRG |
| 29 | antibody 3-HCDR3 | DYSYAMDY |
| 30 | antibody 3-LCDR1 | RASESVTILGSHLIH |
| 31 | antibody 3-LCDR2 | LASNVQT |
| 32 | antibody 3-LCDR3 | LQSRTIPRT |
| 33 | BB2121 VH | qiqlvqsgpelkkpgetvkisckasgytftdysinwvkrapgkglkwmgwintetrepayaydfrgrfafsl etsastaylqinnlkyedtatyfcaldysyamdywgqgtsvtvss |
| 34 | BB2121 VL | divltqsppslamslgkratiscrasesvtilgshlihwyqqkpgqpptlliqlasnvqtgvparfsgsgsrtdftl tidpveeddvavyyclqsrtiprtfgggtkleik |
| 35 | antibody 3-scFv | divltqsppslamslgkratiscrasesvtilgshlihwyqqkpgqpptlliqlasnvqtgvparfsgsgsrtdftl tidpveeddvavyyclqsrtiprtfgggtkleikgstsgsgkpgsgegstkgqiqlvqsgpelkkpgetvkisc kasgytftdysinwvkrapgkglkwmgwintetrepayaydfrgrfafsletsastaylqinnlkyedtatyfc aldysyamdywgqgtsvtvss |
| 36 | antibody 3-BBZ | divltqsppslamslgkratiscrasesvtilgshlihwyqqkpgqpptlliqlasnvqtgvparfsgsgsrtdftl tidpveeddvavyyclqsrtiprtfgggtkleikgstsgsgkpgsgegstkgqiqlvqsgpelkkpgetvkisc kasgytftdysinwvkrapgkglkwmgwintetrepayaydfrgrfafsletsastaylqinnlkyedtatyfc aldysyamdywgqgtsvtvssaaatttpaprpptpaptiasqplslrpeacrpaaggavhtrgldfacdiyiwa plagtcgvlllslvitlyckrgrkkllyifkqpfmrpvqttqeedgcscrfpeeeeggcelrvkfsrsadapayqq gqnqlynelnlgrreeydvldkrrgrdpemggkprrknpqeglynelqkdkmaeayseigmkgerrrgkg hdglyqglstatkdtydalhmqalppr |
| 37 | antibody 4- antibody sequence | qvkleesggglvqagrslrlscaasehtfsshvmgwfrqapgkeresvavigwrdistsyadsvkgrftisrdn akktlylqmnslkpedtavyycaarridaadfdswgqgtqvtvssggggsevqlvesggglvqaggslrlsc aasgrtftmgwfrqapgkerefvaaislsptlayyaesvkgrftisrdnakntvvlqmnslkpedtalyycaad rksvmsirpdywgqgtqvtvss |
| 38 | antibody 4-BBZ | qvkleesggglvqagrslrlscaasehtfsshvmgwfrqapgkeresvavigwrdistsyadsvkgrftisrdn akktlylqmnslkpedtavyycaarridaadfdswgqgtqvtvssggggsevqlvesggglvqaggslrlsc aasgrtftmgwfrqapgkerefvaaislsptlayyaesvkgrftisrdnakntvvlqmnslkpedtalyycaad rksvmsirpdywgqgtqvtvssststttpaprpptpaptiasqplslrpeacrpaaggavhtrgldfacdiyiwa plagtcgvlllslvitlyckrgrkkllyifkqpfmrpvqttqeedgcscrfpeeeeggcelrvkfsrsadapayqq gqnqlynelnlgrreeydvldkrrgrdpemggkprrknpqeglynelqkdkmaeayseigmkgerrrgkg hdglyqglstatkdtydalhmqalppr |
| 39 | Antigen extracellular domain of PD-1 | FLDSPDRPWNPPTFSPALLVVTEGDNATFTCSFSNTSESFVLNWYRMSPS NQTDKLAAFPEDRSQPGQDCRFRVTQLPNGRDFHMSVVRARRNDSGT YLCGAISLAPKAQIKESLRAELRVTERRAEVPTAHPSPSPRPAGQFQTLV |
| 40 | Antigen extracellular domain of PD-L1 | FTVTVPKDLYVVEYGSNMTIECKFPVEKQLDLAALIVYWEMEDKNIIQF VHGEEDLKVQHSSYRQRARLLKDQLSLGNAALQITDVKLQDAGVYRC MISYGGADYKRITVKVNAPYNKINQRILVVDPVTSEHELTCQAEGYPKA EVIWTSSDHQVLSGKTTTTNSKREEKLFNVTSTLRINTTTNEIFYCTFRR LDPEENHTAELVIPELPLAHPPNER |
| 41 | Amino acids of the hinge region of CD8α | TTTPAPRPPTPAPTIASQPLSLRPEACRPAAGGAVHTRGLDFACD |
| 42 | Amino acids of transmembrane domain of CD28 | FWVLVVVGGVLACYSLLVTVAFIIFWV |
| 43 | Amino acids of transmembrane domain of CD8 | IYIWAPLAGTCGVLLLSLVITLYC |
| 44 | Amino acids of intracellular signaling domain of CD137 | KRGRKKLLYIFKQPFMRPVQTTQEEDGCSCRFPEEEEGGCEL |
| 45 | Amino acids of intracellular signaling domain of CD28 | RSKRSRLLHSDYMNMTPRRPGPTRKHYQPYAPPRDFAAYRS |
| 46 | Amino acids of CD3ζ | RVKFSRSADAPAYQQGQNQLYNELNLGRREEYDVLDKRRGRDPEMGG KPQRRKNPQEGLYNELQKDKMAEAYSEIGMKGERRRGKGHDGLYQGL STATKDTYDALHMQALPPR |
| 47 | Nucleic acid of signal peptide of CD8α | ATGGCCTTACCAGTGACCGCCTTGCTCCTGCCGCTGGCCTTGCTGCTC CACGCCGCCAGGCCG |
| 48 | nucleic acids of the hinge region of CD8α | ACCACGACGCCAGCGCCGCGACCACCAACACCGGCGCCCACCATCG CGTCGCAGCCCCTGTCCCTGCGCCCAGAGGCGTGCCGGCCAGCGGC GGGGGCGCAGTGCACACGAGGGGGCTGGACTTCGCCTGTGAT |
| 49 | nucleic acids of transmembrane domain of CD28 | TTTTGGGTGCTGGTGGTGGTTGGTGGAGTCCTGGCTTGCTATAGCTTG CTAGTAACAGTGGCCTTTATTATTTCTGGGTG |
| 50 | Nucleic acid of intracellular signaling domain of CD28 | AGGAGTAAGAGGAGCAGGCTCCTGCACAGTGACTACATGAACATGA CTCCCCGCCGCCCCGGGCCAACCCGCAAGCATTACCAGCCCTATGCC CCACCACGCGACTTCGCAGCCTATCGCTCC |
| 51 | nucleic acids of CD3ζ | AGAGTGAAGTTCAGCAGGAGCGCAGACGCCCCCGCGTACCAGCAGG GCCAGAACCAGCTCTATAACGAGCTCAATCTAGGACGAAGAGAGGA GTACGATGTTTTGGACAAGAGACGTGGCCGGGACCCTGAGATGGGG GGAAAGCCGCAGAGAAGGAAGAACCCTCAGGAAGGCCTGTACAAT GAACTGCAGAAAGATAAGATGGCGGAGGCCTACAGTGAGATTGGGA TGAAAGGCGAGCGCCGGAGGGGCAAGGGGCACGATGGCCTTTACCA GGGTCTCAGTACAGCCACCAAGGACACCTACGACGCCCTTCACATGC AGGCCCTGCCCCCTCGC |
| 52 | antibody 2-28Z | diqmtqttsslsaslgdrvtiscrasqdiskylnwyqqkpdgtvklliyhtsrlhsgvpsrfsgsgsgtdysltisn leqediatyfcqqgntlpytfgggtkleitgstsgsgkpgsgegstkgevklqesgpglvapsqslsvtctvsgv slpdygvswirqpprkglewlgviwgsettyynsalksrltiikdnsksqvflkmnslqtddtaiyycakhyy yggsyamdywgqgtsvtvssaaaievmypppyldneksngtiihvkgkhlcpsplfpgpskpfwvlvvv ggvlacysllvtvafiifwvrskrsrllhsdymnmtprrpgptrkhyqpyapprdfaayrsrvkfsrsadapay qqgqnqlynelnlgrreeydvldkrrgrdpemggkprrknpqeglynelqkdkmaeayseigmkgerrrg kghdglyqglstatkdtydalhmqalppr |
| 53 | Amino acids of signal peptides of CD8α | MALPVTALLLPLALLLHAARP |
| 54 | nucleic acids of transmembrane domain of CD8 | ATCTACATCTGGGCGCCCTTGGCCGGGACTTGTGGGGTCCTTCTCCTG TCACTGGTTATCACCCTTTACTGC |
| 55 | Nucleic acids of intracellular signaling domain of CD137 | AAACGGGGCAGAAAGAAACTCCTGTATATATTCAAACAACCATTTAT GAGACCAGTACAAACTACTCAAGAGGAAGATGGCTGTAGCTGCCGA TTTCCAGAAGAAGAAGAAGGAGGATGTGAACTG |
| 56 | Amino acids of Human CLD18A2 | MAVTACQGLGFVVSLIGIAGIIAATCMDQWSTQDLYNNPVTAVFNYQGL WRSCVRESSGFTECRGYFTLLGLPAMLQAVRALMIVGIVLGAIGLLVSIF ALKCIRIGSMEDSAKANMTLTSGIMFIVSGLCAIAGVSVFANMLVTNFW MSTANMYTGMGGMVQTVQTRYTFGAALFVGWVAGGLTLIGGVMMCI ACRGLAPEETNYKAVSYHASGHSVAYKPGGFKASTGFGSNTKNKKIYD GGARTEDEVQSYPSKHDYV |
| 57 | Nucleic acids of Human CLDN18A2 | ATGGCCGTGACTGCCTGTCAGGGCTTGGGGTTCGTGGTTTCACTGAT TGGGATTGCGGGCATCATTGCTGCCACCTGCATGGACCAGTGGAGCA CCCAAGACTTGTACAACAACCCCGTAACAGCTGTTTTCAACTACCAG GGGCTGTGGCGCTCCTGTGTCCGAGAGAGCTCTGGCTTCACCGAGT GCCGGGGCTACTTCACCCTGCTGGGGCTGCCAGCCATGCTGCAGGCA GTGCGAGCCCTGATGATCGTAGGCATCGTCCTGGGTGCCATTGGCCT CCTGGTATCCATCTTTGCCCTGAAATGCATCCGCATTGGCAGCATGGA GGACTCTGCCAAAGCCAACATGACACTGACCTCCGGGATCATGTTCA TTGTCTCAGGTCTTTGTGCAATTGCTGGAGTGTCTGTGTTTGCCAACA TGCTGGTGACTAACTTCTGGATGTCCACAGCTAACATGTACACCGGC ATGGGTGGGATGGTGCAGACTGTTCAGACCAGGTACACATTTGGTGC GGCTCTGTTCGTGGGCTGGGTCGCTGGAGGCCTCACACTAATTGGGG GTGTGATGATGTGCATCGCCTGCCGGGGCCTGGCACCAGAAGAAAC CAACTACAAAGCCGTTTCTTATCATGCCTCAGGCCACAGTGTTGCCTA CAAGCCTGGAGGCTTCAAGGCCAGCACTGGCTTTGGGTCCAACACC AAAAACAAGAAGATATACGATGGAGGTGCCCGCACAGAGGACGAGG TACAATCTTATCCTTCCAAGCACGACTATGTG |
| 58 | antibody 5-scFv | qvqlqqsgaelarpgasvkmsckasgytftsytmhwvkqrpgqglewigyinpssgytnynqkfkdkatlt adkssstaymqlssltsedsavyycariyygnsfaywgqgttvtvssggggsggggsggggsdivmtqsps sltvtagekvtmsckssqsllnsgnqknyltwyqqkpgqppklliywastresgvpdrftgsgsgtdftltissv qaedlavyycqndysypltfgagtklelkr |
| 59 | antibody 6-scFv | QVQLQQSGPELVKPGALVKISCKASGYTFTSYDINWVKQRPGQGLEWI GWIYPGDGSTKYNEKFKGKATLTADKSSSTAYMQLSSLTSENSAVYFCA RGGYRYDEAMDYWGQGTTVTVSSggggsggggsggggsQIVLTQSPAIMSAS |
| | | PGEKVTMTCSASSSISYMHWYQQKPGTSPKRWIYDTSKLASGVPARFS GSGSGTSYSLTISSMEAEDAATYYCHQRSSYPYTFGGGTKLEIKR |
| 60 | antibody 7-scFv | qiqlvqsgpelkkpgetvkisckasgytftnygmnwvkqapgkglkwmgwintntgeptyaeefkgrfaf sletsastaylqinnlknedtatyfcarfsygnsfaywgqgttvtvssggggsggggsggggsdivmtqspssl svsagekvtmsckssqsllnsgnqknylawyqqkpgqppklliygastresgvpdrftgsgsgtdftltissvq aedlavyycqndhsypltfgagtklelkr |
| 61 | antibody 8-scFv | dvqlqesgpdlvkpsqslsltctvtgysitsgynwhwirqfpgnkmewmgyihytgstnynpslrsrisitrd tsknqfflqlnsvttddtatyyctriyngnsfpywgqgtsvtvssggggsggggsggggsdivmtqspssltvt pgekvtmtckssqslfnsgnqknyltwyqqrpgqppkmliywastresgvpdrftgsgsgtdftltissvqae dlavfycqnaysfpytfgggtkleikr |
| 62 | antibody 9-scFv | qvqlqqsgaelarpgasvkmsckasgytftsytmhwvkqrpgqglewigyidpssgytnynqkfkdkatlt adkssstaymqlssltsedsavyycariyygnsfaywgqgttvtvssggggsggggsggggsdivmtqsps sltvtagekvtmsckssqsllnsgnqknyltwyqqkpgqppklliywastresgvpdrftgsgsgtdftltissv qaedlavyycqndysypltfgagtklelkr |
| 63 | antibody 10 -scFv | qvqlqqsgaelarpgasvkmsckasgytftsytmhwvkqrpgqglewigyinpasgytnynqkfkdkatl tadkssstaymqlssltsedsavyycariyygnsfaywgqgttvtvssggggsggggsggggsdivmtqsps sltvtagekvtmsckssqsllnsgnqknyltwyqqkpgqppklliywastresgvpdrftgsgsgtdftltissv qaedlavyycqndysypltfgagtklelkr |
| 64 | antibody 11 -scFv | qvqlvqsgaevkkpgasvkvsckasgytftsytmhwvrqapgqglewmgyinpasgytnynqkfkdrvt mtrdtststaymelsslrsedtavyycariyygnsfaywgqgtlvtvssggggsggggsggggsdivmtqsp dslavslgeratinckssqsllnsgnqknyltwyqqkpgqppklliywastresgvpdrfsgsgsgtdftltissl qaedvavyycqndysypltfgggtkveikr |
| 65 | antibody 12 -scFv | qvqlqesgpglvkpsqtlsltctvsggsissgynwhwirqppgkglewigyihytgstnynpalrsrvtisvdt sknqfslklssvtaadtavyycariyngnsfpywgqgttvtvssggggsggggsggggsdivmtqspdslav slgeratinckssqslfnsgnqknyltwyqqkpgqppklliywastresgvpdrfsgsgsgtdftltisslqaed vavyycqnaysfpytfgggtkleikr |
| 66 | antibody 13-scFv | qiqlvqsgpelkkpgetvkisckasgytftnygmnwvkqapgkglkwmgwintntgeptyaeefkgrfaf sletsastaylqinnlknedtatyfcarlgfgnamdywgqgtsvtvssggggsggggsggggsdivmtqsps sltvtagekvtmsckssqsllnsgnqknyltwyqqkpgqppklliywastresgvpdrftgsgsgtdftltissv qaedlavyycqndysypltfgagtklelkr |
| 67 | antibody 14-scFv | qvqlqqpgaelvrpgasvklsckasgytftsywinwvkqrpgqglewigniypsdsytnynqkfkdkatlt vdkssstaymqlssptsedsavyyctrswrgnsfdywgqgttltvssggggsggggsggggsdivmtqsps sltvtagekvtmsckssqsllnsgnqknyltwyqqkpgqppklliywastresgvpdrftgsgsgtdftltissv qaedlavyycqndysypftfgsgtkleikr |
| 68 | Amino acids of Human GPC3 | magtvrtaclvvamllsldfpgqaqpppppdatchqvrsffqrlqpglkwvpetpvpgsdlqvclpkgptc csrkmeekyqltarlnmeqllqsasmelkfliiqnaavfqeafeivvrhaknytnamfknnypsltpqafefv geffdvslyilgsdinvddmvnelfdslfpviytqlmnpglpdsaldineclrgarrdlkvfgnfpklimtqvs kslqvtriflqalnlgievinttdhlkfskdcgrmltrmwycsycqglmmvkpcggycnvvmqgcmagv |
| | | veidkywreyilsleelvngmyriydmenvllglfstihdsiqyvqknagkltttigklcahsqqrqyrsayyp edlfidkkvlkvahveheetlssrrreliqklksfisfysalpgyicshspvaendtlcwngqelverysqkaar ngmknqfnlhelkmkgpepvvsqiidklkhinqllrtmsmpkgrvldknldeegfesgdcgddedecigg sgdgmikvknqlrflaelaydldvddapgnsqqatpkdneistfhnlgnvhsplklltsmaisvvcffflvh |
| 69 | Nucleic acids of Human GPC3 | atggccgggaccgtgcgcaccgcgtgcttggtggtggcgatgctgctcagcttggacttcccgggacaggcgc agcccccgccgccgccgccggacgccacctgtcaccaagtccgctccttcttccagagactgcagcccggact caagtgggtgccagaaactcccgtgccaggatcagatttgcaagtatgtctccctaagggcccaacatgctgctc aagaaagatggaagaaaaataccaactaacagcacgattgaacatggaacagctgcttcagtctgcaagtatgg agctcaagttcttaattattcagaatgctgcggttttccaagaggcctttgaaattgttgttcgccatgccaagaacta caccaatgccatgttcaagaacaactacccaagcctgactccacaagcttttgagtttgtgggtgaatttttcacaga tgtgtctctctacatcttgggttctgacatcaatgtagatgacatggtcaatgaattgtttgacagcctgtttccagtcat ctatacccagctaatgaacccaggcctgcctgattcagccttggacatcaatgagtgcctccgaggagcaagacg tgacctgaaagtatttgggaatttccccaagcttattatgacccaggtttccaagtcactgcaagtcactaggatcttc cttcaggctctgaatcttggaattgaagtgatcaacacaactgatcacctgaagttcagtaaggactgtggccgaat gctcaccagaatgtggtactgctcttactgccagggactgatgatggttaaaccctgtggcggttactgcaatgtgg tcatgcaaggctgtatggcaggtgtggtggagattgacaagtactggagagaatacattctgtcccttgaagaactt gtgaatggcatgtacagaatctatgacatggagaacgtactgcttggtctcttttcaacaatccatgattctatccagt atgtccagaagaatgcaggaaagctgaccaccactattggcaagttatgtgcccattctcaacaacgccaatatag atctgcttattatcctgaagatctctttattgacaagaaagtattaaaagttgctcatgtagaacatgaagaaaccttat ccagccgaagaagggaactaattcagaagttgaagtctttcatcagcttctatagtgctttgcctggctacatctgca gccatagccctgtggcggaaaacgacaccctttgctggaatggacaagaactcgtggagagatacagccaaaa ggcagcaaggaatggaatgaaaaaccagttcaatctccatgagctgaaaatgaagggccctgagccagtggtc agtcaaattattgacaaactgaagcacattaaccagctcctgagaaccatgtctatgcccaaaggtagagttctgg ataaaaacctggatgaggaagggtttgaaagtggagactgcggtgatgatgaagatgagtgcattggaggctct ggtgatggaatgataaaagtgaagaatcagctccgcttccttgcagaactggcctatgatctggatgtggatgatg cgcctggaaacagtcagcaggcaactccgaaggacaacgagataagcacctttcacaacctcgggaacgttcat tccccgctgaagcttctcaccagcatggccatctcggtggtgtgcttcttcttcctggtgcactga |
| 70 | antibody 15-scFv | QVQLQESGGGLVQPGRSLRLSCAASGFTFSSYAMHWVRQAPGKGLEW VSAISGSGGSTYYADSVKGRFTISRDNSKNTLYLQMNSLRAEDTAVYYC AKDRRGSHADAFDVWGQGTLVTVSSGGGGSGGGGSGGGGSQSALTQP PSASGSPGQSVTISCTGTSSDVGGYNYVSWYQQYPGKAPKLLIYGNSN RPSGVPDRFSGSKSGTSASLAITGLQAEDGADYYCQSYDSSLRVVFGGG TKVTVLG |
| 71 | antibody 16- scFv | QVQLQESGGGLVQPGRSLRLSCAASGFTFSTYAMTWVRQAPGKGLEW VSSISSSGESTYYADSVKGRFTISRDNSKNTLYLQMNSLRAEDTAVYYC AKDRRGSHADAFDVWGQGTLVTVSSGGGGSGGGGSGGGGSQSALTQP PSASGSPGQSVTISCTGTSSDVGGYNYVSWYQQYPGKAPKLLIYGNSN RPSGVPDRFSGSKSGTSASLAITGLQAEDGADYYCQSYDSSLRVVFGGG |
| | | TKVTVLG |
| 72 | antibody 17-scFv | QVQLQESGGGLVQPGRSLRLSCAASGFTFSTYAMAWVRQAPGKGLEW VSEISSSGSRTYYADSVKGRFTISRDNSKNTLYLQMNSLRAEDTAVYYC AKDRRGSHADAFDVWGQGTLVTVSSGGGGSGGGGSGGGGSQSALTQP PSASGSPGQSVTISCTGTSSDVGGYNYVSWYQQYPGKAPKLLIYGNSN RPSGVPDRFSGSKSGTSASLAITGLQAEDGADYYCQSYDSSLRVVFGGG TKVTVLG |
| 73 | antibody 18-scFv | QVQLQESGGGLVQPGRSLRLSCAASGFTFSTYAMNWVRQAPGKGLEW VSAISMSGESTYYADSVKGRFTISRDNSKNTLYLQMNSLRAEDTAVYYC AKDRRGSHADAFDVWGQGTLVTVSSGGGGSGGGGSGGGGSQSALTQP PSASGSPGQSVTISCTGTSSDVGGYNYVSWYQQYPGKAPKLLIYGNSN RPSGVPDRFSGSKSGTSASLAITGLQAEDGADYYCQSYDSSLRVVFGGG TKVTVLG |
| 74 | antibody 19-scFv | QVQLQESGGGLVQPGRSLRLSCAASGFTFSSYAMHWVRQAPGKGLEW VSAISSSGGSTYYADSVKGRFTISRDNSKNTLYLQMNSLRAEDTAVYYC AKDRRGSHADAFDVWGQGTLVTVSSGGGGSGGGGSGGGGSQSALTQP PSASGSPGQSVTISCTGTSSDVGHKFPVSWYQQYPGKAPKLLIYKNLLR PSGVPDRFSGSKSGTSASLAITGLQAEDGADYYCQSYDSSLRVVFGGGT KVTVLG |
| 75 | antibody 20-scFv | QVQLQESGGGLVQPGRSLRLSCAASGFTFSSYAMHWVRQAPGKGLEW VSAISSSGGSTYYADSVKGRFTISRDNSKNTLYLQMNSLRAEDTAVYYC AKDRRGSHADAFDVWGQGTLVTVSSGGGGSGGGGSGGGGSQSALTQP PSASGSPGQSVTISCTGTSSDVGLMHNVSWYQQYPGKAPKLLIYKSSSR PSGVPDRFSGSKSGTSASLAITGLQAEDGADYYCQSYDSSLRVVFGGGT KVTVLG |
| 76 | antibody 21-scFv | QVQLQESGGGLVQPGRSLRLSCAASGFTFSSYAMHWVRQAPGKGLEW VSAISSSGRSTYYADSVEGRFTISRDNSKNTLYLQMNSLRAEDTAVYYC AKDRRGSHADALNVWGQGTLVTVSSGGGGSGGGGSGGGGSQSALTQP PSASGSPGQSVTISCTGTSSDVGGYNYVSWYQQYPGKAPKLLIYGNSN RPSGVPDRFSGSKSGTSASLAITGLQAEDGADYYCQSYDSSLRVVFGGG TKVTVLG |
| 77 | antibody 22-scFv | EVQLVQSGAEVKKPGASVKVSCKASGYTFSDYEMHWVRQAPGQGLE WMGAIHPGSGDTAYNQRFKGRVTITADKSTSTAYMELSSLRSEDTAVYY CARFYSYAYWGQGTLVTVSAGGGGSGGGGSGGGGSDIVMTQTPLSLPV TPGEPASISCRSSQSLVHSNGNTYLQWYLQKPGQSPQLLIYKVSNRFSGV PDRFSGSGSGTDFTLKISRVEAEDVGVYYCSQSIYVPYTFGQGTKLEIKR |
| 78 | antibody 23-scFv | QVQLQQSGTELVRPGASVKLSCKALGYTFTDYEMHWVKQTPVHGLE WIGAIHPGSGDTAYNQRFKGKATLTADKSSSTAYMEYSSLTSEDSAVYY |
| | | CTRFYSYAYWGQGTLVTVSAggggsggggsggggsDVVMTQTPLSLPVSLGD QASISCRSSQSLVHSNGNTYLQWYLQKPGQSPKLLIYKVSNRFSGVPDR FSGRGSGTDFTLKISRVEAEDLGVYFCSQSIYVPYTFGGGTKLEIKR |
| 79 | antibody 24-scFv | dvvmtqsplslpvtpgepasiscrssqslvhsnantylhwylqkpgqspqlliykvsnrfsgvpdrfsgsgsg tdftlkisrveaedvgvyycsqnthvpptfgqgtkleikrggggsggggsggggsqvqlvqsgaevkkpgas vkvsckasgytftdyemhwvrqapgqglewmgaldpktgdtaysqkfkgrvtltadeststaymelsslrse dtavyyctrfysytywgqgtlvtvss |
| 80 | 28Z | TTTPAPRPPTPAPTIASQPLSLRPEACRPAAGGAVHTRGLDFACDFWVLV VVGGVLACYSLLVTVAFIIFWVRSKRSRLLHSDYMNMTPRRPGPTRKH YQPYAPPRDFAAYRSRVKFSRSADAPAYQQGQNQLYNELNLGRREEYD VLDKRRGRDPEMGGKPQRRKNPQEGLYNELQKDKMAEAYSEIGMKGE RRRGKGHDGLYQGLSTATKDTYDALHMQALPPR |
| 81 | BBZ | TTTPAPRPPTPAPTIASQPLSLRPEACRPAAGGAVHTRGLDFACDIYIWAP LAGTCGVLLLSLVITLYCKRGRKKLLYIFKQPFMRPVQTTQEEDGCSCR FPEEEEGGCELRVKFSRSADAPAYQQGQNQLYNELNLGRREEYDVLDK RRGRDPEMGGKPQRRKNPQEGLYNELQKDKMAEAYSEIGMKGERRRG KGHDGLYQGLSTATKDTYDALHMQALPPR |
| 82 | 28BBZ | TTTPAPRPPTPAPTIASQPLSLRPEACRPAAGGAVHTRGLDFACDFWVLV VVGGVLACYSLLVTVAFIIFWVRSKRSRLLHSDYMNMTPRRPGPTRKH YQPYAPPRDFAAYRSKRGRKKLLYIFKQPFMRPVQTTQEEDGCSCRFPE EEEGGCELRVKFSRSADAPAYQQGQNQLYNELNLGRREEYDVLDKRRG RDPEMGGKPQRRKNPQEGLYNELQKDKMAEAYSEIGMKGERRRGKGH DGLYQGLSTATKDTYDALHMQALPPR |
| 83 | antibody 22-28Z | EVQLVQSGAEVKKPGASVKVSCKASGYTFSDYEMHWVRQAPGQGLE WMGAIHPGSGDTAYNQRFKGRVTITADKSTSTAYMELSSLRSEDTAVYY CARFYSYAYWGQGTLVTVSAGGGGSGGGGSGGGGSDIVMTQTPLSLPV TPGEPASISCRSSQSLVHSNGNTYLQWYLQKPGQSPQLLIYKVSNRFSGV PDRFSGSGSGTDFTLKISRVEAEDVGVYYCSQSIYVPYTFGQGTKLEIKR TTTPAPRPPTPAPTIASQPLSLRPEACRPAAGGAVHTRGLDFACDFWVLV VVGGVLACYSLLVTVAFIIFWVRSKRSRLLHSDYMNMTPRRPGPTRKH YQPYAPPRDFAAYRSRVKFSRSADAPAYQQGQNQLYNELNLGRREEYD VLDKRRGRDPEMGGKPQRRKNPQEGLYNELQKDKMAEAYSEIGMKGE RRRGKGHDGLYQGLSTATKDTYDALHMQALPPR |
| 84 | antibody 22-BBZ | EVQLVQSGAEVKKPGASVKVSCKASGYTFSDYEMHWVRQAPGQGLE WMGAIHPGSGDTAYNQRFKGRVTITADKSTSTAYMELSSLRSEDTAVYY CARFYSYAYWGQGTLVTVSAGGGGSGGGGSGGGGSDIVMTQTPLSLPV TPGEPASISCRSSQSLVHSNGNTYLQWYLQKPGQSPQLLIYKVSNRFSGV PDRFSGSGSGTDFTLKISRVEAEDVGVYYCSQSIYVPYTFGQGTKLEIKR |
| | | TTTPAPRPPTPAPTIASQPLSLRPEACRPAAGGAVHTRGLDFACDIYIWAP LAGTCGVLLLSLVITLYCKRGRKKLLYIFKQPFMRPVQTTQEEDGCSCR FPEEEEGGCEL RVKFSRSADAPAYQQGQNQLYNELNLGRREEYDVLDKRRGRDPEMGG KPQRRKNPQEGLYNELQKDKMAEAYSEIGMKGERRRGKGHDGLYQGL STATKDTYDALHMQALPPR |
| 85 | antibody 22-28BBZ | EVQLVQSGAEVKKPGASVKVSCKASGYTFSDYEMHWVRQAPGQGLE WMGAIHPGSGDTAYNQRFKGRVTITADKSTSTAYMELSSLRSEDTAVYY CARFYSYAYWGQGTLVTVSAGGGGSGGGGSGGGGSDIVMTQTPLSLPV TPGEPASISCRSSQSLVHSNGNTYLQWYLQKPGQSPQLLIYKVSNRFSGV PDRFSGSGSGTDFTLKISRVEAEDVGVYYCSQSIYVPYTFGQGTKLEIKR TTTPAPRPPTPAPTIASQPLSLRPEACRPAAGGAVHTRGLDFACDFWVLV VVGGVLACYSLLVTVAFIIFWVRSKRSRLLHSDYMNMTPRRPGPTRKH YQPYAPPRDFAAYRSKRGRKKLLYIFKQPFMRPVQTTQEEDGCSCRFPE EEEGGCELRVKFSRSADAPAYQQGQNQLYNELNLGRREEYDVLDKRRG RDPEMGGKPQRRKNPQEGLYNELQKDKMAEAYSEIGMKGERRRGKGH DGLYQGLSTATKDTYDALHMQALPPR |

## Claims

1. Use of a cell therapy product in the preparation of a medicament for treating a cancer in a patient who has failed in previous treatment of the cancer, said previous treatment includes treatment with an anti-PD-1 antibody and/or an anti-PD-L1 antibody.

2. The use according to claim 1, wherein the cell therapy product comprises an immune effector cell expressing an exogenous receptor.

3. The use according to claim 2, wherein the immune effector cell is selected from the group consisting of: a T cell, an NK cell, an NKT cell, a mast cell, a macrophage, a dendritic cell, a CIK cell, a stem cell-derived immune effector cell, or any combination thereof.

4. The use according to claim 2, wherein the immune effector cell is derived from a natural T cell, and/or a T cell induced by a pluripotent stem cell.

5. The use according to any one of claims 2-4, wherein the immune effector cell is an autologous/allogeneic T cell, or a primary T cell.

6. The use according to any one of claims 3-5, wherein the T cell comprises a memory stem cell-like T cell (Tscm cell), a central memory T cell (Tcm), an effector T cell (Tef), a regulatory T cell (Tregs), an effector memory T cell (Tern), a γδ T cell, an αβ T cell, or any combination thereof.

7. The use according to any one of claims 2-6, wherein the exogenous receptor is selected from the group consisting of: a chimeric antigen receptor (CAR), a T cell receptor (TCR), a T cell fusion protein (TFP), a T cell antigen coupler (TAC), or any combination thereof.

8. The use according to any one of claims 2-7, wherein the antigen-binding domain of the exogenous receptor specifically recognizes a tumor antigen.

9. The use according to claim 8, wherein the tumor antigen is selected from the group consisting of: CD19, CD20, CD22, CD30, Mesothelin, BCMA, EGFR, EGFRvIII, PSMA, Mucl, claudin18.2, GPC3, IL13RA2, SLAMF7, GPRC5D, LILRB4, or any combination thereof; preferably, the tumor antigen is selected from the group consisting of: GPC3, CD19, BCMA, Claudin18.2, or any combination thereof.

10. The use according to any one of claims 1-9, wherein the cancer comprises a solid tumor and/or a blood tumor; preferably the solid tumor comprises gastrointestinal tumors; more preferably the gastrointestinal tumors comprise gastric cancer/esophagogastric junction adenocarcinoma, gallbladder cancer, liver cancer, Krukenberg tumor, or any combination thereof.

11. Use according to any one of claims 1-10, wherein the cancer is selected from the group consisting of: krukenberg tumor, gastric cancer, pancreatic cancer, gallbladder cancer, melanoma, non-small cell lung cancer (NSCLC), small cell lung cancer (SCLC), head and neck squamous cell carcinoma (HNSCC), classical Hodgkin lymphoma (cHL), primary mediastinal large B-cell lymphoma (PMBCL), urothelial carcinoma (UC), esophageal cancer, cervical cancer, liver cancer, Merkel cell carcinoma, renal cell carcinoma (RCC), colorectal cancer (mCRC), and breast cancer.

12. The use according to any one of claims 1-11, wherein the anti-PD-1 antibody/ anti-PD-L1 antibody is selected from the group consisting of: nivolumab, pembrolizumab, cemiplimab, camrelizumab, toripalimab, sintilimab, tislelizumab, cepalimumab (GLS-010), tislelizumab, or any combination thereof.

13. The use according to any one of claims 1-12, wherein the previous treatment comprises drug therapy, surgical treatment, radiotherapy, or any combination thereof; the drug therapy comprises administration of a chemical drug and/or a biological drug; preferably, the previous treatment comprises administration of olaparib, lenvatinib, cabozantinib, axitinib, ipilimumab, a platinum-based chemotherapy drug, pemetrexed, etoposide, a taxane compound, bevacizumab, regorafenib, rotinib, apatinib, lenvatinib, fruquintinib, regorafenib, or any combination thereof.

14. The use according to claim 1, wherein the cell therapy product is administered to a patient with the cancer for treatment for at least one treatment cycle; preferably, the cell therapy product is administered to the patient with the cancer for treatment for 1-3 treatment cycles.

15. The use according to any one of claims 7-14, wherein the chimeric antigen receptor (CAR) comprises:
(i) an antigen-binding domain specifically binding to a tumor antigen, a transmembrane region of CD28 or CD8, and CD3ζ;
(ii) an antigen-binding domain specifically binding to a tumor antigen, a transmembrane region of CD28 or CD8, a co-stimulatory signal domain of CD28, and CD3ζ;
(iii) an antigen-binding domain specifically binding to a tumor antigen, a transmembrane region of CD28 or CD8, a co-stimulatory signal domain of CD137, and CD3ζ; and/or
(iv) an antigen-binding domain specifically binding to a tumor antigen, a transmembrane region of CD28 or CD8, a co-stimulatory signal domain of CD28, a co-stimulatory signal domain of CD137, and CD3ζ.

16. The use according to claim 14 or 15, wherein the dose of cells in the cell therapy product administered per treatment cycle does not exceed about 2×10⁹ cells/kg, 2×10⁸ cells/kg, or 2×10⁷ cells/kg of patient body weight; or the dose of cells in the cell therapy product does not exceed about 1×10¹¹ cells/patient, 1×10¹⁰ cells/patient, 5×10⁹ cells/patient, 2×10⁹ cells/patient, or 1×10⁹ cells/patient.

17. The use according to claim 16, wherein the dose of cells in the cell therapy product administered per treatment cycle is about 1×10⁵ cells/kg to 2×10⁷ cells/kg of patient body weight, or about 1×10⁶ cells/kg to 2×10⁷ cells/kg patient body weight; or
the dose of cells in the cell therapy product administered per treatment cycle is about 1×10⁷ cells to 5×10⁹ cells/patient, about 1×10⁷ cells to 2×10⁹ cells/patient, or about 1×10⁷ cells to 1×10⁹ cells/patient; or the dose of cells in the cell therapy product administered per treatment cycle is about 1×10⁸ cells to 5×10⁹ cells/patient, about 1×10⁸ cells to 2×10⁹ cells/patient, or about 1×10⁸ cells to 1×10⁹ cells/patient; or the dose of cells in the cell therapy product administered per treatment cycle is about 2.5×10⁸ cells to 5×10⁸ cells/patient.

18. The use according to any one of claims 14-17, wherein pretreatment is performed before administration of the cell therapy product in each treatment cycle, and the pretreatment comprises administration of a chemical drug, a biological drug, radiotherapy, or any combination thereof to the patient.

19. The use according to claim 18, wherein the pretreatment is implemented 1-8 days before administration of the cell therapy product; preferably, 2-6 days before administration of the cell therapy product; preferably, a chemical drug, a biological drug, a radiotherapy or any combination thereof is administered for no more than 4 consecutive days.

20. The use according to claim 18 or 19, wherein the chemical drug is any one or at least two selected from the group consisting of: cyclophosphamide, fludarabine, a tubulin inhibitor, and pyrimidine antineoplastic drugs; or the chemical drug comprises cyclophosphamide and fludarabine; or the chemical drug comprises cyclophosphamide, fludarabine and a tubulin inhibitor.

21. The use according to claim 20, wherein the tubulin inhibitor is a taxane compound; preferably the taxane compound is selected from the group consisting of: paclitaxel, nab-paclitaxel, and docetaxel; more preferably the taxane compound is nab-paclitaxel.

22. The use according to claim 20 or 21, wherein the amount of fludarabine to be administered is about 10-50mg/m²/day, or about 15-40mg/m²/day, or about 15-30mg/m²/day, or about 20-30mg/m²/day, or about 25mg/m²/day, or about 30-60mg/day, or about 30-50mg/day, or about 35-45mg/day;
the amount of cyclophosphamide to be administered is about 200-400mg/m²/day, or about 200-300mg/m²/day, or about 250mg/m²/day, or about 300-700mg/day, or about 300-550mg/day, or about 300-500mg/day;
the dosage of the taxane compound is not more than about 300mg/day, or not more than about 200 mg/day, or about 90-120 mg/day.

23. The use according to any one of claims 20-22, wherein the cyclophosphamide is administered 2-3 times; or the fludarabine is administered 1-2 times; or the taxane compound is administered once.

24. The use according to any one of claims 8-15, wherein the antigen binding domain comprises:
HCDR1 shown in SEQ ID NO:1, HCDR2 shown in SEQ ID NO:2, HCDR3 shown in SEQ ID NO:3, LCDR1 shown in SEQ ID NO:4, LCDR2 shown in SEQ ID NO:5, LCDR3 shown in SEQ ID NO: 6; or
HCDR1 shown in SEQ ID NO:16, HCDR2 shown in SEQ ID NO:17, HCDR3 shown in SEQ ID NO:18, LCDR1 shown in SEQ ID NO:19, LCDR2 shown in SEQ ID NO:20, LCDR3 shown in SEQ ID NO:21; or
HCDR1 shown in SEQ ID NO:27, HCDR2 shown in SEQ ID NO:28, HCDR3 shown in SEQ ID NO:29, LCDR1 shown in SEQ ID NO:30, LCDR2 shown in SEQ ID NO:31, LCDR3 shown in SEQ ID NO:32;
or the antigen binding domain comprises:
the heavy chain variable region shown in SEQ ID NO:7, and the light chain variable region shown in SEQ ID NO:9; or
the heavy chain variable region shown in SEQ ID NO:22, and the light chain variable region shown in SEQ ID NO:23; or
the heavy chain variable region shown in SEQ ID NO:33, and the light chain variable region shown in SEQ ID NO:34;
or the antigen-binding domain comprises the sequence shown in SEQ ID NO: 14, 24, 35, or 37.

25. The use according to any one of claims 7-24, wherein the chimeric antigen receptor comprises an amino acid sequence shown in any one of SEQ ID NO: 11, 12, 13, 25, 26, 36, 38 or 52, or a polypeptide formed by sequentially connecting any sequence shown in SEQ ID NO: 58, 59, 60, 61, 62, 63, 64, 65, 66, or 67 with any sequence shown in SEQ ID NO: 80, 81, or 82, or a polypeptide formed by sequentially connecting any sequence shown in SEQ ID NO: 70, 71, 72, 73, 74, 75, 76, 77, 78 or 79 with any sequence shown in SEQ ID NO: 80, 81 or 82.

26. The use according to any one of claims 14-25, wherein before administering the cell therapy product in each treatment cycle, the serum levels of a cytokine indicating CRS, a cytokine indicating neurotoxicity, an indicator indicating tumor burden, and/or a factor indicating host anti-CAR immune response in the patient are evaluated.

27. The use according to any one of claims 1-26, wherein after administration of the cell therapy product, the patient does not show severe CRS, or does not show neurotoxicity exceeding Level 3.

28. The use according to any one of claims 1-27, wherein at least a part, preferably at least 40%, more preferably at least 50% of the patients who have failed to treat a cancer with anti-PD-1 antibody or anti-PD-L1 antibody respond to the cell therapy product.

29. The use according to claim 28, wherein the response means an ORR greater than 40%, preferably greater than 50%.

30. A method for treating a cancer in a patient who has failed in previous treatment of the cancer by using a cell therapy product, wherein the previous treatment comprises treatment with an anti-PD-1 antibody and/or an anti-PD-L1 antibody.

31. The method according to claim 30, wherein the cell therapy product comprises an immune effector cell expressing an exogenous receptor.

32. The method according to claim 31, wherein the immune effector cell is selected from the group consisting of: a T cell, an NK cell, an NKT cell, a mast cell, a macrophage, a dendritic cell, a CIK cell, and a stem cell-derived immune effector cell, or any combination thereof.

33. The method according to claim 31, wherein the immune effector cell is derived from a natural T cell, and/or a T cell induced by a pluripotent stem cell.

34. The method according to any one of claims 31-33, wherein the immune effector cell is an autologous/allogeneic T cell, or a primary T cell.

35. The method according to any one of claims 32-34, wherein the T cell comprises a memory stem cell-like T cell (Tscm cell), a central memory T cell (Tcm), an effector T cell (Tef), a regulatory T cell (Tregs), an effector memory T cell (Tern), a γδ T cell, an αβ T cell, or any combination thereof.

36. The method according to any one of claims 31-35, wherein the exogenous receptor is selected from the group consisting of: a chimeric antigen receptor (CAR), a T cell receptor (TCR), a T cell fusion protein (TFP), a T cell antigen coupler (TAC), or any combination thereof.

37. The method according to any one of claims 31-36, wherein the antigen binding domain of the exogenous receptor specifically recognizes a tumor antigen.

38. The method according to claim 37, wherein the tumor antigen is selected from the group consisting of: CD19, CD20, CD22, CD30, Mesothelin, BCMA, EGFR, EGFRvIII, PSMA, Mucl, claudin18.2, GPC3, IL13RA2, SLAMF7, GPRC5D, LILRB4, or any combination thereof; preferably, the tumor antigen is selected from the group consisting of: GPC3, CD19, BCMA, Claudin18.2, or any combination thereof.

39. The method according to any one of claims 30-38, wherein the cancer comprises a solid tumor and/or a hematological tumor; preferably the solid tumor comprises a gastrointestinal tumor; more preferably the gastrointestinal tumor comprises gastric cancer/esophagogastric junction adenocarcinoma, gallbladder cancer, liver cancer, Krukenberg tumor, or any combination thereof.

40. The method according to any one of claims 30-39, wherein the cancer is selected from the group consisting of: krukenberg tumor, gastric cancer, pancreatic cancer, gallbladder cancer, melanoma, non-small cell lung cancer (NSCLC), small cell lung cancer (SCLC), head and neck squamous cell carcinoma (HNSCC), classical Hodgkin lymphoma (cHL), primary mediastinal large B-cell lymphoma (PMBCL), urothelial carcinoma (UC), esophageal cancer, cervical cancer, liver cancer, Merkel cell carcinoma, renal cell carcinoma (RCC), colorectal cancer (mCRC), and breast cancer.

41. The method according to any one of claims 30-40, wherein the anti-PD-1 antibody/ anti-PD-L1 antibody is selected from the group consisting of: nivolumab, pembrolizumab, cemiplimab, camrelizumab, toripalimab, sintilimab, tislelizumab, cepalimumab (GLS-010), tislelizumab, or any combination thereof.

42. The method according to any one of claims 30-41, wherein the previous treatment comprises drug therapy, surgical treatment, radiotherapy, or any combination thereof; the drug therapy comprises administration of a chemical drug and/or a biological drug; preferably, the previous treatment comprises administration of olaparib, lenvatinib, cabozantinib, axitinib, ipilimumab, a platinum-based chemotherapy drug, pemetrexed, etoposide, a taxane compound, bevacizumab, regorafenib, rotinib, apatinib, lenvatinib, fruquintinib, regorafenib, or any combination thereof.

43. The method according to claim 30, wherein the cell therapy product is administered to a patient with the cancer for treatment for at least one treatment cycle; preferably, the cell therapy product is administered to the patient with the cancer for treatment for 1-3 treatment cycles.

44. The method according to any one of claims 36-43, wherein the chimeric antigen receptor (CAR) comprises:
(i) an antigen-binding domain specifically binding to a tumor antigen, a transmembrane region of CD28 or CD8, and CD3ζ;
(ii) an antigen-binding domain specifically binding to a tumor antigen, a transmembrane region of CD28 or CD8, a co-stimulatory signal domain of CD28, and CD3ζ;
(iii) an antigen-binding domain specifically binding to a tumor antigen, a transmembrane region of CD28 or CD8, a co-stimulatory signal domain of CD137, and CD3ζ; and/or
(iv) an antigen-binding domain specifically binding to a tumor antigen, a transmembrane region of CD28 or CD8, a co-stimulatory signal domain of CD28, a co-stimulatory signal domain of CD137, and CD3ζ.

45. The method according to claim 43 or 44, wherein the dose of cells in the cell therapy product administered per treatment cycle does not exceed about 2×10⁹ cells/kg, 2×10⁸ cells/kg, or 2×10⁷ cells/kg of patient body weight; or the dose of cells in the cell therapy product does not exceed about 1×10¹¹ cells/patient, 1×10¹⁰ cells/patient, 5×10⁹ cells/patient, 2×10⁹ cells/patient, or 1×10⁹ cells/patient.

46. The method according to claim 45, wherein the dose of cells in the cell therapy product administered per treatment cycle is about 1×10⁵ cells/kg patient body weight to 2×10⁷ cells/kg patient body weight, or about 1×10⁶ cells/kg patient body weight to 2×10⁷ cells/kg patient body weight; or
the dose of cells in the cell therapy product administered per treatment cycle is about 1×10⁷ cells to 5×10⁹ cells/patient, about 1×10⁷ cells to 2×10⁹ cells/patient, or about 1×10⁷ cells to 1×10⁹ cells/patient; or the dose of cells in the cell therapy product administered per treatment cycle is about 1×10⁸ cells to 5×10⁹ cells/patient, about 1×10⁸ cells to 2×10⁹ cells/patient, or about 1×10⁸ cells to 1×10⁹ cells/patient; or the dose of cells in the cell therapy product administered per treatment cycle is about 2.5×10⁸ cells to 5×10⁸ cells/patient.

47. The method according to any one of claims 43-46, wherein pretreatment is performed before administration of the cell therapy product in each treatment cycle, and the pretreatment comprises administration of a chemical drug, a biological drug, radiotherapy, or any combination thereof to the patient.

48. The method according to claim 47, wherein the pretreatment is implemented 1-8 days before administration of the cell therapy product; preferably, 2-6 days before administration of the cell therapy product; preferably, a chemical drug, a biological drug, radiotherapy or any combination thereof is administered for no more than 4 consecutive days.

49. The method according to claim 47 or 48, wherein the chemical drug is any one or at least two selected from the group consisting of: cyclophosphamide, fludarabine, a tubulin inhibitor, and a pyrimidine antineoplastic drug; or the chemical drug comprises cyclophosphamide and fludarabine; or the chemical drug comprises cyclophosphamide, fludarabine and a tubulin inhibitor.

50. The method according to claim 49, wherein the tubulin inhibitor is a taxane compound;
preferably the taxane compound is selected from the group consisting of: paclitaxel, nab-paclitaxel, and docetaxel; more preferably the taxane compound is nab-paclitaxel.

51. The method according to claim 49 or 50, wherein the amount of fludarabine to be administered is about 10-50mg/m²/day, or about 15-40mg/m²/day, or about 15-30mg/m²/day, or about 20-30mg/m²/day, or about 25mg/m²/day, or about 30-60mg/day, or about 30-50mg/day, or about 35-45mg/day;
the amount of cyclophosphamide to be administered is about 200-400mg/m²/day, or about 200-300mg/m²/day, or about 250mg/m²/day, or about 300-700mg/day, or about 300-550mg/day, or about 300-500mg/day;
the dosage of the taxane compound is not more than about 300mg/day, or not more than about 200 mg/day, or about 90-120 mg/day.

52. The method according to any one of claims 49-51, wherein the cyclophosphamide is administered 2-3 times; or the fludarabine is administered 1-2 times; or the taxane compound is administered once.

53. The method according to any one of claims 37-44, wherein the antigen binding domain comprises:
HCDR1 shown in SEQ ID NO:1, HCDR2 shown in SEQ ID NO:2, HCDR3 shown in SEQ ID NO:3, LCDR1 shown in SEQ ID NO:4, LCDR2 shown in SEQ ID NO:5, LCDR3 shown in SEQ ID NO: 6; or
HCDR1 shown in SEQ ID NO:16, HCDR2 shown in SEQ ID NO:17, HCDR3 shown in SEQ ID NO:18, LCDR1 shown in SEQ ID NO:19, LCDR2 shown in SEQ ID NO:20, LCDR3 shown in SEQ ID NO:21; or
HCDR1 shown in SEQ ID NO:27, HCDR2 shown in SEQ ID NO:28, HCDR3 shown in SEQ ID NO:29, LCDR1 shown in SEQ ID NO:30, LCDR2 shown in SEQ ID NO:31, LCDR3 shown in SEQ ID NO:32;
or the antigen binding domain comprises:
the heavy chain variable region shown in SEQ ID NO:7 and the light chain variable region shown in SEQ ID NO:9; or
the heavy chain variable region shown in SEQ ID NO:22 and the light chain variable region shown in SEQ ID NO:23; or
the heavy chain variable region shown in SEQ ID NO:33 and the light chain variable region shown in SEQ ID NO:34;
or the antigen-binding domain comprises the sequence shown in SEQ ID NO: 14, 24, 35, or 37.

54. The method according to any one of claims 36-53, wherein the chimeric antigen receptor comprises the amino acid sequence shown in any one of SEQ ID NO: 11, 12, 13, 25, 26, 36, 38 or 52, or a polypeptide formed by sequentially connecting any sequence shown in SEQ ID NO: 58, 59, 60, 61, 62, 63, 64, 65, 66, or 67 with any sequence shown in SEQ ID NO: 80, 81, or 82, or a polypeptide formed by sequentially connecting any sequence shown in SEQ ID NO: 70, 71, 72, 73, 74, 75, 76, 77, 78 or 79 with any sequence shown in SEQ ID NO: 80, 81 or 82.

55. The method according to any one of claims 43-54, wherein before administering the cell therapy product in each treatment cycle, the serum levels of a cytokine indicating CRS, a cytokine indicating neurotoxicity, an indicator indicating tumor burden, and/or a factor indicating host anti-CAR immune response in the patient are evaluated.

56. The method according to any one of claims 30-55, wherein after administration of the cell therapy product, the patient does not show severe CRS, or does not show neurotoxicity exceeding Level 3.

57. The method according to any one of claims 30-56, wherein at least a part, preferably at least 40%, more preferably at least 50% of the patients who have failed to treat a cancer with anti-PD-1 antibody or anti-PD-L1 antibody respond to the cell therapy product.

58. The method according to claim 57, wherein the response means an ORR greater than 40%, preferably greater than 50%.
